# EUROPEAN PATENT SPECIFICATION

(11) **EP 1 966 146 B1**
(45) Date of publication and mention of the grant of the patent: **02.03.2011**
(21) Application number: 06841437.4
(22) Date of filing: 19.12.2006
(51) Int. Cl.: C07D 231/06, A61K 31/40, A61P 25/00, C07D 403/12, C07D 405/06, C07D 409/04, C07D 409/06, C07C 49/213

(54) **4,5-DIHYDRO- (1H)-PYRAZOLE DERIVATIVES AS CANNABINOID CB1 RECEPTOR MODULATORS**
4,5-DIHYDRO-(1H)-PYRAZOL-DERIVATE ALS CANNABINOID-CB1-REZEPTORMODULATOREN
DERIVES DE 4,5-DIHYDRO-(1H)-PYRAZOLE EN TANT QUE MODULATEURS DU RECEPTEUR CANNABINOIDE CB1

(30) Priority: 20.12.2005 EP 05112482; 20.12.2005 US 751667 P
(43) Date of publication of application: 10.09.2008
(73) Proprietor: Abbott Healthcare Products B.V., 1381 CP Weesp (NL)
(72) Inventor: LANGE, Josephus H.M., NL-1381 CP Weesp (NL); VAN DER NEUT, Martina A.W., NL-1381 CP Weesp (NL); VAN VLIET, Bernard J., NL-1381 CP Weesp (NL); IWEMA BAKKER, Wouter I., NL-1381 CP Weesp (NL)
(74) Representative: Verhage, Marinus
(86) International application number: PCT/EP2006/069878
(87) International publication number: WO 2007/071662

(56) References cited:
- WO-A-01/70700
- WO-A-2005/077911
- PATENT ABSTRACTS OF JAPAN vol. 011, no. 013 (C-397), 14 January 1987 (1987-01-14) & JP 61 189270 A (NISSAN CHEM IND LTD), 22 August 1986 (1986-08-22)
- WANG SHUOWEN ET AL: "A convenient synthesis of novel 4-(1,2,4-triazol-1-yl)-2-pyrazolines and their derivatives" SYNTHETIC COMMUNICATIONS, vol. 33, no. 9, 2003, pages 1449-1457, XP009081622
- BARLUENGA, JOSE ET AL: "First highly regio- and diastereoselective [3+2] cycloaddition of chiral nonracemic alkenyl Fischer carbene complexes with diazomethane derivatives: preparation and synthetic applications of enantiomerically pure .DELTA.2-pyrazolines" CHEMISTRY--A EUROPEAN JOURNAL , 5(3), 883-896 CODEN: CEUJED; ISSN: 0947-6539, 1999, XP002381484
- HERTZOG D L: "Recent advances in the cannabinoids" EXPERT OPINION ON THERAPEUTIC PATENTS, ASHLEY PUBLICATIONS, GB, vol. 14, no. 10, 2004, pages 1435-1452, XP002362228 ISSN: 1354-3776 cited in the application
- OGATA M ET AL: "SYNTHESIS AND ORAL ANTIFUNGAL ACTIVITY OF NOVEL AZOLYLPROPANOLONES AND RELATED COMPOUNDS" JOURNAL OF MEDICINAL CHEMISTRY, vol. 30, no. 6, 1987, pages 1054-1068, XP002427593 ISSN: 0022-2623
- CHUL-HO JUN, HYUK LEE, JUN-BAE HONG, BONG-IL KWON: "Efficient and Selective Hydroacylation of 1-Alkynes with Aldehydes by a Chelation-Assisted Catalytic System" ANGEW. CHEM. INT. ED., vol. 41, no. 12, 2002, pages 2146-2147, XP002427594 Weinheim, Germany
- CRAGOE E J JR ET AL: "AGENTS FOR THE TREATMENT OF BRAIN EDEMA 2. 2 3 9 9A TETRAHYDRO-3-OXO-9A-SUBSTITUTED-1H-FLUOREN -7-YLOXYALKANOIC ACIDS AND SOME OF THEIR ANALOGS" JOURNAL OF MEDICINAL CHEMISTRY, vol. 29, no. 5, 1986, pages 825-841, XP002427595 ISSN: 0022-2623
- HON Y-S ET AL: "Dibromomethane as one-carbon source in organic synthesis: microwave-accelerated alpha-methylenation of ketones with dibromomethane and diethylamine" TETRAHEDRON, ELSEVIER SCIENCE PUBLISHERS, AMSTERDAM, NL, vol. 59, no. 9, 24 February 2003 (2003-02-24), pages 1509-1520, XP004409950 ISSN: 0040-4020
- JONES D G ET AL: "Discovery of non-steroidal mifepristone mimetics: Pyrazoline-based PR antagonists" BIOORGANIC & MEDICINAL CHEMISTRY LETTERS, OXFORD, GB, vol. 15, no. 13, 1 July 2005 (2005-07-01), pages 3203-3206, XP004947938 ISSN: 0960-894X

## Description

### SUMMARY: TECHNICAL FIELD OF THE INVENTION

This invention is directed to 4,5-dihydro -(1H)-pyrazole (pyrazoline) derivatives as cannabinoid CB₁ receptor modulators, to pharmaceutical compositions containing these compounds, to methods for the preparation of these compounds, methods for preparing novel intermediates useful for their synthesis, and methods for preparing compositions. The invention also relates to the uses of such compounds and compositions, particularly their use in administering them to patients to achieve a therapeutic effect in disorders in which CB₁ receptors are involved, or that can be treated via manipulation of those receptors.

### RELATED APPLICATIONS

This application claims priority benefit under Article 87 EPC of EP 05 112482.4 filed on December 20, 2005, and also under Title 35 § 119(e) of U.S. Provisional Application No. 60/751,667 filed on December 20, 2005, the contents of which are herein incorporated by reference.

### BACKGROUND OF THE INVENTION

Cannabinoid receptors are part of the endo-cannabinoid system which is involved in several diseases, such as neurological, psychiatric, cardiovascular, gastrointestinal, reproductive, eating disorders and cancer (De Petrocellis, 2004; Di Marzo, 2004; Lambert and Fowler, 2005; Vandevoorde and Lambert, 2005).

CB₁ receptor modulators have several potential therapeutic applications such as medicaments for treating psychosis, anxiety, depression, attention deficits, memory disorders, cognitive disorders, appetite disorders, obesity, addiction, appetence, drug dependence, neurodegenerative disorders, dementia, dystonia, muscle spasticity, tremor, epilepsy, multiple sclerosis, traumatic brain injury, stroke, Parkinson's disease, Alzheimer's disease, Huntington's disease, Tourette's syndrome, cerebral ischaemia, cerebral apoplexy, craniocerebral trauma, stroke, spinal cord injury, neuroinflammatory disorders, plaque sclerosis, viral encephalitis, demyelinisation related disorders, as well as for the treatment of pain disorders, including neuropathic pain disorders, septic shock, glaucoma, diabetes, cancer, emesis, nausea, gastrointestinal disorders, gastric ulcers, diarrhoea, sexual disorders, impulse control disorders and cardiovascular disorders.

CB₂ receptors occur predominantly in the immune system (spleen, tonsils, immune cells), but also in astrocytes, microglial cells and in the brainstem and have been linked to the perception of neuropathic pain as well as allergy/asthma and (neuro)inflammatory conditions (Van Sickle, 2005).

Diarylpyrazoline derivatives having cannabinoid CB₁ receptor antagonistic or inverse agonistic affinity have been claimed in WO 01/70700, WO 03/026647, WO 03/026648, WO 2005/074920, and were described by Lange (2004, 2005).

Pyrazoline derivatives which act as agonists or partial agonists on the CB₁ receptor have not been reported yet, but certain pyrazoline derivatives have been claimed as vermin controlling agents (JP 61 189270).

There is abundant recent literature containing general information on CB receptor modulators (Lange and Kruse, 2004, 2005; Hertzog, 2004; Smith and Fathi, 2005; Thakur, 2005; Padgett, 2005; Muccioli, 2005; Raitio,2005; Muccioli and Lambert, 2006).

The objective of the present invention was to develop novel compounds with CB₁ receptor agonistic activity.

### DETAILED DESCRIPTION OF THE INVENTION

Surprisingly, we have found that the modification of the original 3-aryl or 3 -heteroaryl group R in prior art pyrazolines of general formula (I) by a (substituted) alkyl moiety - in combination with a different substitution pattern at the 1-position of the pyrazoline moiety - results in novel compounds with potent CB₁ receptor affinity. Moreover, some of the compounds of the invention also have been found to act as partial agonists or full agonists at the CB₁ receptor, whereas other compounds of the invention have been found to act as antagonists or inverse agonists at the CB₁ receptor. The majority of the compounds of the invention showed also affinity for the CB₂ receptor. These compounds may act as CB₂ receptor agonists, CB₂ receptor antagonists or CB₂ receptor inverse agonists.

The present invention relates to compounds of the general formula (I): 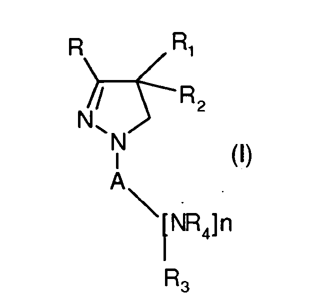 wherein
- R represents a C₂₋₁₀ alkyl group, a C₄₋₁₀ alkenyl group, a C₄₋₁₀ alkynyl group, a C_{2 10}-heteroalkyl group, a C₅₋₈₋cycloalkyl-C₁₋₅-alkyl group or a C₅₋₈-heterocycloalkyl-C₁₋₅-alkyl group wherein the heteroatom(s) are either N, O or S, which C₂₋₁₀ alkyl group, C₄₋₁₀ alkenyl group, C₄₋₁₀ alkynyl group, C₂₋₁₀-heteroalkyl group, C₅₋₈-cycloalkyl-C₁₋₈-alkyl group or C₅₋₈-heterocycloalkyl-C₁₋₅-alkyl group may be substituted with 1-5 substituents selected from methyl, ethyl, hydroxy, amino or fluoro, or R represents an aryl-C₁₋₃-alkyl group or an aryl-C₁₋₃-heteroalkyl group in which the aryl groups may be substituted with 1-5 substituents Y, which can be the same or different, selected from the group C₁₋₃-alkyl or alkoxy, hydroxy, halogen, trifluoromethyl, trifluoromethylthio, trifluoromethoxy, nitro, amino, mono- or dialkyl (C₁₋₂)-amino, mono- or dialkyl (C₁₋₂)-amido, (C₁₋₃)-alkyl sulfonyl, dimethylsulfamido, C₁₋₃-alkoxycarbonyl, carboxyl, trifluoromethyl-sulfonyl, cyano, carbamoyl, sulfamoyl, phenyl and acetyl, or R represents a cyclopropyl group which cyclopropyl group may be substituted with 1-5 substituents selected from methyl, ethyl, fluoro or with a C₃₅ linear or branched alkyl group or with a benzyl or aryl group, in which the aryl or benzyl group may be substituted with 1-5 substituents Y,
- R₁ represents hydrogen, hydroxy, C₁₋₃-alkoxy, acetyloxy or propionyloxy,
- R₂ represents an aryl group which may be substituted with 1-5 substituents Y, wherein Y has the abovementioned meaning,
- n is either 0 or 1
- R₃ represents a linear C₃₋₁₀ alkyl group, a branched C₅₋₁₀ alkyl group, a cyclopropyl, cyclobutyl, cyclopentyl, cycloheptyl or cyclooctyl group, C₅₋₁₀ bicycloalkyl group, C₆₋₁₀ tricycloalkyl group or C₈₋₁₁ tetracycloalkyl group which groups may be substituted with 1-5 substituents selected from methyl, ethyl,
- hydroxy, amino, fluoro or R₃ represents a C₃₋₈ cycloalkyl group which C₃₋₈ cycloalkyl group is substituted with an aryl group which aryl group may be substituted with 1-5 substituents Y wherein Y has the abovementioned meaning, or R₃ represents a 2,2,2-trifluoroethyl or 2-fluoroethyl group or R₃ represents a cyclohexyl group which group is substituted with 1-5 substituents selected from methyl, ethyl, hydroxy, amino or fluoro, or R₃ represents a C₅₋₈ heterocycloalkyl group, C₆₋₁₀ bicycloheteroalkyl group, C₇₋₁₀ tricycloheteroalkyl group, which groups may be substituted with 1-5 substituents selected from methyl, ethyl, hydroxy, amino or fluoro, or R₃ represents a C₃₋₈ cycloalkyl-C₁₋₃-alkyl group, C₅₋₁₀-bicycloalkyl-C₁₋₃-alkyl group, C₆₋₁₀-cycloalkyl-C₁₋₃-alkyl group, which groups may be substituted with 1-5 substituents selected from methyl, ethyl, hydroxy, amino or fluoro, or R₃ represents a branched or linear C₃₋₈ heterocycloalkyl-C₁₋₃-alkyl group, C₅₋₁₀ bicycloheteroalkyl-C₁₋₃-alkyl group, C₆₋₁₀ tricycloheteroalkyl-C₁₋₃ alkyl group, which groups may be substituted with 1-5 substituents selected from methyl, ethyl, hydroxy, amino or fluoro, or R₃ represents an aryl group, which group may be substituted with 1-5 substituents Y, wherein Y has the abovementioned meaning, or R₃ represents a aryl-C₁₋₅-alkyl group or a diaryl-C₁₋₅-alkyl group, in which groups the phenyl or heteroaromatic rings may be substituted with 1-5 substituents Y, wherein Y has the abovementioned meaning, or R₃ represents a linear or branched C₄₋₈ alkenyl or C₄₋₈ alkynyl group which linear or branched C₄₋₈ alkenyl or C₄₋₈ alkynyl group may be substituted with 1-3 fluoro atoms, or, only when n=1, R₃ may Additional represent a branched or linear C₂₋₁₀ heteroalkyl group, containing 1-2 heteroatoms selected from N,O or S,
- R₄ represents a hydrogen atom, a C₁₋₄ alkyl group or R₃ and R₄ - together with the nitrogen atom to which they are bonded - form a saturated or unsaturated, nonaromatic or partly aromatic, monocyclic, bicyclic or tricyclic heterocyclic group having 5 to 11 ring atoms, which heterocyclic group may be substituted with 1-5 substituents selected from aryl, aryl-C₁₋₃-alkyl, diarylmethyl, or Y, wherein Y has the abovementioned meaning
- A represents a carbonyl (C=O), thiocarbonyl (C=S) or sulfonyl (SO₂) group with the proviso that when A represents a thiocarbonyl (C=S),group, n has the value 1,
and stereoisomers, and N-oxides thereof, and isotopically-labelled compounds of formula (I), as well as pharmacologically acceptable salts, hydrates, solvates, complexes and conjugates of said compounds of formula (I) and its stereoisomers, N-oxides, or isotopically-labelled analogs.

Although not known in the literature, compounds of formula (I) with A representing thiocarbonyl (C=S), and with n=0, were outside the scope of the project, and have not been synthesized.

The invention particularly relates to compounds of the general formula (I) wherein R₁ represents a hydrogen atom, and the other symbols have the meanings as given above.

More particular, the invention relates to compounds of the general formula (I) wherein R₁ represents a hydrogen atom, A represents a carbonyl group, and the other symbols have the meanings as given above.

Even more particular, the invention relates to compounds of the general formula (I) wherein R₁ represents a hydrogen atom, A represents a carbonyl group, R₂ represents a phenyl, thienyl or pyridyl group, which phenyl, pyridyl or thienyl group may be substituted with 1, 2 or 3 substituents Y, and the other symbols have the meanings as given above.

Also in particular, the invention relates to compounds of the general formula (I) wherein n=1, R₁ represents a hydrogen atom, A represents a carbonyl group, R₂ represents a phenyl, thienyl or pyridyl group, which phenyl, pyridyl or thienyl group may be substituted with 1, 2 or 3 substituents Y, and the other symbols have the meanings as given above.

Likewise, the invention particularly relates to compounds of the general formula (I) wherein n=1, R₁ and R₄ represent hydrogen atoms, A represents a carbonyl group, R₂ represents a phenyl, thienyl or pyridyl group, which phenyl, pyridyl or thienyl group may be substituted with 1, 2 or 3 substituents Y, and the other symbols have the meanings as given above.

Most particularly the invention relates to compounds of the general formula (I) wherein n = 1, R represents a C₃₋₈ branched or linear alkyl group, which C₃₋₈ branched or linear alkyl group may be substituted with 1-3 fluoro atoms, R₁ and R₄ represent hydrogen atoms, R₂ represents a phenyl or pyridyl group, which phenyl or pyridyl group may be substituted with 1, 2 or 3 substituents Y, and the other symbols have the meanings as given above.

The compounds of the invention of the general formula (I), as well as the pharmacologically acceptable salts thereof, have cannabinoid CB₁ receptor modulating activity. They are useful in the treatment of disorders in which cannabinoid receptors are involved, or that can be tre ated via manipulation of those receptors.

The invention is also directed to:
a pharmaceutical composition for treating, for example, a disorder or condition that may be treated by modulating cannabinoid CB₁ receptors, the composition comprising a compound of formula (I) or a pharmaceutically acceptable salt thereof, and a pharmaceutically acceptable carrier;
a pharmaceutical composition for treating, for example, a disorder or condition selected from the group consisting of psychosis, anxiety, depession, attention deficits, memory disorders, cognitive disorders, appetite disorders, obesity, addiction, appetence, drug dependence, neurodegenerative disorders, dementia, dystonia, muscle spasticity, tremor, multiple sclerosis, traumatic brain injury, stroke, Parkinson's disease, Alzheimer's disease, epilepsy, Huntington's disease, Tourette's syndrome, cerebral ischaemia, cerebral apoplexy, craniocerebral trauma, stroke, spinal cord injury, neuroinflammatory disorders, plaque sclerosis, viral encephalitis, demyelinisation related disorders, as well as for the treatment of pain disorders, including neuropathic pain disorders, septic shock, glaucoma, diabetes, cancer, emesis, nausea, gastrointestinal disorders, gastric ulcers, diarrhoea, sexual disorders, impulse control disorders and cardiovascular disorders;
a pharmaceutical composition for treatment of a disorder or condition selected from the group consisting of the disorders listed herein, the composition comprising a compound of formula (I) or a pharmaceutically acceptable salt thereof, and a pharmaceutically acceptable carrier;

The hvention also provides the use of a compound or salt according to formula (I) for the manufacture of a medicament.

The invention further relates to combination therapies wherein a compound of the invention, or a pharmaceutically acceptable salt thereof, or a pharmaceutical composition or formulation comprising a compound of the invention, is administered concurrently or sequentially or as a combined preparation with another therapeutic agent or agents, for the treatment of one or more of the conditions listed. Such other therapeutic agent(s) may be administered prior to, simultaneously with, or following the administration of the compounds of the invention.

The invention also provides compounds, pharmaceutical compositions, and kits for the treatment of a disorder or condition that may be treated by modulating cannabinoid CB₁ receptors, the method comprising administering to a patient in need of such treatment a compound of formula (I) or a pharmaceutically acceptable salt thereof.

The compounds of the invention possess cannabinoid CB₁ receptor modulating activity. The (ant)agonizing activities of the compounds of the invention is readily demonstrated, for example, using one or more of the assays described herein or known in the art.

The invention also provides methods of preparing the compounds of the invention and the intermediates used in those methods.

The compounds of the present invention may contain one or more asymmetric centers and can thus occur as racemates and racemic mixtures, single enantiomers, diastereomeric mixtures and individual diastereomers.

All compounds of the present invention do contain at least one chiral centre (at the 4-position of the4,5-dihydropyrazole ring). Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within the ambit of this invention. The present invention is meant to comprehend all such isomeric forms of these compounds. The independent syntheses of these diastereomers or their chromatographic separations may be achieved as known in the art by appropriate modification of the methodology disclosed herein. Their absolute stereochemistry may be determined by X-ray crystallography of crystalline products or crystalline intermediates which are derivatized, if necessary, with a reagent containing an asymmetric center of known absolute configuration. If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography. The coupling reaction is often the formation of salts using an enantiomerically pure acid or base, such as for example (-)-di-p-toluoyl-D-tartaric acid and/or (+)-di-p-toluoyl-L-tartaric acid The diasteromeric derivatives may then be converted to the pure enantiomers by cleavage of the added chiral residue. The racemic mixture of the compounds can also be separated directly by chromatographic methods utilizing chiral stationary phases, which methods are well known in the art. Alternatively, any enantiomer of a compound may be obtained by stereoselective synthesis using optically pure starting materials or reagents of known configuration by methods well known in the art.

Cis and trans isomers of the compound of formula (I) or a pharmaceutically acceptable salt thereof are also within the scope of the invention, and this also applies to tautomers of the compounds of formula (I) or a pharmaceutically acceptable salt thereof.

Some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

Isotopically-labeled compound of formula (I) or pharmaceutically acceptable salts thereof, including compounds of formula (I) isotopically-labeled to be detectable by PET or SPECT, are also included within the scope of the invention, and same applies to compounds of formula (I) labeled with [¹³C]-, [¹⁴C]-, [³H]-, [¹⁸F]-, [¹²⁵l]- or other isotopically enriched atoms, suitable for receptor binding or metabolism studies.

### DEFINITIONS OF CHEMICAL TERMS

The term **'alkyl'** refers to straight or branched saturated hydrocarbon radicals. **'Alkyl(C₁₋₃)'** for example, means methyl, ethyl, n-propyl or isopropyl, and **alkyl(C₁₋₄)'** means 'methyl, ethyl, *n*-propyl, isopropyl, butyl, *sec-*butyl, isobutyl or *tert*-butyl'. The term '**alkenyl**' denotes straight or branched hydrocarbon radicals having one or more carbon-carbon double bonds, such as vinyl, allyl, butenyl, etc.. In **'alkynyl'** groups the straight or branched hydrocarbon radicals have one or more carbon-carbon triple bonds, such as ethynyl, propargyl, 1-butynyl, 2-butynyl, etc.. The term **'acyl'** means alkyl(C₁₋₃) carbonyl, arylcarbonyl or aryl-alkyl(C₁₋₃)carbonyl. **'Hetero'** as in 'heteroalkyl, heteroaromatic' etc. means either N, O or S. **'heteroalkyl'** includes alkyl groups with heteroatoms in any position, thus including Nbound, O-bound or S-bound alkyl groups. The abbreviation **'aryl'** means monocyclic or fused bicyclic aromatic or heteroaromatic groups, which heteroaromatic groups contain one or two heteroatoms selected from the group (N, O, S). Aryl groups include but are not limited to furyl, thienyl, pyrrolyl, oxazolyl, thiazolyl, imidazolyl, pyrazolyl, isoxazolyl, isothiazolyl, pyridyl, pyridazinyl, pyrimidinyl, pyrazinyl, phenyl, indazolyl, indolyl, indolizinyl, isoindolyl, benzo[b]furanyl, 1,2,3,4-tetrahydronaphtyl, 1,2,3,4-tetrahydroisoquinolinyl, indanyl, indenyl, benzo[b]thiophenyl, 2,3-dihydro-1,4-benzodioxin-5-yl, benzimidazolyl, benzo-thiazolyl, quinolinyl, isoquinolinyl, phtalazinyl, quinazolinyl, quinoxalinyl, 1,8-naphthyridinyl, naphthyl. The abbreviation **'halogen'** means chloro, fluoro, bromo or iodo. The abbreviation **'C_{3-5'}cycloalkyl'** means cyclopropyl, cyclobutyl, cyclopentyl, cyclohexyl, cyclopheptyl or cyclooctyl. The abbreviation **'C₅₈ heterocycloalkyl'** refers to (N, O S) heteroatom containing rings including but not limited to piperidinyl, morpholinyl, azepanyl, pyrrolidinyl, thiomorpholinyl, piperazinyl, tetrahydrofuryl, tetrahydropyranyl. The abbreviation 'C₅₋₁₀ **bicycloalkyl group'** refers to carbo-bicyclic ring systems including but not limited to bicydo[2.2.1]heptanyl, bicyclo[3.3.0]octanyl or the bicyclo[3.1.1 ]heptanyl group. The abbreviation 'C₆₋₁₀ **tricycloalkyl group'** refers to carbo-tricyclic ring systems including but not limited to the 1 -adamantyl, noradamantyl or the 2-adamantyl group. The abbreviation 'C₈₋₁₁ **tetracycloalkyl group'** refers to carbo-tetracyclic ring systems including but not limited to the cubyl, homocubyl or bishomocubyl group.

The terms **"oxy", "thio"** and **"carbo"** as used herein as part of another group respectively refer to an oxygen atom, a sulphur atom and a carbonyl (C=O) group, serving as linker between two groups, such as for instance hydroxyl, oxyalkyl, thioalkyl, carboxyalkyl, etc. The term **"amino"** as used herein alone or as part of another group refers to a nitrogen atom that may be either terminal or a linker between two other groups, wherein the group may be a primary, secondary or tertiary (two hydrogen atoms bonded to the nitrogen atom, one hydrogen atom bonded to the nitrogen atom and no hydrogen atoms bonded to the nitrogen atom, respectively) amine. The terms **"sulfinyl"** and **"sulfonyl"** as used herein as part of another group respectively refer to an-SO- or an - SO₂- group.

As used herein, unless otherwise noted, the term **"leaving group"** shall mean a charged or uncharged atom or group which departs during a substitution or displacement reaction. Suitable examples include, but are not limited to, Br, Cl, I, mesylate, tosylate, and the like.

**N-oxides** of the compounds mentioned above are in the scope of the present invention. Tertiary amines may or may not give rise to Oxide metabolites. The extent to what N-oxidation takes place varies from trace amounts to a near quantitative conversion. Noxides may be more active than their corresponding tertiary amines or less active. Whilst N-oxides are easily reduced to their corresponding tertiary amines by chemical means, in the human body this happens to varying degrees. Some N-oxides undergo nearly quantitative reductive conversion to the corresponding tertiary amines, in other cases the conversion is a mere trace reaction or even completely absent (Bickel, 1969).

### DEFINITIONS OFOTHER TERMS

With reference to substituents, the term **"independently"** means that when more than one of such substituents is possible, such substituents may be the same or different from each other.

To provide a more concise description, some of the quantitative expressions given herein are not qualified with the term **'about".** It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

Any compound that can be converted in vivo to provide the bioactive agent (i.e., the compound of formula (I) ) is a **prodrug** within the scope and spirit of the application. Prodrugs are therapeutic agents which are inactive per se but are transformed into one or more active metabolites. Thus, in the methods of treatment of the present invention, the term "administering" shall encompass the treatment of the various disorders described with the compound specifically disclosed or with a compound which may not be specifically disclosed, but which converts to the specified compound in vivo after administration to the patient. Prodrugs are bioreversible derivatives of drug molecules used to overcome some barriers to the utility of the parent drug molecule. These barriers include, but are not limited to, solubility, permeability, stability, presystemic metabolism and targeting limitations (Bundgaard, 1985; King, 1994; Stella, 2004; Ettmayer, 2004; Järvinen, 2005). Prodrugs, i.e. compounds which when administered to humans by any known route, are metabolised to compounds having formula (I) , belong to the invention. In particular this relates to compounds with primary or secondary amino or hydroxy groups. Such compounds can be reacted with organic acids to yield compounds having formula (I) wherein an additional group is present which is easily removed after administration, for instance, but not limited to amidine, enamine, a Mannich base, a hydroxylmethylene derivative, an O-(acyloxymethylene carbamate) derivative, carbamate, ester, amide or enaminone.

The term **"composition"** as used herein is intended to encompass a product comprising specified ingredients in predetermined amounts or proportions, as well as any product which results, directly or indirectly, from combination of the specified ingredients in the specified amounts. This term in relation to pharmaceutical compositions is intended to encompass a product comprising one or more active ingredients, and an optional carrier comprising inert ingredients, as well as any product which results, directly or indirectly, from combination, complexation or aggregation of any two or more of the ingredients, or from dissociation of one or more of the ingredients, or from other types of reactions or interactions of one or more of the ingredients. In general, pharmaceutical compositions are prepared by uniformly and intimately bringing the active ingredient into association with a liquid carrier or a finely divided solid carrier or both, and then, if necessary, shaping the product into the desired formulation. In the pharmaceutical composition the active object compound is included in an amount sufficient to produce the desired effect upon the process or condition of disease s. Accordingly, the pharmaceutical compositions of the present invention encompass any composition made by admixing a compound of the present invention and a pharmaceutically acceptable carrier. By "pharmaceutically acceptable" it is meant the carrier, diluent or excipient must be compatible with the other ingredients of the formulation and not deleterious to the recipient thereof.

**Dose.** The affinity of the compounds of the invention for cannabinoid CB₁ receptors was determined as described below. From the binding affinity measured for a given compound of formula (I) , one can estimate a theoretical lowest effective dose . At a concentration of the compound equal to twice the measured Kᵢ-value, nearly 100% of the cannabinoid CB₁ receptors likely will be occupied by the compound. Converting that concentration to mg of compound per kg of patient yields a theoretical lowest effective dose, assuming ideal bioavailability. Pharmacokinetic, pharmaco-dynamic, and other considerations may alter the dose actually admin istered to a higher or lower value. The dose of the compound to be administered will depend on the relevant indication, the age, weight and sex of the patient and may be determined by a physician. The dosage will preferably be in the range of from 0.01 mg/kg to 10 mg/kg. The typical daily dose of the active ingredients varies within a wide range and will depend on various factors such as the relevant indication, the route of administration, the age, weight and sex of the patient and may be determined by a physician. In general, oral and parenteral dosages will be in the range of 0.1 to 1,000 mg per day of total active ingredients.

The term **"therapeutically effective amount"** as used herein refers to an amount of a therapeutic agent to treat or prevent a cond ition treatable by administration of a composition of the application. That amount is the amount sufficient to exhibit a detectable therapeutic, preventative or ameliorative response in a tissue system, animal or human. The effect may include, for example, treatment or prevention of the conditions listed herein. The precise effective amount for a subject will depend upon the subject's size and health, the nature and extent of the condition being treated, recommendations of the treating physician (researcher, veterinarian, medical doctor or other clinician), and the therapeutics or combination of therapeutics selected for administration. Thus, it is not useful to specify an exact effective amount in advance.

The term **"pharmaceutically acceptable salt"** refers to those salts which are, within the scope of sound medical judgment, suitable for use in contact with the tissues of humans and lower animals without undue toxicity, irritation, allergic response, and the like, and are commensurate with a reasonable benefit/risk ratio. Pharmaceutically acceptable salts are well-known in the art. The salts can be prepared *in situ* during the final isolation and purification of the compounds of the invention, or separately by reacting compounds of the invention with pharmaceutically acceptable non-toxic bases or acids, including inorganic or organic bases and inorganic or organic acids.

The term **"treatment"** as used herein refers to any treatment of a mammalian, preferably human condition or disease, and includes: (1) preventing the disease or condition from occurring in a subject which may be predisposed to the disease but has not yet been diagnosed as having it, (2) inhibiting the disease or condition, i.e., arresting its development, (3) relieving the disease or condition, i.e., causing regression of the condition, or (4) relieving the conditions caused by the disease, i.e., stopping the symptoms of the disease.

The term **medical therapy** as used herein is intended to include prophylactic, diagnostic and therapeutic regimens carried out in vivo or ex vivo on humans or other mammals.

The term "subject" as used herein, refers to an animal, preferably a mammal, most preferably a human, who has been the object of treatment, observation or experiment.

### ABBREVIATIONS

- ACN: acetonitrile
- API-ES: atmospheric pressure ionization - electron spray
- BOC: *tert*-butoxycarbonyl
- BSA: bovine serum albumin
- CB₁: cannabinoid receptor subtype-1
- CB₂: cannabinoid receptor subtype-2
- CHO: Chinese Hamster Ovary (cells)
- CNS: central nervous system
- CUR: curtain gas
- DF: deflector voltage
- DIPEA.: *N*,*N*-diisopropylethylamine
- DMAP: 4-dimethylaminopyridin
- DMEM: Dulbecco's Modified Eagle's Medium
- DMSO: dimethylsulfoxide
- DSC: differential scanning calorimetry
- EDCl: 1-(3-diimethylaminopropyl)-3-ethylcarbodiimide hydrochloride
- EP: entrance potential
- FP: focusing potential
- g: gram(s)
- h: hour(s)
- HOBt: N-hydroxybenzotriazole
- HPLC: high performance liquid chromatography
- IBMX: 3-isobutyl-1-methylxanthine
- IS: ionspray voltage
- MeOH: methanol
- mg: milligram(s)
- min: minute(s)
- ml: milliliter(s)
- m.p.: melting point *c.q.* melting range
- MTBE: methyl *tert*-butylether
- NEB: nebulizer gas
- NMM: N-methylmorpholine
- P BS: phosphate buffered saline
- PET: positron emission tomography
- R_{f}: retention factor (thin layer chromatography)
- Rₜ: retention time (LC/MS)
- RT: room temperature
- SPECT: single photon emission computed tomography
- TEM: temperature
- THF: tetrahydrofuran

### EXAMPLES

### EXAMPLE 1: MATERIALS AND METHODS

¹H NMR spectra were recorded on either a Varian 300 MHz, instrument, a Varian UN400 instrument (400 MHz) using DMSO-d₆ or CDCl₃ as solvents with tetramethylsilane as an internal standard. ¹³C NMR spectra were recorded on a Varian UN400 instrument using CDCl₃ as solvent. Chemical shifts are given in ppm (δ scale) downfield from tetramethylsilane. Coupling constants (J) are expressed in Hz. Flash chromatography was performed using silica gel 60 (0.040-0.063 mm, Merck). Column chromatography was performed using silica gel 60 (0.063-0.200 mm, Merck). Sepacore chromatographic separations were carried out using Supelco equipment, VersaFLASH^{™} columns, VersaPak^{™} silica cartridges, Büchi UV monitor C-630, Büchi Pump module G605, Büchi fraction collector C-660 and Büchi pump manager G615. Melting points were recorded on a Büchi B545 melting point apparatus or determined by DSC (differential scanning calorimetry) methods. Optical rotations ([α]_{D}) were measured on an Optical Activity polarimeter. Specific rotations are given as deg/dm, the concentration values are reported as g/100 mL of the specified solvent and were recorded at 23 °C.

**LC-MS instrumentation for method A and method B:** Hardware: An Agilent 1100 LC/MS system was used consisting of:

| | |
|---|---|
| G1322A | solvent degasser |
| G1311A | quaternary pump |
| G1313A | auto sampler |
| G1316A | column oven + switch |
| G1315B | DAD + standard flow cell |
| G1946D | (SL)-MSD |

### Method A :

| | |
|---|---|
| Column: | Discovery C₁₈ (150 x 4.6 mm) Supelco |
| Mobile phase: | 100% Solution B (16 min) |
| Flow rate : | 1.0 ml/min. |
| UV wavelength: | 216 & 251 nm |
| Sample: | ∼1 mg/ml in MeOH |
| Injected volume: | 3 µl |
| Temperature: | 22 °C |
| Mass detection . : | API-ES positive |
| Solution B: | 9.65 g Ammoniumacetate; 250 ml H₂O; 1350 ml MeOH; 900 ml Acetonitrile |

### Method B :

| | |
|---|---|
| Column: | Agilent Zorbax Extend-C18 (4.6* 50 mm; 3.5µm) |
| Mobile phase: | Gradient: 0 - 3 minutes: Solution A/Solution B = 20/80 (v/v)). > 3 minutes : Solution B, unless indicated otherwise. |
| Flow rate : | 1.0 ml/min. |
| UV wavelength : | 218 and 250 nm |
| Sample: | ∼1 mg/ml in MeOH |
| Injected volume: | 1.0 µl |
| Temperature: | 22°C |
| Mass detection : | API-ES positive & negative |
| | |
| Solution A: | 9.65 g Ammoniumacetate; 2250 ml H₂O; 150 ml MeOH; 100 ml Acetonitrile |
| Solution B: | 9.65 g Ammoniumacetate; 250 ml H₂O; 1350 ml MeOH; 900 ml Acetonitrile |

### Preparative LC/MS instrumentation and procedure for method C

Sciex API 150 EX masspectrome ter with electron spray,
2 Shimadzu LC8A LC pump,
Shimadzu SCL-10A VP system controller,
Shimadzu SPD-10A VP UV meter,
Gilson 215 injector/collector,

| | |
|---|---|
| Column | : Phenomenex Luna C18 (2) |
| | :150x21.2x5µ |
| Eluant | : A 100% Water + 0.1 % Formic acid on pH=3 : B100% Acetonitrile + 0.1 % Formic acid |
| Injection | : 2.5 ml |
| Splitter | : 1 to 50,000 with a make -up flow of 0.2 ml/min |
| | (25% H₂O/75% ACN met 0.25% HCOOH) |
| MS scan | : from 100- 900 amu step 1 amu scan time 1 sec. |
| Method | : Flow rates and gradient profiles. |

| **Total Time (min)** | **Flow rate (ml/min)** | **A% (v/v)** | **B % (v/v)** |
|---|---|---|---|
| | | | |
| 0 | 5 | 95 | 5 |
| 2 | 5 | 95 | 5 |
| 2.1 | 20 | 95 | 5 |
| 12 | 20 | 0 | 100 |
| 14 | 20 | 0 | 100 |
| 14.5 | 20 | 95 | 5 |
| 15 | 20 | 95 | 5 |

### Preparative LC/MS instrumentation and procedure for method D

### Analytical 3 minutes method

The LC-MS system consists of 2 Perkin-Elmer series 200 micro pumps. The pumps are connected to each other by a 50 ul tee mixer. The mixer is connected to the Gilson 215 auto sampler.

The LC method is :

| step | total time | flow (ul/min) | A(%) | B(%) |
|---|---|---|---|---|
| 0 | 0 | 2300 | 95 | 5 |
| 1 | 1.8 | 2300 | 0 | 100 |
| 2 | 2.5 | 2300 | 0 | 100 |
| 3 | 2.7 | 2300 | 95 | 5 |
| 4 | 3.0 | 2300 | 95 | 5 |

| | | | | |
|---|---|---|---|---|
| A= 100% Water with 0.2% HCOOH and 10mmol NH4COOH pH= +/- 3 B= 100% ACN with 0.2% HCOOH | | | | |

The auto sampler has a 2 ul injection loop. The auto sampler is connected to a Waters Atlantis C18 30*4.6 mm column with 3 um particles. The column is thermo stated in a Perkin-Elmer series 200 column oven at 40 degrees Celsius. The column is connected to an Applied biosystems ABI 785 UV meter with a 2.7 ul flow cel. The wavelength is set to 254 nm. The UV meter is connected to a Sciex API 150EX mass spectrometer. The mass spectrometer has the following parameters:
Scan rang:150-900 Amu
Polarity: positive
Scan mode: profile
Resolution Q1: UNIT
Step size: 0.10 amu
Time per scan: 0.500 sec
NEB: 10
CUR: 10
IS: 5200
TEM:325
DF: 30
FP: 225
EP:10

The light scattering detector is connected to the Sciex API 150. The light scattering detector is a Polymerlabs PLS21 00 operating at 70 °C and 1.7 bar N₂ pressure. The complete systems is controlled by a Dell precision 370 computer operating under Windows 2000.

### EXAMPLE 2: GENERAL ASPECTS OF SYNTHESES

Pyrazoline derivatives can be obtained by published methods (Barluenga, 1999 *(and references cited therein);* Wang, 2003). The synthesis of compounds having formula (I) is outlined in Scheme 1. Ketone derivatives of general formula (II) can be made by various methods known to those skilled in the art. Examples are the application of a so-called Weinreb amide RC(=O)N(OCH₃)CH₃ which can be reacted with a Grignard reagent R₂CH₂MgCl or R₂CH₂Mg Br or a reaction of RMgBr or RMgCl with a Weinreb amide of general formula R₂CH₂C(=O)N(OCH₃)CH₃. Alternatively, a Grignard reagent R₂CH₂MgCl or R₂CH₂MgBr can be reacted with a cyanide analog R₁CN, followed by acidic hydrolysis, for example by using hydrochloric acid. A ketone derivative of general formula (II) wherein R and R₂ have the abovementioned meaning can be reacted with formaldehyde in the presence of an amine, such as piperidine and an acid, for example acetic acid, in an inert organic solvent such as methanol to give a compound of general formula (III), wherein R and R₂ have the abovementioned meaning. This reaction can be classified as a so-called Mannich reaction, followed by elimination of the applied amine. Alternatively, a ketone derivative of general formula (II) wherein R and R₂ have the abovementioned meaning can be reacted with N,N,N',N'-tetramethyldiaminomethane in acetic anhydride to give a compound of general formula (III), wherein R and R₂ have the abovementioned meaning (Ogata, 1987^{a}, 1987^{b}). The compound of general formula (III) can be reacted with hydrazine or hydrazine hydrate in the presence of an inert organic solvent such as ethanol to give a pyrazoline derivative of general formula (IV), wherein R and R₂ have the abovementioned meaning and R₁ represents a hydrogen atom. Alternatively, the compound of general formula (III) can be oxidized with an oxidizing reagent such as hydrogen peroxide to give a epoxyketone derivative of general formula (V), wherein R and R₂ have the abovementioned meaning. A compound of general formula (V) can be reacted with hydrazine or hydrazine hydrate in the presence of an inert organic solvent such as ethanol to give a pyrazoline derivative of general formula (IV), wherein R and R₂ have the abovementioned meaning and R₁ represents a hydroxy group.

A compound of general formula (IV) can be reacted with a carboxylic acid R₃-CO₂H wherein R₃ has the abovementioned meaning in the presence of an so-called activating reagent or coupling reagent in an inert organic solvent such as dichloromethane to give a pyrazoline derivative of general formula (I), wherein n=0, A represents a carbonyl group and all other symbols have the meanings as given above. Additional information on activating aid coupling methods of amines to carboxylic acids can be found in the literature (Bodanszky and Bodanszky, 1994; Akaji, 1994; Albericio, 1997; Montalbetti and Falque, 2005).

Alternatively, a compound of general formula (IV) wherein R, R₁ and R₂ have the abovementioned meaning can be reacted with an acid chloride R₃-COCl wherein R₃ has the abovementioned meaning to give a pyrazoline derivative of general formula (I), wherein n=0, A represents a carbonyl group and all other symbols have the meanings as given above.

A compound of general formula (IV) wherein R, R₁ and R₂ have the abovementioned meaning can be reacted with an isocyanate derivative R₃-N=C=O (VII) wherein R₃ has the abovementioned meaning in the presence of an inert organic solvent such as diethyl ether to give a pyrazoline-1-carboxamide derivative of general formula (I), wherein n=1 and R₄ represents H, A represents a carbonyl group and all other symbols have the meanings as given above. Isocyanates R₃N=C=O can also be prepared in situ from the corresponding amine R₃-NH₂ and a so-called carbonyl donor such as phosgene, diphosgene (trichloromethyl chloroformate) or triphosgene (bis(trichloromethyl) carbonate). Alternatively, isocyanates R₃-N=C=O can be prepared from the corresponding carboxylic acid R₃-COOH via the acylazide R₃-CON₃ in a so-called Curtius rearrangement

An amine of general formula R₃R₄NH wherein R₃ and R₄ have the abovementioned meaning can be reacted with a carbonylating agent such as phosgene and the like in the presence of an inert organic solvent such as toluene or benzene to give a compound of general formula (VI), wherein L represents a so-called leaving group such as chloride. A compound of general formula (VI) wherein L represents a so-called leaving group can be reacted with a compound of general formula (IV) wherein R, R₁ and R₂ have the abovementioned meaning to give a pyrazoline derivative of general formula (I), wherein n=1 and all other symbols have the meanings as given above. Preferably, a base such as triethylamine or Hünigs base may be added in such reactions. Furthermore, 4-(dimethylamino)pyridine (DMAP) may serve as a catalyst in such reactions.

A compound of general formula (IV) wherein R, R₁ and R₂ have the abovementioned meaning can be reacted with an isothiocyanate derivative R₃-N=C=S (VIIa) wherein R₃ has the abovementioned meaning in the presence of an inert organic solvent such as tetrahydrofuran to give a pyrazoline-1-carbothioamide derivative of general formula (I), wherein n=1 and R₄ represents H, A represents a thiocarbonyl group and all other symbols have the meanings as given above.

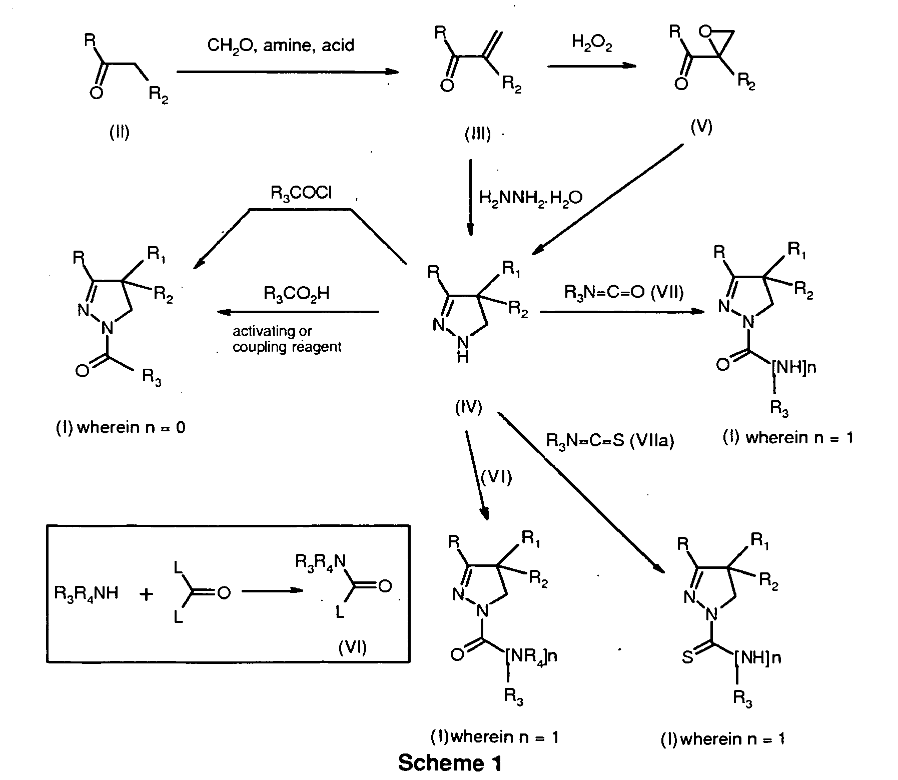

Alternatively, a compound of general formula (IV) wherein R and R₂ have the abovementioned meaning and R₁ represents a hydrogen atom can be reacted with phosgene, diphosgene or triphosgene to give a compound of general formula (VIII) wherein R and R₂ have the abovementioned meaning and R₁ represents a hydrogen atom (Scheme 2). A compound of general formula (VIII) can be reacted with a compound R₃R₄NH to give a pyrazoline-1-carboxamide derivative of general formula (I), wherein n=1 , A represents a carbonyl group.

A compound of general formula (IV) wherein R and F₁ have the abovementioned meaning and R₁ represents a hydrogen atom can be reacted with a sulfonylchloride derivative of general formula F₆SO₂Cl to give a pyrazoline derivative of general formula (I), wherein n=0, A represents a sulfo nyl group and all other symbols have the meanings as given above. Preferably, a base such as triethylamine or Hünigs base (DIPEA) may be added in such reactions.

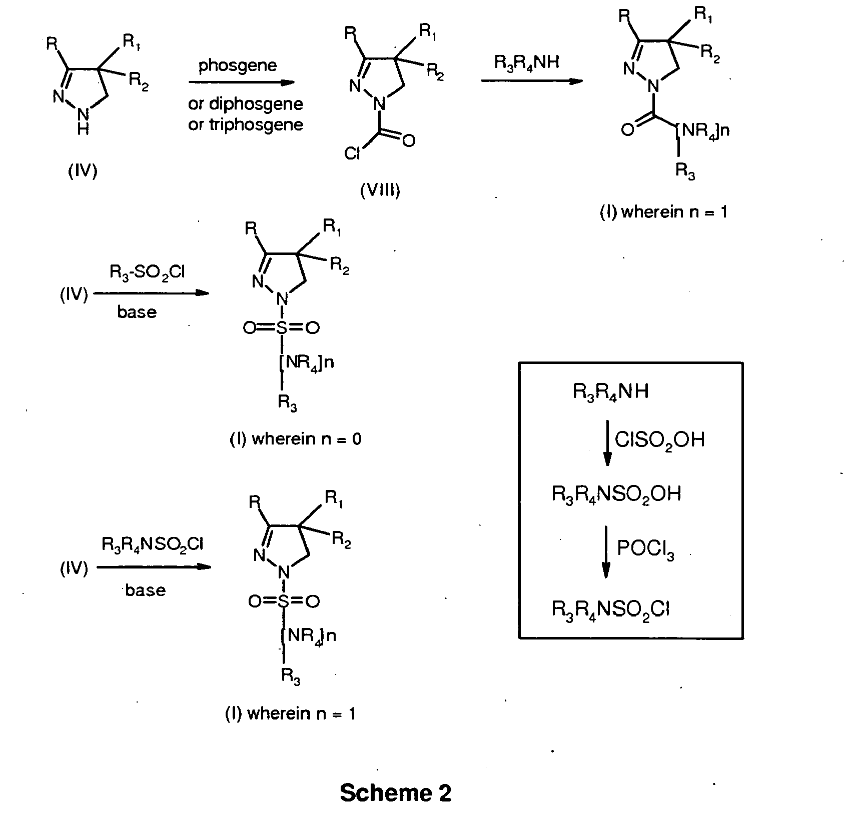

A compound of general formula (IV) wherein R and R₂ have the abovementioned meaning and R₁ represents a hydrogen atom can be reacted with a compound of general formula R₃R₄NSO₂Cl to give a pyrazoline derivative of general formula (I), wherein n=1, A represents a sulfonyl group and all other symbols have the meanings as given above. Preferably, a base such as triethylamine or Hünigs base (DIPEA) may be added in such reactions.

A compound of general formula R₃R₄NSO₂Cl can be obtained from a reaction of a sulfamic acid derivative R₃R₄NSO₂OH with a chlorinating agent such as POCl₃ in an inert organic solvent such as dichloromethane. A compound of general formula R₃R₄NSO₂OH can be obtained from a reaction of an amine R₃R₄NH and chlorosulfonic acid in an inert organic solvent such as dichloromethane. Preferably, a base such as triethylamine or Hünigs base (DIPEA) may be added in such a reaction.

The selection of the particular synthetic procedures depends on factors known to those skilled in the art such as the compatibility of functional groups with the reagents used, the possibility to use protecting groups, catalysts, activating and coupling reagents and the ultimate structural features present in the final compound being prepared.

Compounds of the general formula (III), wherein R represent a phenyl group which is substituted with 1-3 substituents Y1 wherein Y1 represents halogen, CF₃, OCF₃ or OCH₃, or R represents a pyridyl or thienyl group, and R₂ represents a n-butyl, n-propyl, 1,1-dimethylpropyl, 1,1-dimethylbutyl, 3,3,3-trifluoropropyl, 4,4,4-trifluorobutyl or 1,1-dimethyl-3,3,3-trifluoropropyl group, or R represent a phenyl group and R₂ represents a 1,1 -dimethylpropyl, 1,1 -dimethylbutyl, 3,3,3-trifluoropropyl, 4,4,4-trifluorobutyl or 1,1-dimethyl-3,3,3-trifluoropropyl group are new. Such compounds are useful in the synthesis of compounds of the general formula (I).

Compounds of the general formula (IV) wherein R and R₁ have the same meanings as given in claim 1 and R₂ represents an phenyl group which may be substituted with 1-5 substituents Y2 which can be the same or different, selected from the group C₁₋₃-alkoxy, hydroxy, trifluoromethyl, trifluoromethylthio, trifluoromethoxy, nitro, amino, mono- or dialkyl (C₁₋₂)-amino, mono- or dialkyl (C₁₋₂)-amido, (C₁₋₃)-alkyl sulfonyl, dimethylsulfamido, C₁₋₃-alkoxycarbonyl, carboxyl, trifluoromethyl-sulfonyl, cyano, carbamoyl, sulfamoyl, ortho-halogen, meta-halogen, ortho-C₁₋₃-alkyl, meta-C₁₋₃alkyl and acetyl, or R₂ represents a thienyl or pyridyl group, which groups may be substituted with one or two substituents Y, which Y group has the meaning as in claim 1, are new. Such compounds are useful in the synthesis of compounds of formula (I).

Compounds of the general formula (VIII) wherein R and R₂ have the same meanings as given hereinabove and R represents hydrogen are new. Such compounds are useful in the synthesis of compounds of the general formula (I) wherein n = 1.

Pharmaceutically acceptable salts may be obtained using standard procedures well known in the art, for example by mixing a compound of the present invention with a suitable acid, for instance an inorganic acid such as hydrochloric acid, or with an organic acid such as fumaric acid.

According to these procedures the compounds described below have been prepared. They are intended to further illustrate the invention in more detail, and therefore are not deemed to restrict the scope of the invention in any way. Other embodiments of the invention will be apparent to those skilled in the art from consideration of the specification and practice of the invention disclosed herein. It is thus intended that the specification and examples be considered as exemplary only.

### EXAMPLE 3: SYNTHESIS AND SPECTRAL DATA OF INTERMEDIATES

### Intermediate II-1

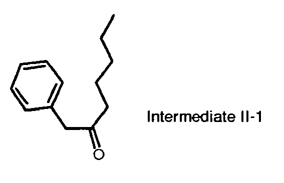

To a magnetically stirred solution of hexanoic add methoxy-methyl-amide (12.2 g, 77 mmol) at 0°C in tetrahydrofuran (THF) was slowly added benzylmagnesium chloride (20 weight percent solution in THF, 90 ml 116 mmol) and the resulting mixture was reacted for two hours. The reaction mixture was poured in excess aqueous hydrochloric acid (4N solution) and extracted with tert-butyl-methyl ether (MTBE). Concentration in vacuo, followed by flash chromatographic purification (heptane/ethylacetate = 40/1 (v/v)) gave 1-phenylheptan-2-one (Intermediate II-1 ) (116 gram) as an oil; ¹H-NMR (300 MHz, CDCl₆) δ 0.86 (t, J = 7, 3H), 1.20-1.27 (m, 4H), 1.52-1.60 (m, 2H), 2.40-2.46 (m, 2H), 3.68 (s, 2H), 7.18-7.33 (m, 5H).

### Intermediate II-2

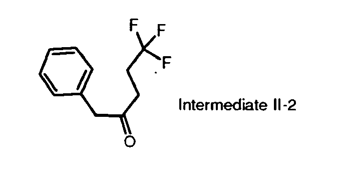

4,4,4-Trifluoro-N-methoxy-N-methylbutyramide (7.68 g) was obtained in 87 % yield as an oil from the reaction of 4,4,4-trifluorobutyric acid (6.77 g, 0.0477 mol) with N-methyl-N-methoxy-amine.HCl in the presence of N-hydroxybenzotriazole (HOBt), 1-(3-dimethylaminopropyl)-3-ethylcarbodi-imide.HCl (EDCl) and N-methylmorpholine (NMM) in dichloromethane as the solvent (room temperature, 16 hours). ¹H-NMR (400 MHz, CDCl₃) δ2.40-2.54 (m, 2H), 2.67-2.73 (m, 2H), 3.20 (s, 3H), 3.71 (s, 3H). 4,4,4-Trifluoro-N-methoxy-N-methylbutyramide (7.68 g) was converted with benzylmagnesium chloride at 0 °C in tetrahydrofuran (THF) analogously to the procedure described for the synthesis of intermediate II-1 to give 6.37 gram (71 %) 5,5,5-trifluoro-1-phenylpentan-2-one (Intermediate II-2). Chromatogra-phic sepacore purification (petroleum ether/diethyl ether = 47/1 (v/v)) was used to purify intermediate II-2. ¹H-NMR (400 MHz, CDCl₃) δ 2.31-2.44 (m, 2H), 2.68-2.75 (m, 2H), 3.73 (s, 2H), 7.18-7.38 (m, 5H).

### Intermediate II-3

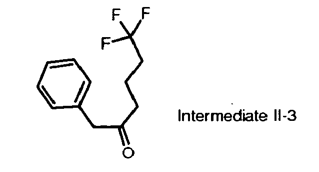

Intermediate II-3 (6,6,6-trifluoro-1-phenyl-hexan-2-one) was prepared analogously to intermediate II-1 from 5,5,5-trifluoropentanoic acid methoxy-methyl-amide and benzylmagnesium chloride (20 weight percent solution in THF) at 0 °C in tetrahydrofuran as an oil;¹H-NMR (400 MHz, CDCl₃) δ 1.75-1.85 (m, 2H), 1.98-2.11 (m, 2H), 2.55 (t, J = 7, 2H), 3.69 (s, 2H), 7.18-7.22 (m, 2H), 7.2&7.37 (m, 3H). 5,5,5-Trifluoropentanoic acid methoxy-methyl-amide: ¹H-NMR (400 MHz, CDCl₃) δ 1.86-1.95 (m, 2H), 2.11-2.24 (m, 2H), 2.53 (br t, J = 7, 2H), 3.19 (s, 3H), 3.69 (s, 3H). 5,5,5-Trifluoropentanoic acid methoxy-methyl-amide was obtained from the reaction of 5,5,5-trifluoropentanoic acid and N-methyl-N-methoxy-amine.HCl in the presence of N-hydroxybenzotriazole, 1-(3-dimethylaminopropyl)-3-ethylcarbodiimide.HCl and N-methylmorpholine in dichloromethane.

### Intermediate II-4

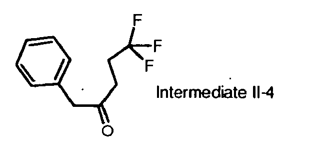

Intermediate II-4 (6,6,6-trifluoro-1-phenyl-pentan-2-one)was prepared analogously to intermediate II-1, from 4,4,4-trifluoro -N-methoxy-N-methyl-butyramide and benzylmagnesium chloride (20 weight percent solution in THF) at 0 °C in tetrahydrofuran as an oil; ¹H-NMR (400 MHz, CDCl₃) δ 2.31-2.44 (m, 2H), 2.71 (t, J = 7, 2H), 3.73 (s, 2H), 7.18-7.22 (m, 2H), 7.26-7.38 (m, 5H).

4,4,4-Trifluoro-N-methoxy-N-methyl-butyramide: ¹H-NMR (400 MHz, CDCl₃ δ 2.41 - 2.53 (m, 2H), 2.70 (br t, J = 7, 2H), 3.20 (s, 3H), 3.71 (s, 3H). 4,4,4-Trifluoro-N-methoxy-N-methyl-butyramide was obtained from the reaction of 4,4,4-trifluorobutyric acid and N-methyl-N-methoxy-amine.HCl in the presence of N-hydroxybenzotriazole, 1-(3-dimethylaminopropyl)-3-ethylcarbodiimide.HCl and N-methylmorpholine in dichloromethane.

### Intermediate II-5

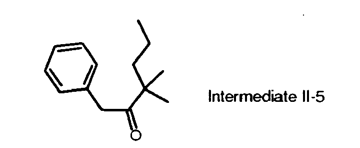

Intermediate II-5 (3,3-dimethyl-1-phenyl-hexan-2-one) was prepared analogously to intermediate II-1 from 2,2-dimethylpentanoic acid methoxy-methyl-amide and benzylmagnesium chloride (20 weight percent solution in THF) at 0 °C in tetrahydrofuran as an oil; ¹H-NMR (400 MHz, CDCl₃ δ 0.89 (t, J=7, 3H), 1.14-1.23 (m, 8H), 1.53-1.60 (m, 2H), 3.76 (s, 2H), 7.15-7.33 (m, 5H).

2,2-Dimethylpentanoic acid methoxy-methyl-amide: ¹H-NMR (400 MHz, CDCl₃) δ 0.90 (t, J=7, 3H), 1.20-1.29 (m, 8H), 1.55-1.60 (m, 2H), 3.17 (s, 3H), 3.67 (s, 3H). 2,2-Dimethylpentanoic acid methoxy-methyl-amide was obtained from the reaction of 2,2-dimethylpentanoic acid and Nmethyl-N-methoxy-amine.HCl in the presence of N-hydroxybenzotriazole, 1-(3-dimethylaminopropyl)-3-ethylcarbodiimide.HCl and N-methylmorpholine in dichloromethane.

### Intermediate II-6

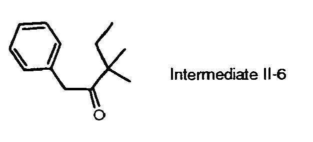

Intermediate II-6 (3,3-dimethy)-1-phenyl-pentan-2-one) was prepared analogously to intermediate II-1 from 2,2,N-trimethyl-N-methoxy-butyramide and benzylmagnesium chloride (20 weight percent solution in THF) at 0 °C in tetrahydrofuran as an oil; ¹H-NMR (400 MHz, CDCl₃) δ 0.81 (t, J=7, 3H), 1.15 (s, 6H), 1.64 (q, J = 7.5, 2H), 3.76 (s, 2H), 7.15-7.33 (m, 5H).

2,2,N-Trimethyl-N-methoxy-butyramide: ¹H-NMR (400 MHz, CDCl₃ δ 0.85 (t, J=7, 3H), 1.21 (s, 6H), 1.61 -1.69 (m, 2H), 3.18 (s, 3H), 3.67 (s, 3H). 2,2,N-Trimethyl-N-methoxy-butyramide was obtained from the reaction of 2,2-dimethylbutyric acid and N-methyl-N-methoxy-amine.HCl in the presence of N-hydroxybenzotriazole, 1-(3-dimethylaminopropyl)-3-ethylcarbodiimide.HCl and N-methylmorpholine in dichloromethane.

### Intermediate II-7

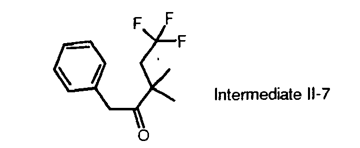

Intermediate II-7 (3,3-dimethyl-5,5,5 -trifluoro-1-phenyl-pentan -2-one) was prepared analogously to intermediate II-1 from 4,4,4-trifluoro-2,2,N-trimethyl-N-methoxy-butyramide and benzylmagnesium chloride (20 weight percent solution in THF) at 0 °C in tetrahydrofuran as an oil; ¹H-NMR (400 MHz, CDCl₃) δ 1.34 (s, 6H), 2.47 (d, J∼12, 1H), 2.52 (d, J∼12, 1H), 3.84 (s, 2H), 7.15 (br d, J∼8, 2H), 7.23-7.36 (m, 3H).

4,4,4-Trifluoro-2,2,N-trimethyl-N-methoxy-butyramide: ¹H-NMR (400 MHz, CDCl₃) δ 1.35 (s, 6H), 2.55 (d, J∼12, 1H), 2.60 (d, J∼12,1H), 3.19 (s, 3H), 3.70 (s, 3H). 4,4,4-Trifluoro-2,2,N-trimethyl-N-methoxy-butyramide was obtained from the reaction of 4,4,4-trifluoro-2,2-dimethylbutyric acid and N-methyl-N-methoxy-amine.HCl in the presence of N-hydroxybenzotriazole, 1 -(3-dimethylaminopropyl)-3-ethylcarbodiimide:HCl and N-methylmorpholine in dichloromethane.

### Intermediate II-8

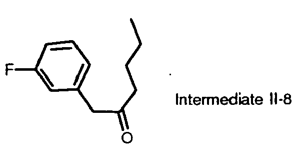

3-Fluorobenzyl bromide (25 g, 0.132 mol) was converted to 3-fluorobenzyl magnesiumbromide in anhydrous diethyl ether (85 ml) using magnesium (3.17 g) in the presence of catalytic amounts of iodine and 1,2-dibromoethane. The in situ formed 3-fluorobenzyl magnesium bromide was reacted with pentanenitrile (11 ml) in toluene (100 ml) at 110 °C for 2 hours. After hydrolysis of the formed mixture with concentrated hydrochloric acid (12 N) at 80 °C for 4 hours and subsequent extraction with toluene 1-(3-fluorophenyl)-hexan-2-one was obtained in 86 % yield as an oil. ¹H-NMR (400 MHz, CDCl₃) δ 0.87 (t, J = 7, 3H), 1.22-1.33 (m, 2H), 1.50-1.59 (m, 2H), 2.46 (t, J = 7, 2H), 3.68 (s, 2H), 6.90-7.00 (m, 3H), 7.26 7.32 (m, 2H).

### Intermediate II-9

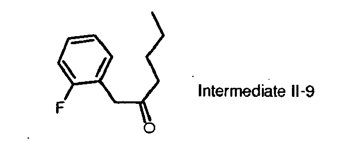

2-Fluorobenzyl bromide was converted to 2-fluorobenzyl magnesiumbromide in anhydrous diethyl ether using magnesium in the presence of catalytic amounts of iodine and 1,2-dibromoethane analogously to the procedure described for the synthesis of Intermediate II-8. The in situ formed 2-fluorobenzyl magnesium bromide was reacted with pentanenitrile in toluene at 110 °C for 2 hours. After hydrolysis of the formed mixture with concentrated hydrochloric acid (12 N) at 80 °C for 20 hours 1-(2-fluorophenyl)-hexan-2-one was obtained in 70 % yield as an oil. ¹H-NMR (400 MHz, CDCl₃) δ 0.88 (t, J = 7, 3H), 1.24-1.35 (m, 2H), 1.53-1.62 (m, 2H), 2.48 (t, J = 7, 2H), 3.72 (br s, 2H), 6.98-7.28 (m, 4H).

### Intermediate II-10

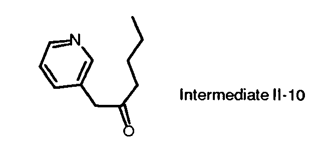

To a magnetically stirred solution of N-methoxy-N-methyl-2-(pyridin-3-yl)acetamide (12 g, 67 mmol) at -15 °C in tetrahydrofuran (THF) was slowly added n-butylmagnesium chloride (2 M solution in THF, 75 ml, 150 mmol) and the resulting mixture was reacted for 1 hour at -15 °C and successively stirred at room temperature overnight. The reaction mixture was poured in excess aqueous NH₄Cl and extracted twice with ethylacetate. Concentration in vacuo, followed by sepacore chromatographic purification (ethylacetate) gave 1-(pyridin-3-yl)hexan-2-one (Intermediate II-10) (5.95 gram, 50 % yield) as an oil; ¹H-NMR (400 MHz, CDCl₆) δ 0.89 (t, J = 7, 3H), 1.24-1.35 (m, 2H), 1.52-1.62 (m, 2H), 2.50 (t, J = 7, 2H), 3.70 (s, 2H), 7.25-7.29 (m, 1H), 7.52-7.57 (m, 1H), 8.45 (br,d, J = 2, 1H), 8.52 (dd, J - 6 and 2, 1H).

### Intermediate III-1

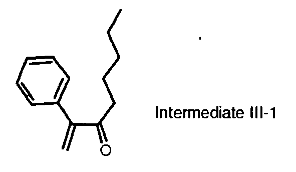

To a magnetically stirred solution of 1 -phenytheptan-2-one (Intermediate II-1) (11.6 gram, 61 mmol) in methanol (100 ml) was added piperidine (1 ml) and acetic acid (1 ml), followed by a formaldehyde solution (20 ml of a 35 % solution in water, 226 mmol) and the resulting mixture was stirred at 55 °C for 60 hours. The reaction mixture was cooled to room temperature, concentrated and taken up in a mixture of MTBE and water. The organic layer was collected, dried over Na₂SO₄, filtered and concentrated to g ive 2-phenyl-oct-1 -en-3-one (Intermediate III-1 ) (11.4 gram) as an oil. Intermediate III-1: ¹H-NMR (400 MHz, CDCl₃) δ 0.80 (t, J = 7, 3H), 1.18-1.30 (m, 4H), 1.54-1.63 (m, 2H), 2.65 (t, J = 7, 2H), 5.80 (s,1H), 6.02 (s, 1H), 7.20-7.32 (m, 5H).

### Intermediate III-2

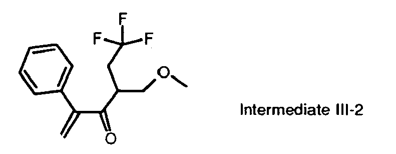

5,5,5-Trifluoro-1-phenylpentan-2-one (Intermediate II-2) was reacted in methanol with piperidine and acetic acid, followed by a formaldehyde solution (35 % solution in water) and the resulting mixture was stirred at 55 °C for 60 hours analogously to the procedure described for the synthesis of intermediate III-1 to give 6,6,6-trifluoro-4-methoxymethyl-2-phenyl-hex-1-en-3-one (intermediate III-2) in 16 % yield. Chromatographic sepacore purification (petroleum ether/diethyl ether = 19/1 (v/v)) was used to purify intermediate III-2

¹H-NMR (400 MHz, CDCl₃) ? 2.28-2.42 (m, 1H), 2.70-2.85 (m, 1H), 3.29 (s, 3H), 3.47-3.60 (m, 2H), 3.68-3.76 (m, 1H), 6.01 (s, 1H), 6.13 (s, 1H), 7.28-7.40 (m, 5H).

### Intermediate III-3

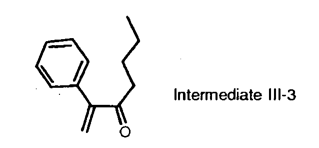

Intermediate III-3 (2-phenyl-hept-1-en-3-one) was prepared analogously to intermediate III-1, from 1-pheny)hexan-2-one, piperidine, acetic acid and formaldehyde solution (35 % solution in water) at 55 °C for 60 hours. Intermediate III-3: ¹H-NMR (400 MHz, CDCl₃) δ 0.91 (t, J = 7, 3H), 1.30-1.40 (m, 2H), 1.59-1.69 (m, 2H), 2.73 (t, J = 7, 2H), 5.87 (s, 1H), 6.09 (s, 1H), 7.28-7.40 (m, 5H).

### Intermediate III-4

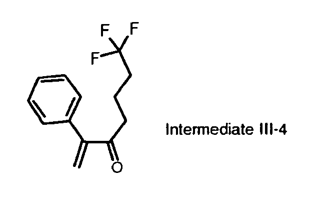

Intermediate III-4 (7,7,7-trifluoro-2-phenyl-hept-1 -en-3-one) was prepared analogously to intermediate III-1, from 6,6,6-tifluoro-1-phenylhexan 2-one, piperidine, acetic acid and formaldehyde solution (35 % solution in water) at 55 °C for 60 hours. Intermediate III-4: ¹H-NMR (400 MHz, CDCl₃) δ 1.89-1.98 (m, 2H), 2.09-2.22 (m, 2H), 2.84 (t, J = 7, 2H), 5.91 (s, 1H), 6.13 (s, 1H), 7.26-7.40 (m, 5H).

### Intermediate III-5

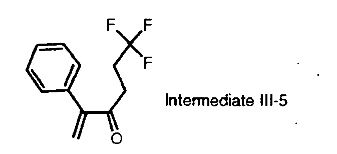

Intermediate III-5 (6,6,6-trifluoro-2-phenyl-hex-1-en-3-one)was prepared analogously to intermediate III-1 with some modifications (temperature and amount of formaldehyde used), from 5,5,5-trifluoro-1-phenylpentan-2-one, piperidine, acetic acid and formaldehyde solution (1.1 molar equivalent CH₂O, 35 % solution in water) at 40 °C for 40 hours in 57 % yield. Purification was performed by sepacore chromatographic purification (petroleum ether/diethyl ether = 39/1 (v/v)). R_{f} = 0.4 (petroleum ether/diethyl ether = 9/1 (v/v)). Intermediate III-5: ¹H-NMR (400 MHz, CDCl₃) δ 2.43-2.56 (m, 2H), 3.03 (t, J = 7, 2H), 5.97 (s, 1H), 6.19 (s, 1H), 7.26-7.40 (m, 5H).

### Intermediate III-6

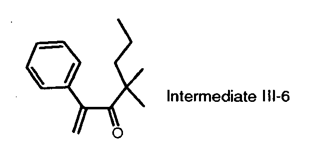

Intermediate III-6 (4,4-dimethy)-2-pheny)-hept-1-en-3-one)was prepared analogously to intermediate III-1, from 3,3-dimethyl -1-phenylhexan -2-one, piperidine, acetic acid and formaldehyde solution (35 % solution in water) at 55°C for 60 hours. Intermediate III-6: ¹H-NMR (400 MHz, CDCl₃) δ 0.83 (t, J = 7, 3H), 1.02 (s, 6H), 1.10-1.19 (m, 2H), 1.40-1.50 (m, 2H), 5.13 (s, 1H), 5.45 (s, 1H), 7.09-7.38 (m, 5H).

### Intermediate III-7

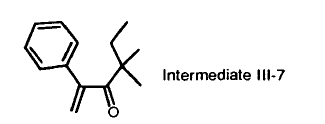

Intermediate III-7 (4,4-dimethyl-2-phenyl-hex-1-en-3 -one) was prepared analogously to intermediate III-1, from 3,3-dimethyl-1-phenylpentan-2-one, piperidine, acetic acid and formaldehyde solution (35 % solution in water) at 55 °C for 60 hours. Intermediate III-7: ¹H-NMR (400 MHz, CDCl₃) δ 0.81 (t, J = 7, 3H), 1.09 (s, 6H), 1.59 (q, J = 7, 2H). 520 (s, 1H), 5.52 (s, 1H), 7.29-7.37 (m, 5H).

### Intermediate III-8

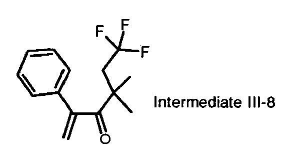

Intermediate III-8 (4,4-dimethyl-6,6,6-trifluoro-2-phenyl-hex-1-en-3-one) was prepared analogously to intermediate III-1, from 3,3-dimethyl-5,5,5-trifluoro-1-phenylpentan-2-one, piperidine, acetic acid and formaldehyde solution (35 % solution in water) at 55°C for 60 hours. Intermediate III-8: ¹H-NMR (400 MHz, CDCl₃) δ 1.22 (s, 6H), 2.49 (d, J∼12, 1H), 2.56 (d, J∼12, 1H), 529 (s, 1H), 5.57 (s, 1H), 7.29-7.39 (m, 5H).

### Intermediate III-9

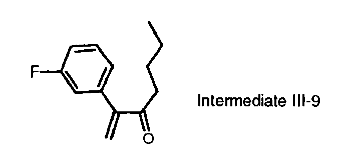

Intermediate III-9 (2-(3-fluorophenyl)-hept-1-en-3-one) was prepared analogously to intermediate III-1, from 1-(3-fluorophenyl)-hexan-2-one, piperidine, acetic acid and formaldehyde solution (35 % solution in water) at 55 °C for 60 hours. Intermediate III-9: ¹H-NMR (400 MHz, CDCl₃) δ 0.93 (t, J = 7, 3H), 1.30-1.41 (m, 2H), 1.60-1.69 (m, 2H), 2.75 (t, J = 7, 2H), 5.93 (s, 1H), 6.15 (s, 1H), 7.00-7.09 (m, 3H) 7.28-7.35 (m, 1H).

### Intermediate III-10

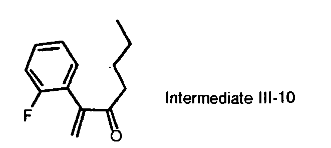

Intermediate III-10 (2-(2-fluorophenyl)-hept-1-en-3-one) was prepared analogously to intermediate III-1, from 1-(2-fluorophenyl)-hexan-2-one, piperidine, acetic acid and formaldehyde solution (35 % solution in water) at 55 °C for 60 hours. Intermediate III-10: ¹H-NMR (400 MHz, CDCl₃) δ 0.91 (t, J = 7, 3H), 1.30-1.40 (m, 2H), 1.59-1.69 (m, 2H), 2.70 (t, J = 7, 2 H), 588 (s, 1H), 6.26 (s, 1H), 6.98-7.37 (m, 4H).

### Intermediate III-11

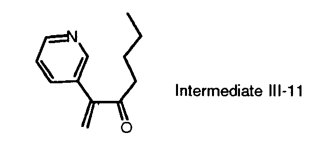

To a magnetically stirred, ice-cooled solution of 1-(pyridin-3-yl)hexan-2-one (6 g, 34 mmol) and N,N,N',N'-tetramethyldiaminomethane (7 ml, 51 mmol) at 0°C was slowly added acetic anhydride (Ac₂O) (4.8 ml, 51 mmol). The resulting mixture was reacted for 30 minutes at 45°C and successively cooled to room temperature. The reaction mixture was poured in excess ice and brine was added. Extraction with ethylacetate (2x) and dichloromethane followed by drying (Na₂SO₄) of the combined organic layers, filtering and concentration in vacuo gave crude product. Subseqentsepacore chromatographic purification (ethylacetate) gave 2-(pyridin-3-yl)hept-1-en-3-one (Intermediate III-11) (3.86 gram, 60 % yield);¹H-NMR (400 MHz, CDCl₆) δ 0.93 (t, J = 7, 3H), 1.32-1.43 (m, 2H), 1.62-1.71 (m, 2H), 2.81 (t, J = 7, 2H), 6.06 (s, 1H), 6.28 (s, 1H), 7.25-7.31 (m, 1H), 7.63-7.68 (m, 1H), 8.53-8.58 (m, 2H).

### Intermediate III-12

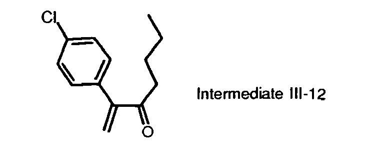

Intermediate III-12 (2-(4-chlororophenyl)-hept-1 en-3one)was prepared analogously to intermediate III-1, from 1-(4-chlorophenyl)-hexan-2-one, piperidine, acetic acid and formaldehyde solution (35 % solution in water) at 55 °C for 60 hours. Intermediate III-12:¹H-NMR (400 MHz, CDCl₃) δ 0.92 (t, J = 7, 3H), 1.30-1.41 (m, 2H), 1.59-1.68 (m, 2H), 2.74 (t, J = 7, 2 H), 5.92 (s, 1H), 6.13 (s, 1H), 7.24 (br d, J = 8, 2H), 7.32 (br d, J = 8, 2H).

### Intermediate III-13

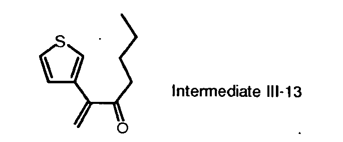

Intermediate III-13 (2-(thien-3-yl)-hept-1-en-3-one) was prepared analogously to intermediate III-1, from 1-(thien-3-yl)hexan-2-one, piperidine, acetic acid and formaldehyde solution (35 % solution in water) at 55 °C for 60 hours. Intermediate III-13: ¹H-NMR (400 MHz, CDCl₃) δ 0.93 (t, J = 7, 3H), 1.31-1.42 (m, 2H), 1.61-1.69 (m, 2H), 2.77 (t, J - 8, 2H), 6.03 (s, 1H). 6D4 (s, 1H), 7.18 (dd, J = 6 and 2, 1H), 7.28 (dd, J - 6 and 3, 1H), 7.51-7.53 (m, 1H).

### Intermediate IV-2

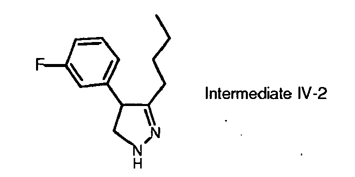

Intermediate IV-2 (3-(n-butyl)-4-(3-fluorophenyl-4,5-dihydropyrazole) was prepared analogously to 3-(n-pentyl)-4-phenyl-4,5-dihydropyrazole (Intermediate IV-1, *see preparation of compound 1),* from 2-(3-fluorophenyl)-hept-1-en-3-one and hydrazine hydrate. Some characteristic pyrazoline ring proton NMR signals: (400 MHz, CDCl₃) δ 3.37 (t, J ∼ 10, 1H, H₅), 3.81 (t, J ∼ 10, 1H, H₅), 3.99 (t, J ∼ 9, 1H, H₄).

### Intermediate IV-3

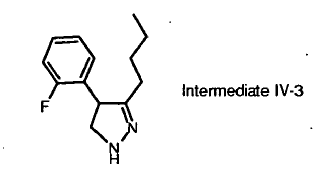

Intermediate IV-3 (3-(n-butyl)-4-(2-fluorophenyl-4,5-dihydropyrazole) was prepared analogously to 3-(n-pentyl)-4-phenyl-4,5-dihydropyrazole (Intermediate IV-1), from 2-(2-fluorophenyl)-hept-1-en-3-one and hydrazine hydrate. Some characteristic pyrazoline ring proton NMR signals: (400 MHz, CDCl₃) δ 3.37 (t, J - 9, 1H, H₅), 3.78 (t, J ∼ 10, 1H, H₅), 4.35 (t, J ∼ 10, 1H, H₄).

### Intermediate -VII-1

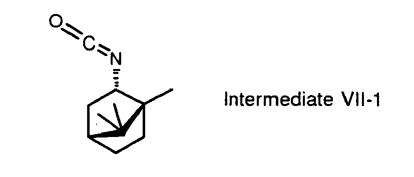

To a magnetically stirred solution of diphosgene (4.26 ml, 0.0353 mol) in dichloromethane (90 ml) was slowly added a solution of endo-1R, 2S, 4R-)-1,7,7-trimethylbicyclo[2.2.1]hept-2-ylamine (CAS 32511-34-5) and N,N-dimethylaniline (15.2 ml, 0.12 mol)) in dichloromethane (90 ml) at 0 °C. The resulting mixture was allowed to attain room temperature and stirred for 30 minutes. The mixture was concentrated and the residue taken up in dichloromethane, washed (3 x with 1N HCl and 1x brine), dried (MgSO₄) filtered and concentrated in vacuo to give endo-2-isocyanato-[(1R,2S,4R)1,7,7-trimethylbicyclo[2.2.1]heptane (10.43 g, 97 % yield. ¹H-NMR (400 MHz, CDCl₃) δ 0.85 (s, 3H), 0.86 (s, 3H), 0.89 (s, 3H), 1.11 (dd, J=132 and 4.2, 1H), 1.21-1.28 (m, 1H), 1.30-1.38 (m, 1H), 1.67 (t, J=4, 1H), 1.71-1.83 (m, 2H); 2.26-2.34 (m, 1H), 3.75 (ddd, J = 10.5, 4.1 and 2.3, 1H). Optical rotation ([α]_{D})= + 40.2 (c = 1.07, dichloromethane).

### Intermediate VII-2

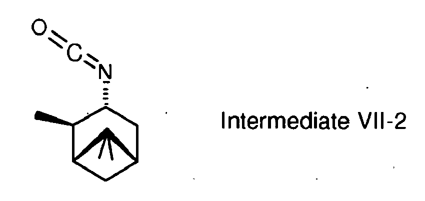

3-Isocyanato-[(1R,2R,3R,5S)-2,7,7-trimethylbicyclo[3.1.1]heptane (intermediate VII-2) was prepared from the reaction of (-)-3-amino-[(1R,2R,3R,5S)-2,7,7-trimethylbicyclo[3.1.1]heptane (CAS 69460-11-3) and triphosgene in the presence of DIPEA in dichloro methane at 0 °C. ¹H-NMR (400 MHz, CDCl₃) δ 0.95 (s, 3H), 1.00 (d, J=9, 1H), 1.13 (d, J= 7, 3H), 1.23 (s, 3H), 1.80-1.90 (m, 2H), 1.93-2.00 (m, 1H). 2.04-2.13 (m, 1H), 2.38-2.44 (m, 1H), 2.49-2.58 (m, 1H), 3.80-3.88 (m, 1H),.

### Intermediate VII-3

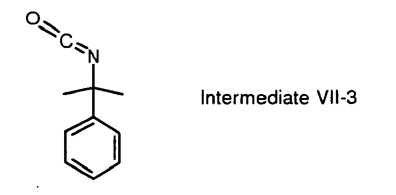

Intermediate VII-3 was prepared from diphosgene, cumylamine and N,N-dimethylaniline in dichloromethane analogously to the procedure described for intermediate VII-1. ¹H-NMR (400 MHz, CDCl₃) δ 1.71 (s, 6H), 7.22-7.29 (m, 1H), 7.32-7.38 (m, 2H), 7.42-7.46 (m, 2H).

### Intermediate VII-4

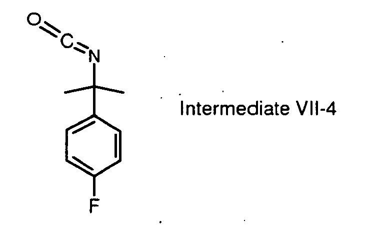

Intermediate VII-4 was prepared from diphosgene, 1-(4-fluorophenyl)-1-(methyl)ethylamine and N,N-dimethylaniline in dichloromethane analogously to the procedure described for intermediate VII-1. ¹H-NMR (400 MHz, CDCl₃) δ 1.70 (s, 6H), 6.99-7.05 (m, 2H), 7.37-7.43 (m, 2H).

### 3-(n-Butyl)-4-(2-fluorophenyl)-4,5-dihydro-(1H)-pyrazole-1-carbonyl chloride Intermediate VIII-1

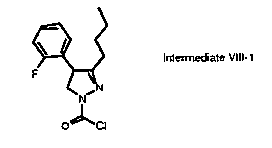

To a magnetically stirred solution of crude 3-(n-butyl)-4-(2-fluorophenyl)-4,5-dihydro - (1H)-pyrazole (Intermediate (IV-3) (2.0 gram, 8.93 mmol maximally) in dichloromethane (25 ml) was successively added DIPEA (1.50 g, 2.0 ml, 11.61 mmol) and triphosgene (0.79 g, 2.68 mmol, dissolved in 10 ml dichloromethane) at 0 °C and the resulting solution was allowed to attain room temperature and subsequently reacted at room temperature for 1 hour. Column chromatographic purification (eluant: dichloromethane) gave pure 3-(n-butyl)-4-(2-fluorophenyl)-4,5-dihydro-(1H)-pyrazole-1-carbonyl chloride (Intermediate VIII-1) (1.26 g, ∼50 % yield). ¹H-NMR (400 MHz, CDCl₃) δ 0.86 (t, J = 7, 3H), 1.22-1.36 (m, 2H), 1.42-1.60 (m, 2H), 2.08-2.18 (m, 1H), 2.27-2.40 (m, 1H), 3.96 (dd, J = 12 and 7, 1H), 4.34 (t, J = 12, 1H), 4.54-4.64 (m, 1H), 7.08-7.22 (m, 3H), 7.30-7.38 (m, 1H).

### 3-(n-pentyl)-4-phenyl-4,5-dihydro-(1H)-pyrazole-1-carbonyl chloride Intermediate VIII-2

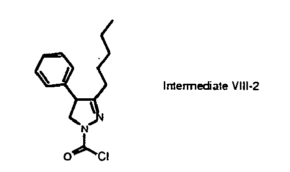

To a magnetically stirred solution of 3-(n-pentyl)-4-phenyl-4,5-dihydro-(1H)-pyrazole (116 ml of a 0.25 M solution in dichloromethane) was added DIPEA (116 ml of a 0.30 M solution in dichloromethane) and triphosgene (0.3 mol equivalent as a solution in dichloromethane) at 0 °C and the resulting solution was allowed to attain room temperature and subsequently reacted at room temperature for 1 hour to give a stock solution of crude 3-(n-pentyl)-4-phenyl-4,5-dihydro-(1H)-pyrazole-1-carbonyl chloride (Intermediate VIII-2). This stock solution was used in parallel reactions with various amines, to prepare compounds 103- 123.

### EXAMPLE 4: SYNTHESES OF SPECIFIC COMPOUNDS

### Compound 1

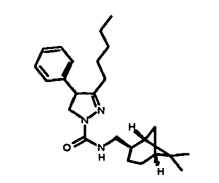

### N-[(1R,2S,5R)-rel-6,6-dimethylbicyclo[3.1.1]heptan-2-methyl]-3-(n-pentyl)-4-phenyl-4,5-dihydro-(1H)-pyrazole-1-carboxamide

**Part A:** To a magnetically stirred solution of 2-phenyl-oct-1-en-3-one (Intermediate III-1) (5 gram, 24.7 mmol) in ethanol (30 ml) was added hydrazine.hydrate (2.46 ml, 50.7 mmol) and the resulting solution was heated at reflux temperature for 4 hours. The resulting solution was allowed to attain room temperature, concentrated and taken up in a mixture of MTBE and water. The organic layer was collected, dried over Na₂SO₄, filtered and concentrated to give crude 3-(n-pentyl)-4-phenyl-4,5-dihydropyrazole (Intermediate IV-1) (4.8 gram) as an impure oil which was used immediately in the subsequent step. (intermediate IV-1) some characteristic pyrazoline ring proton NMR signals: (400 MHz, CDCl₃) δ 3.36 (t, J ∼ 10.1H), 3.81 (t, J∼10,1H),4.00(t,J∼10,1H).
**Part B:** To a magnetically stirred solution of (-)-cis-myrtanylamine (2.4 ml, 14.2 mmol) (CAS 38235-68-6)) in dichloromethane (40 ml) was added triethylamine (2 ml, 14.2 mmol). The resulting solution was slowly added to a solution of triphosgene (1.4 gram, 4.7 mmol) in dichloromethane (60 ml) and the resulting mixture was stirred at room temperature for 16 hours. The mixture was then poured in water and extracted with dichloromethane, dried over Na₂SO₄, filtered and concentrated to give cis-myrtanylisocyanate (2.12 gram) as an oil.
**Part C.** 3-(n-Pentyl)-4-phenyl-4,5-dihydropyrazole (2.2 gram, 10.3 mmol) was dissolved in benzene (25 ml) and treated with cis-myrtanylisocyanate (2.12 g, 11.8 mmol) and 5 drops of triethylamine and the resulting solution was stirred at room temperature for 16 hours. The solution was concentrated, followed by flash chromatographic purification (heptane/ethylacetate = 6:1 (v/v)) to give N-[(1R,2S,5R)-rel-6,6-dimethylbicyclo[3.1.1]heptan-2-methyl]-3-(n-pentyl)-4-phenyl-4,5-dihydro - (1H)-pyrazole-1-carboxamide] as an oil. ¹H-NMR (400 MHz, CDCl₃) δ 0.85-0.95 (m, 4H), 1.06 (s, 3H), 1.19-1.31 (m, 7H), 1.38-1.60 (m, 3H), 1.82-2.41 (m, 9H), 3.22-3.40 (m, 2H), 3.83-3.90 (m, 1H), 4.12 (dd, J = 12 and 7, 1H), 4.18-4.26 (m, 1H), 5.92-5.96 (m, 1H), 7.15 (br d, J ∼ 8, 2H), 7.25 7.37 (m, 3H). LC/MS (Method A). Retention time: 7.07 minutes: Found molecular mass (API-ES; positive scan) = 396.

Analogously the compounds 2-84 were prepared:

### Compound 2

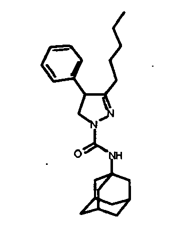

### N-(1-Adamantyl)-3-(n-pentyl)-4-phenyl-4,5-dihydro-(1H)-pyrazole-1-carboxamide

LC/MS (Method A). Retention time: 8.04 minutes: Found molecular mass (API-ES; positive scan) = 394. R_{f} (dichloromethane/methanol = 99/1 (v/v)) = 0.3.

### Compound 3

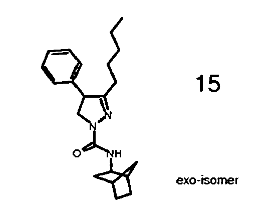

### N-(Exo-bicyclo[2.2.1]hept-2-yl)-3-(n-pentyl)-4-phenyl-4,5-dihydro-(1H)-pyrazole-1-carboxamide

LC/MS (Method A). Retention time: 9.26 minutes: Found molecular mass (API-ES; positive scan) =354. R_{f} (dichloromethane/methanol = 99/1 (v/v)) = 0.2.

### Compound 4

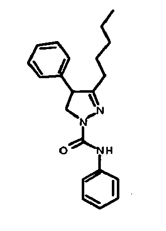

### N-Phenyl-3-(n-pentyl)-4-phenyl-4,5-dihydro-(1H)-pyrazole-1-carboxamide

LC/MS (Method A). Retention time: 4.35 minutes: Found molecular mass (API-ES; positive scan) = 336. R_{f}(dichloromethane/methanol = 99/1 (v/v)) = 0.4.

### Compound 5

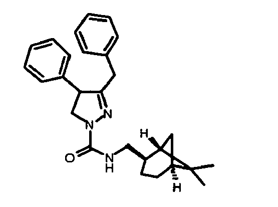

### N-[(1R,2S,5R)-rel-6,6-dimethylbicyclo[3.1.1]heptan-2-methyl]-3-(benzyl)-4-phenyl-4,5-dihydro-(1H)-pyrazole-1-carboxamide (from the isocyanate derived from (-)-cis-myrtanylamine (CAS 38235-68-6))

LC/MS (Method A). Retention time: 4.96 minutes: Found molecular mass (API-ES; positive scan) =416. R_{f} (dichloromethane/methanol = 99/1 (v/v)) = 0.25.

### Compound 6

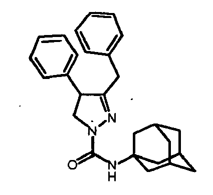

### N-(1-Adamantyl)-3-(benzyl)-4-phenyl-4,5-dihydro-(1H)-pyrazole-1-carboxamide

¹H-NMR (400 MHz, CDCl₃) δ 1.65-1.75 (m, 6H), 2.06-2.13 (m, 9H), 3.20 (d, J ∼ 14, 1H), 3.65 (d, J - 14, 1H), 3.84 (dd, J - 11 and 6,1H), 3.95-4.00 (m, 1H), 4.14 (t, J ∼ 11, 1H), 5.85 (br s, 1H), 7.05-7.11 (m, 4H), 722-7.36 (m, 6 H).

LC/MS (Method A). Retention time: 5.34 minute s: Found molecular mass (API-ES; positive scan) =414. Melting point: 61 °C

### Compound 7

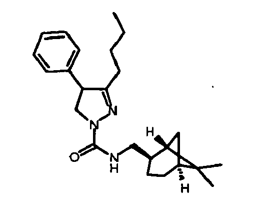

### N-[(1R,2S,5R)-rel-6,6-dimethylbicyclo[3.1.1]heptan-2-methyl]-3-(n-butyl)-4-phenyl-4,5-dihydro-(1H)-pyrazole-1-carboxamide (from the isocyanate derived from (-)-cis-myrtanylamine (CAS 38235-68-6))

LC/MS (Method B). Retention time: 5.03 minutes: Found molecular mass (API-ES; positive scan) = 382. R_{f} (dichloromethane/methanol = 99/1 (v/v)) = 0.2.

¹H-NMR (400 MHz, CDCl₃) δ 0.80-0.90 (m, 3H), 0.92 (d, J - 10, 1H), 1.06 (s, 3H), 1.21 (s, 3H), 1.22-1.60 (m, 5H); 1.82-2.41 (m, 8H), 3.23-3.40 (m, 2H), 3.87 (ddd, J - 11, 7 and 2, 1H), 4.12 (br dd, J - 11 and 7, 1H); 4.18-4.26 (m, 1H), 5.95 (br t, J - 7, 1H), 7.15 (br d, J - 8, 2H), 7.25-7.37 (m, 3H).

### Compound 8

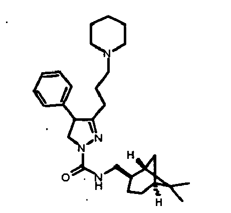

### N-[(1R,2S,5R)-rel-6,6-dimethylbicyclo[3.1.1] heptan-2-methyl]-3-[3-(1-piperidinyl)propyl]-4-phenyl-4,5-dihydro-(1H)-pyrazole-1-carboxamide (from the isocyanate derived from (-)-cis-myrtanylamine (CAS 38235-68-6))

¹H-NMR (400 MHz, CDCl₃) δ 0.92 (d, J = 10,1H), 1.07 (s, 3H), 1.21 (s, 3H), 1.38-2.43 (m, 24H), 3.21 -3.38 (m, 2H), 3.84-3.90 (m, 1H), 4.13 (dd, J = 11 and 6, 1H), 4.19-4.26 (m, 1H), 5.97 (br t, J ∼ 7, 1H), 7.15 (br d, J ∼ 8, 2H), 7.25-7.40 (m, 3H).

### Compound 9

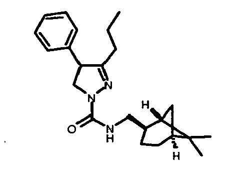

### N-[(1R,2S,5R)-rel-6,6-dimethylbicyclo[3.1.1]heptan-2-methyl]-3-(n-propyl)-4-phenyl-4,5-dihydro-(1H)-pyrazole-1-carboxamide (from the isocyanate derived from (-)-cis-myrtanylamine (CAS 38235-68-6))

¹H-NMR (400 MHz, CDCl₃) δ 0.85-0.95 (m, 4H), 1.16 (s, 3H), 1.21 (s, 3H), 1.41-1.61 (m, 2H), 1.83-2.17 (m, 8H), 2.25-2.41 (m, 2H), 3.22-3.39 (m, 2H), 3.83-3.90 (m, 1H), 4.12 (dd, J = 12 and 7, 1H), 4.18 -4.26 (m, 1H), 5.93-5.99 (m, 1H), 7.15 (br d, J - 8, 2H), 7.26-7.36 (m, 3H).

### Compound 10

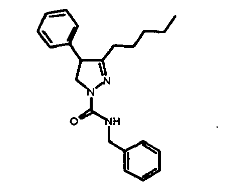

### N-(Benzyl)-3-(n-pentyl)-4-phenyl-4,5-dihydro-(1H)-pyrazole-1-carboxamide

LC/MS (Method B). Retention time: 5.76 minutes: Found molecular mass (API-ES; positive scan) = 350. Mobile phase gradient: 0- 5 minutes: Solution A/Solution B = 30/70 (v/v)). > 5 minutes : Solution B. R_{f} (dichloromethane/ methanol = 99/1 (v/v)) = 0.2.

### Compound 11

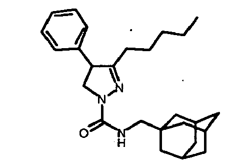

### N-(1-Adamantyl)methyl-3-(n-pentyl)-4-phenyl-4,5-dihydro-(1H)-pyrazole-1-carboxamide

LC/MS (Method B). Retention time: 6.28 minutes: Found molecular mass (API-ES; positive scan) = 408. Mobile phase gradient: 0 - 3 minutes: Solution A/Solution B = 20/80 (v/v)). > 3 minutes : Solution B. R_{f} (dichloromethane/methanol = 99/1 (v/v)) = 0.2.

### Compound 12

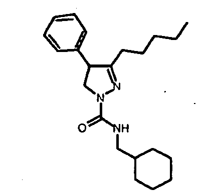

### N-(Cyclohexylmethyl)-3-(n-pentyl)-4-phenyl-4,5-dihydro-(1H)-pyrazole-1-carboxamide

LC/MS (Method B). Retention time: 7.01 minutes: Found molecular mass (API-ES; positive scan) = 356. Mobile phase gradient: 0 - 5 minutes: Solution A/Solution B = 30/70 (v/v)). > 5 minutes: Solution B. R_{f} (dichloromethane/methanol = 99/1 (v/v)) = 0.2.

### Compound 13

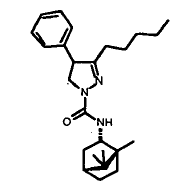

### N-[Endo-(1R,2S,4R)-1,7,7-trimethylbicyclo[2.2.1]hept-2-yl]-3-(n-pentyl)-4-phenyl-4,5-dihydro-(1H)-pyrazole-1-carboxamide (from the isocyanate (intermediate VII-1) derived from 1 R-(+)-bornylamine (CAS 32511-34-5)).

¹H-NMR (400 MHz, CDCl₃) δ 0.80-0.94 (m, 10H), 0.97 (s, 3H), 120-1.69 (m, 10H), 1.74-1.83 (m, 1H), 2.00-222 (m, 2H), 2.33-2.45 (m, 1H), 3.83-3.89 (m, 1H), 409-4.27 (m, 3H), 6.02 (br d, J ∼ 10, 1H), 7.16 (br d, J - 8, 2H), 7.27-7.37 (m, 3H).

LC/MS (Method B). Retention time: 7.43 minutes: Found molecular mass (API-ES; positive scan) = 396. Mobile phase gradient: 0 - 3 minutes: Solution A/Solution B = 20/80 (v/v)). > 3 minutes : Solution B. R_{f} (dich loromethane/methanol = 99/1 (v/v)) = 0.3.

### Compound 14

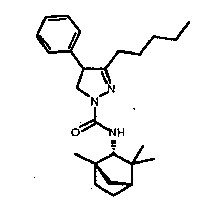

### N-[endo-(1S)-1,3,3-trimethylbicyclo[2.2.1]hept-2-yl]-3-(n-pentyl)-4-phenyl-4,5-dihydro-(1H)-pyrazole-1-carboxamide (from the isocyanate derived from endo-(1S)-1,3,3-trimethylbicyclo[2.2.1]heptan-2-amine (CAS 301822 -76-4).

LC/MS (Method B). Retention time: 5.83 minutes: Found molecular mass (API-ES; positive scan) = 396. Mobile phase gradient: 0 - 5 minutes: Solution A/Solution B = 15/85 (v/v)). > 5 minutes: Solution B.

R_{f} (dichloromethane/methanol = 99/1 (v/v)) = 0.3.

### Compound 15

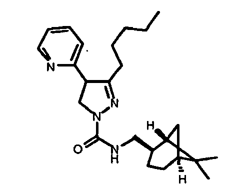

### N-[(1R,2S,5R)-rel-6,6-dimethylbicyclo[3.1.1]heptan-2-methyl]-3-(n-propyl)-4-(2-pyridyl)-4,5-dihydro-(1H)pyrazole-1-carboxamide (from the isocyanate derived from (-)-cis-myrtanylamine (CAS 38235-68-6))

¹H-NMR (400 MHz, CDCl₃) δ 0.85-0.94 (m, 4H), 1.15 (s, 3H), 120-2.40 (m, 18H), 3.20-3.39 (m, 2H), 3.99-4.07 (m, 1H), 4.22-4.30 (m, 1H), 4.37 (dd, J = 12 and 7, 1H), 5.93-5.99 (m, 1H), 7.14-7.23 (m, 2H), 7.65-7.71 (m, 1H), 8.57-8.60 (m, 2H).

### Compound 16

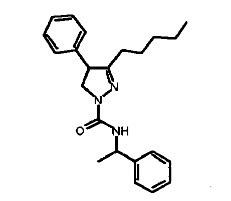

### N-(1-Phenyl-ethyl)-3-(n-pentyl)-4-phenyl-4,5-dihydro-(1H)-pyrazole-1-carboxamide

LC/MS (Method B). Retention time: 4.50 minutes: Found molecular mass (API-ES; positive scan) = 364.

R_{f} (dichloromethane/methanol = 99/1 (v/v)) = 0.25.

### Compound 17

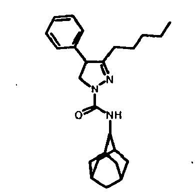

### N-(2-Adamantyl)-3-(n-pentyl)-4-phenyl)-4,5-dihydro-(1H)-pyrazole-1-carboxamide

LC/MS (Method B). Retention time: 5.55 minutes: Found molecular mass (API-ES; positive scan) =394. Melting point: 71 °C.

R_{f} (dichloromethane/methanol = 99/1 (v/v)) = 0.2.

### Compound 18

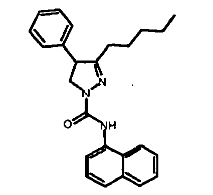

### N-(1-Naphtyl)-3-(n-pentyl)-4-phenyl-4,5-dihydro-(1H)-pyrazole-1-carboxamide

¹H-NMR (400 MHz, CDCl₃) δ 0.90 (t, J = 7, 3H), 125-1.37 (m, 4H), 1.55-1.65 (m, 2H), 2.12-2.32 (m, 2H), 4.02 (dd, J = 10 and 6, 1H), 4.26 (dd, J = 12 and 6, 1H), 4.39 (t, J ∼ 12, 1H), 7.20-7.57 (m, 8H), 7.62 (d, J = 8, 1H), 7.62 (d, J = 8, 1H), 7.87 (d, J = 8, 1H), 7.96 (d, J = 8, 1H), 8.14 (d, J = 8, 1H), 8.60 (br s, 1H).

R_{f} (dichloromethane/methanol = 99/1 (v/v)) = 0.3.

### Compound 19

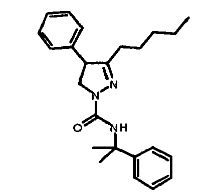

### N-(1-Methyl-1-phenyl-ethyl)-3-(n-pentyl)-4-phenyl-4,5-dihydro-(1H)-pyrazole-1-carboxamide

¹H-NMR (300 MHz, CDCl₃) δ 0.86 (t, J = 7, 3H), 120-1.60 (m, 6H), 1.75 (s, 3H), 1.78 (s, 3H), 2.00-2.20 (m, 2H), 3.79-3.85 (m, 1H), 4.05-4.22 (m, 2H), 6.37 (br s, 1 H), 7.13-7.37 (m, 8H), 7.46-7.51 (m, 2H). R_{f} (dichloromethane/ methanol = 99/1 (v/v)) = 0.2.

### Compound 20

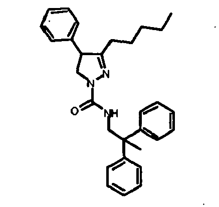

### N-(2,2-Diphenylpropyl)-3-(n-pentyl)-4-phenyl-4,5-dihydro-(1H)-pyrazole-1-carboxamide

LC/MS (Method B). Retention time: 4.99 minutes: Found molecular mass (API-ES; positive scan) = 454. R_{f} (dichloromethane/methanol = 99/1 (v/v)) = 0.3.

### Compound 21

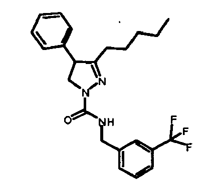

### N-((3-Trifluoromethyl)benzyl)-3-(n-pentyl)-4-phenyl-4,5-dihydro-(1H)-pyrazole-1-carboxamide

LC/MS (Method B). Retention time: 4.43 minutes: Found molecular mass (API-ES; positive scan) =418.

R_{f} (dichloromethane/methanol = 99/1 (v/v)) = 0.2.

### Compound 22

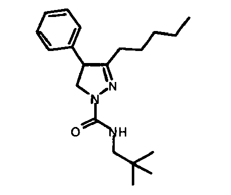

### N-(2,2-Dimethylpropyl)-3-(n-pentyl)-4-phenyl-4,5-dihydro-(1H)-pyrazole-1-carboxamide

LC/MS (Method B). Retention time: 4.36 minutes: Found molecular mass (API-ES; positive scan) = 330.

R_{f} (dichloromethane/methanol = 99/1 (v/v)) = 0.3.

### Compound 23

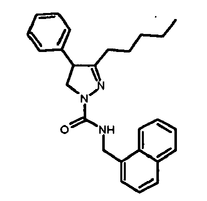

### N-(Naphthalen-1-yl-methyl)-3-(n-pentyl)-4-phenyl-4,5-dihydro-(1H)-pyrazole-1 carboxamide

LC/MS (Method B). Retention time: 6.41 minutes: Found molecular mass (API-ES; positive scan) = 400.

R_{f} (dichloromethane/methanol = 99/1 (v/v)) = 0.3.

### Compound 24

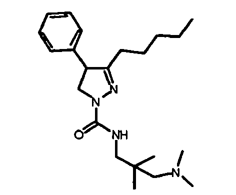

### N-[(3-Dimethylamino)-2,2-dimethylpropyl]-3-(n-pentyl)-4-phenyl-4,5-dihydro-(1H)-pyrazole-1-carboxamide

¹H-NMR (400 MHz, CDCl₃) δ 0.80-0.90 (m, 3H), 0.96 (br s, 6H), 1 20-1.28 (m, 4H), 1.46-1.57 (m, 2H), 2.00-2.16 (m, 2H), 2.24 (s, 2H), 2.32 (s, 6H), 3.15-3.27 (m, 2H), 3.87 (dd, J - 11 and 7, 1H), 4.10 (dd, J ∼ 11 and 7, 1H), 4.23 (br t, J ∼ 11, 1H), 7.14-7.18 (m, 2H), 7.26-7.38 (m, 4H).

### Compound 25

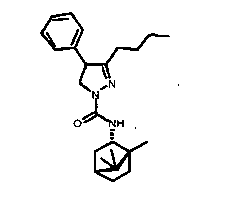

### N-[Endo-(1R,2S,4R)-1,7,7-trimethylbicyclo[2.2.1]hept-2-yl]-3-(n-butyl)-4-phenyl-4,5-dihydro-(1H)-pyrazole-1-carboxamide (from the isocyanate derived from 1R-(+)-bornylamine (CAS 32511-34-5)).

¹H-NMR (400 MHz, CDCl₃) δ 0.80-0.94.(m, 10H), 0.97 (s, 3H), 120-1.70 (m, 8H), 1.72-1.84 (m, 1H), 2.01-2.10 (m, 1H), 2.14-2.24 (m, 1H), 2.34-2.44 (m, 1H), 3.82-3.89 (m, 1H), 4.094.27 (m, 3H), 6.01 (br d, J ∼ 9,1H), 7.16 (br d, J ∼ 8, 2H), 7.26-7.37 (m, 3H).

### Compound 26

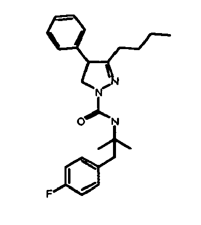

### N-(2-(4-fluorophenyl)-1,1-dimethyl-ethyl)-3-(n-butyl)-4-phenyl-4,5-dihydro-(1H)-pyrazole-1-carboxamide

¹H-NMR (400 MHz, CDCl₃) δ 0.84 (t,J = 7, 3H), 120-1.52 (m, 10H), 1.97-2.17 (m, 2H), 3.02 (d, J = 13,1H),3.09 (d, J =13, 1H), 3.88 (dd,J = 10 and 6,1H), 4.08-4.15 (m, 1H), 4.18-4.24 (m, 1H), 5.76 (br s, 1H), 6.93-7.01 (m, 2H), 7.12-7.18 (m, 4H), 7.26-7.38 (m, 3 H).

### Compound 27

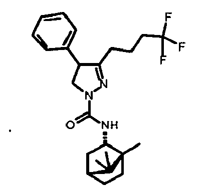

### N-[Endo-(1R,25,4R)-1,7,7-trimethylbicyclo[2.2.1]hept-2-yl]-3-(4,4,4-trifluoro-n-butyl)-4-phenyl-4,5-dihydro-(1H)-pyrazole-1-carboxamide (from the isocyanate derived from 1R-(+)-bornylamine (CAS 32511-34-5)).

¹H-NMR (400 MHz, CDCl₃) δ 0.84-0.94 (m, 7H), 0.97 (s, 3H), 120-1.29 (m, 1H), 1.36-1.47 (m, 1H), 1.53-1.63 (m, 1H), 1.67 (br t, J∼4, 1H), 1.71-1.89 (m, 3H), 2.00-2.23 (m, 4H), 2.35-2.51 (m, 1H), 3.86-3.93 (m, 1H), 4.08-4.30 (m, 3H), 6.00 (br d, J ∼ 9, 1H), 7.15 (br d, J - 8, 2H), 726-7.39 (m, 3H).

### Compound 28

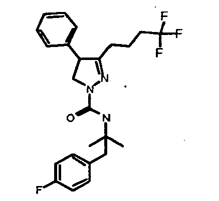

### N-(2-(4-fluorophenyl)-1,1-dimethyl-ethyl)-3-(4,4,4-trifluoro-n-butyl)-4-phenyl-4,5-dihydro-(1H)-pyrazole-1-carboxamide

¹H-NMR (400 MHz, CDCl₃) δ 1.38 (s, 3H), 1.39 (s, 3H), 1.67-1.84 (m, 2H), 1.92-2.16 (m.4H), 3.02 (d.J=13.1H),3.08 (d.J=13.1H),3.91 (dd, J = 11 and 7,1H), 406-4.13 (m, 1H), 424 (t, J = 11,1H), 5.72 (br s, 1H), 6.94-7.00 (m, 2H), 7.12-7.18 (m, 4H), 7.28-7.40 (m, 3H).

### Compound 29

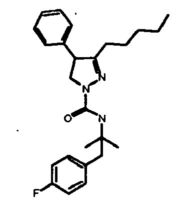

### N-(2-(4-Fluorophenyl)-1,1-dimethyl-ethyl)-3-(n-pentyl)-4-phenyl-4,5-dihydro-(1H)-pyrazole-1-carboxamide

¹H-NMR (400 MHz, CDCl₃) δ 0.84 (t, J = 7, 3H), 1.14-1.30 (m, 4H), 1.32-1.54 (m, 8H), 1.96-2.14 (m, 2H), 3.03 (d, J = 13, 1H), 3.09 (d, J = 13, 1H), 3.88 (dd, J = 11 and 6, 1H), 4.08-4.14 (m, 1H), 421 (t, J = 11, 1H), 5.76 (br s, 1H), 6.93-7.00 (m, 2H), 7.13-7.18 (m, 4H), 7.28-738 (m, 3H).

### Compound 30

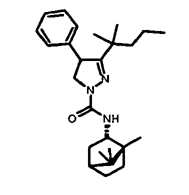

### N-[Endo-(1R,2S,4R)-1,7,7-Trimethylbicyclo[2.2.1]hept-2-yl]-3-(1,1-dimethyl-n-butyl)-4-phenyl-4,5-dihydro-(1H)-pyrazole-1-carboxamide (from the isocyanate derived from 1 R-(+)-bornylamine (CAS 32511-34-5)).

¹H-NMR (400 MHz, CDCl₃) δ 0.73-0.93 (m, 13H), 0.97 (s, 3H), 1.05 (s, 3H), 1.10-1.70 (m, 8H), 1.73-1.85 (m, 1H), 2.36-2.45 (m, 1H), 3.88-3.95 (m, 1H), 4.02-4.21 (m, 3H), 6.12 (br d, J - 9,1H), 713-7.19 (m, 2H), 721-7.32 (m, 3H).

### Compound 31

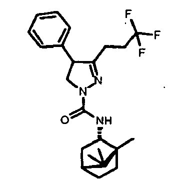

### N-[Endo-(1R,2S,4R)-1,7,7-Trimethylbicyclo[2.2.1]hept-2-yl]-3-(3,3,3-trifluoropropyl)-4-phenyl-4,5-dihydro-(1H)-pyrazole-1-carboxamide (from the isocyanate derived from 1 R-(+)-bornylamine (CAS 32511 -34-5)).

¹H-NMR (400 MHz, CDCl₃) δ 0.81-0.94 (m, 7H), 0.97 (s, 3H), 1.21-1.30 (m, 1H), 1.36-1.48 (m, 1H),1.52-1 .70 (m, 2H),1.74-1.85 (m, 1H), 2.30-2.48 (m, 5H), 3.88-3.95 (m, 1H), 4.10-4.33 (m, 3H), 5.96 (br d, J - 9, 1H), 7.17 (br d, J = 8, 2H), 7.28-7.40 (m, 3H).

### Compound 32

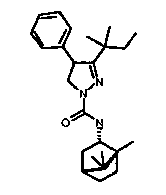

### N-[Endo-(1R,2S,4R)-1,7,7-Trimethylbicyclo[2.2.1]hept-2-yl]-3-(1,1-dimethylpropyl)-4-phenyl-4,5-dihydro(1H)-pyrazole-1-carboxamide (from the isocyanate derived from 1 R-(+)-bornylamine (CAS 32511-34-5)).

¹H-NMR (400 MHz, CDCl₃) δ 0.77 (t, J = 7, 3H), 0.81 -0.95 (m, 10H), 0.97 (s, 3H), 1.04 (s, 3H), 1.10-1.70 (m, 6H), 1.74-1.85 (m, 1H), 2.34-2.46 (m, 1H), 3.88-3.94 (m, 1H), 4.02-4.20 (m, 3H), 6.13 (br d, J - 9,1H), 7.13-7.18 (m, 2H), 7.21-7.33 (m, 3H).

### Compound 33

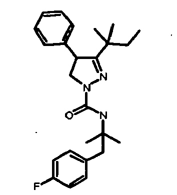

### N-(2-(4-Fluorophenyl)-1,1-dimethyl-ethyl)-3-(1,1-dimethylpropyl)-4-phenyl-45-dihydro-(1H)-pyrazole-1-carboxamide

¹H-NMR (400 MHz, CDCl₃) δ 0.72 (t, J = 7, 3H), 0.83 (s, 3H), 0.99 (s, 3H), 1.22-1.31 (m, 2H), 1.40 (s, 6H), 2.97-3.09 (m, 2H), 3.88-3.94 (m, 1H), 4.01 -4.14 (m, 2H), 5.84 (br s, 1H), 6.93-7.01 (m, 2H), 7.11-7.19 (m, 4H), 7.22-7.33 (m, 3H).

### Compound 34

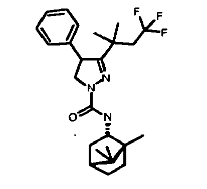

### N-[Endo-(1R,2S,4R)-1,7,7-trimethylbicyclo[2.2.1]hept-2-yl]-3-(1,1-dimethyl-3,3,3-trifluoropropyl)-4-phenyl-4,5-dihydro-(1H)-pyrazole-1-carboxamide (from the isocyanate derived from 1 R-(+)-bornylamine (CAS 32511-34-5)).

¹H-NMR (400 MHz, CDCl₃) δ 0.81-0.95 (m, 7H), 0.97 (s, 3H), 1.09-1.60 (m, 9H, including 2 Me singlets at 1.12 and 1.13 ppm), 1.66-1.71 (m, 1H), 1.75-1.85 (m, 1H), 2.24-2.47 (m, 3H), 3.91-3 98 (m, 1H), 4.08-4.24 (m, 3H), 6.01-6.08 (m,1H), 7.13-7.19 (m,2H), 724-7.36 (m, 3H).

### Compound 35

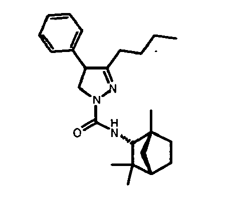

### N-[endo-(1R)-1,3,3-trimethylbicyclo[2.2.1]hept-2-yl]-3-(n-butyl)-4-phenyl-4,5-dihydro-(1H)-pyrazole-1-carboxamide (from the isocyanate derived from endo-(1R)-1,3,3-trimethylbicyclo[2.2.1]heptan-2-amine.

¹H-NMR (400 MHz, CDCl₃) δ 0.78-0.89 (m, 6H), 1.05-1.78 (m, 17H), 2.01-2.22 (m, 2H), 3.56 (dd, J = 10 and 2, 1H), 3.83-3.91 (m, 1H). 4.09-4.27 (m, 2H), 6.07 (br d, J ∼ 10, 1H), 7.14 7.18 (m, 2H), 7.26-7.37 (m, 3H).

### Compound 36

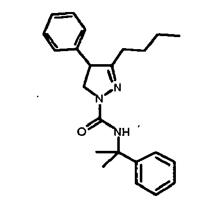

### N-(1-Methyl-1-phenyl-ethyl)-3-(n-butyl)-4-phenyl-4,5-dihydro-(1H)-pyrazole-1-carboxamide

¹H-NMR (400 MHz, CDCl₃) δ 0.86 (t, J = 7, 3H), 1.21-1.33 (m, 2H), 1.38-1.54 (m, 2H), 1.75 (s, 3H), 1.77 (s, 3H), 2.04-2.22 (m, 2H), 3.82 (dd, J = 9.7 and 5.6, 1H), 4.07-4.20 (m, 2H), 6.38 (br s, 1H), 7.13-7.36 (m, 8H), 7.48 (br d J ∼ 8, 2H).

### Compound 37

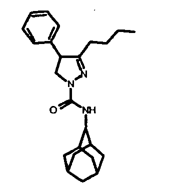

### N-(2-Adamantyl)-3-(n-butyl)-4-phenyl-4,5-dihydro-(1H)-pyrazole-1-carboxamide

¹H-NMR (400 MHz, CDCl₃) δ 0.86 (t, J = 7, 3H), 123-1.34 (m, 2H), 1.41-1.53 (m, 2H), 1.62-2.10 (m, 15H), 2.13 2.22 (m, 1H), 3.86 (dd, J = 10.5 and 6.5, 1H), 3.98-4.03 (m 1H), 4.13-4.26 (m, 2H), 6.38 (br d, J - 8, 1H), 7.16 (br d, J∼8, 2H), 7.24-7.36 (m, 3H).

### Compound 38

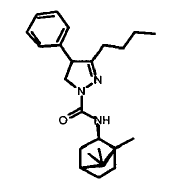

### N-[Exo-(1R,2R,4R)-1,7,7-trimethylbicyclo[2.2.1]hept-2-yl]-3-(n-butyl)-4-phenyl-4,5-dihydro-(1H)pyrazole-1-carboxamide (from the isocyanate derived from exo-1 R-bornylamine.

¹H-NMR (400 MHz, CDCl₃) δ 0.79-0.92 (m, 10H), 0.98 (s, 3H), 1.12-1.77 (m, 9H), 1.86-1.93 (m, 1H),1.99-2.19 (m, 2H), 3.80-3.90 (m, 2H), 4.07-4.25 (m, 2H), 6.06 (br d, J ∼ 9, 1H), 7.15 (br d, J∼ 8, 2H), 726-7.37 (m, 3H).

### Compound 39

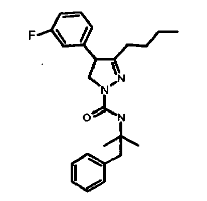

### N-(2-phenyl-1,1-dimethyl-ethyl)-3-(n-butyl)-4-(3-fluorophenyl)-4,5-dihydro-(1H)-pyrazole-1-carboxamide

¹H-NMR (400 MHz, CDCl₃) δ 0.85 (t, J = 7, 3H), 120-1.32 (m, 2H), 1.37-1.51 (m, 8H), 1.98-2.18.(m, 2H), 3.03 (d, J = 18, 1H), 3.11 (d, J = 18, 1H), 3.88 (dd, J = 11 and 7,1H), 4.07-4.24 (m, 2H), 5.82 (br s, 1H), 6.84-7.04 (m, 3H), 7.17-7.36 (m, 6H).

### Compound 40

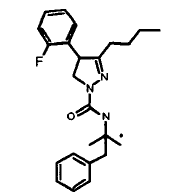

### N-(2-phenyl-1,1-dimethyl-ethyl)-3-(n-butyl)-4-(2-fluorophenyl)-4,5-dihydro-(1H)-pyrazole-1-carboxamide

¹H-NMR (400 MHz, CDCl₃ δ 0.85 (t, J = 7, 3H), 120-1.32 (m, 2H), 1.34-1.53 (m, 8H), 2.00-2.19 (m, 2H), 3.03 (d, J = 18, 1H), 3.11 (d, J = 18, 1H), 3.89 (dd, J = 11 and 7, 1H), 4.20 (t, J = 11, H), 4.47 (dd, J = 11 and 7, 1H), 5.81 (br s, 1H), 7.05-7.31 (m, 9H).

### Compound 41

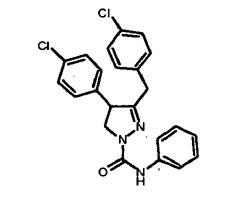

### N-Phenyl-3-(4-chlorobenzyl)-4-(4-chlorophenyl)4,5-dihydro-(1H)-pyrazole-1-carboxamide

Melting point: 156 °C.

### Compound 42

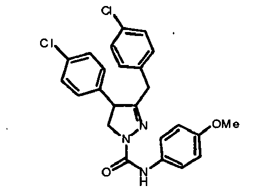

### N-(4-Methoxyphenyl)-3-(4-chlorobenzyl)-(4-chlorophenyl)-4,5-dihydro-(1H)-pyrazole-1-carboxamide

Melting point: 116-119 °C.

### Compound 43

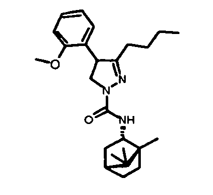

### N-[Endo-(1R,2S,4R)-1,7,7-trimethylbicyclo[2.2.1]hept-2-yl]-3-(n-butyl)-4-(2-methoxyphenyl)-4,5-dihydro-(1H)-pyrazole-1-carboxamide (from the isocyanate derived from 1R-(+)-bornylamine (CAS 32511-34-5)).

¹H-NMR (400 MHz, CDCl₃) δ 0.84-0.95 (m, 10H) 0.97 (s, 3H), 120-1.68 (m, 8H), 1.73-1.83 (m, 1H), 2.01-2.11 (m, 1H), 2.16-2.26 (m, 1H), 2.34-2.44 (m, 1H), 3.78-3.85 (m, 4 H), 4.08-4.23 (m, 2H), 4.50-4.58 (m, 1H), 5.98-6.03 (m, 1H), 6.86-6.96 (m, 2H), 7.06 (dd, J = 8 and 2,1H), 7.22-7.28 (m, 1H).

### Compound 44

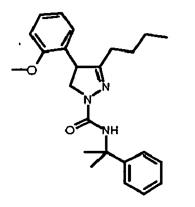

### N-(1-Methyl-1-phenyl-ethyl)-3-(n-butyl)-4-(2-methoxyphenyl)-4,5-dihydro-(1H)-pyrazole-1-carboxamide

¹H-NMR (400 MHz, CDCl₃) δ 0.85 (t, J = 7,3H), 1.24-1.56(m,4H), 1.75 (s, 3H),1.76 (s, 3H), 2.01-2.11 (m, 1H), 2.16-2.25 (m, 1H), 3.75-3.82 (m, 4H), 4.07 (t, J = 11, 1H), 4.53 (dd, J = 11 and 7, 1H), 6.36 (br s, 1H), 6.87 (d, J = 8, 1H), 6.90-6.95 (m, 1H), 7.06 (dd, J = 8 and 2, 1H), 7.19-7.28 (m, 2H), 7.33 (t, J = 8, 2H), 7.48 (br d, J = 8, 2H).

### Compound 45

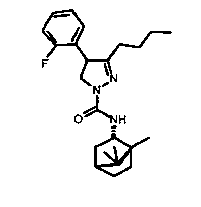

### N-[Endo-(1R,2S,4R)-1,7,7-trimethylbicyclo[2.2.1]hept-2-yl]-3-(n-butyl)-4-(2-fluorophenyl)-4,5-dihydro-(1H)-pyrazole-1-carboxamide (from the isocyanate derived from 1R-(+)-bornylamine (CAS 32511-34-5)).

¹H-NMR (400 MHz, CDCl₃) δ 0.85-0.95 (m, 10H), 0.97 (s, 3H), 1.21-1.83 (m, 9H), 2.05-2.14 (m, 1H), 2.19-228 (m, 1H), 2.35-2.45 (m, 1H), 3.82-3.90 (m, 1H), 4.13-4.24 (m, 2 H), 4.49 (dd, J = 11 and 7, 1H), 6.01 (br d, J ∼9, 1H), 7.03-7.18 (m, 3H), 7.23-7.30 (m, 1H).

### Compound 46

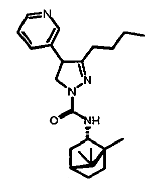

### N-[Endo-(1R,2S,4R)-1,7,7-trimethylbicyclo[2.2.1]hept-2-yl]-3-(n-butyl)-4-(pyrid-3-yl)-4,5-dihydro-(1H)-pyrazole-1-carboxamide (from the isocyanate derived from 1 R-(+)-bornylamine (CAS 32511 -34-5)).

¹H-NMR (400 MHz, CDCl₃) δ 0.85-0.94 (m, 10 H), 0.97 (s, 3H), 121-1.70 (m, 8H), 1.74-1.84 (m, 1H), 2.02-2.12 (m, 1H), 2.16-2.27 (m, 1H), 2.33-2.46 (m, 1H), 3.82-3.89 (m, 1H), 4.12-4.28 (m, 3H), 6.02 (br d, J - 9, 1H), 7.28-7.33 (m, 1H), 7.47-7.52 (m, 1H), 8.47 (br d, J - 2, 1H), 8.56 (dd, J = 5 and 2, 1H).

### Compound 47

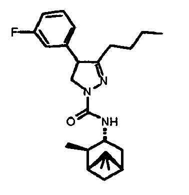

### N-[(1R,2R,3R,5S)-2,7,7-trimethylbicyclo[3.1.1]hept-3-yl]-3-(n-butyl)-4-(3-fluorophenyl)-4,5-dihydro-(1H)-pyrazole-1-carboxamide (from the isocyanate derived from (1R,2R,3R,5S)-(-)-isopinocampheylamine (CAS 69460-11-3)).

¹H-NMR (400 MHz, CDCl₃) δ0.87 (t, J = 7, 3H), 0.96 (d, J = 9, 1H), 1.02-2.00 (m, 14H), 2.02-2.10 (m, 1H), 2.13-223 (m, 1H), 2.36-2.46 (m, 2 H), 2.58-2.70 (m, 2H), 3.83-3.90 (m,1H). 3.98-4.27 (m, 4 H), 5.82 (br d, J ∼ 9, 1H), 6.85-6.90 (m, 1H), 6.94-7.01 (m, 2 H), 727-7.34 (m, 1H).

### Compound 48

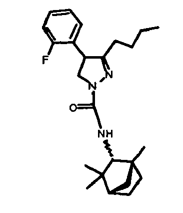

### N-[endo-(1R)-1,3,3-trimethylbicyclo[2.2.1]hept-2-yl]-3-(n-butyl)-4-(2-fluorophenyl)-4,5-dihydro-(1H)-pyrazole-1-carboxamide (from the isocyanate derived from endo-(1R)-1,3,3-trimethylbicyclo[2.2.1]heptan-2-amine

¹H-NMR (400 MHz, CDCl₃) δ 0.75-0.83 (m, 6H), 1.02, 1.03, 1.04, 1.05 (4 x singlet from diastereomeric CH₃ groups, 6H), 1.08-1.70 (m, 11 H), 1.95-2.18 (m, 2H), 3.48 (br d, J ∼ 10, 1H), 3.76 3.84 (m, 1H), 4.08-4.17 (m, 1H), 4.37-4.47 (m, 1H), 5.99 (br d, J ∼ 10, 1H), 6.95 7.09 (m, 3H), 7.15-7.22 (m, 1H).

### Compound 49

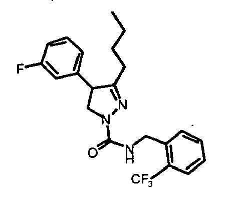

### N-[2-(trifluoromethyl)benzyl]-3-(n-butyl)-4-(3-fluorophenyl)-4,5-dihydro-(1H)-pyrazole-1-carboxamide

¹H-NMR (400 MHz, CDCl₃) δ 0.85 (t, J = 7,3H), 1.21-1.32 (m, 2H), 1.39-1.53 (m, 2H), 2.01-2.20 (m, 2H), 3.89 (dd, J=11 and 6.4, 1H), 4.09-4.15 (m, 1H), 4.23 (t, J = 11, 1H), 4.70 (d, J = 7, 2H), 6.36 (br t, J = 7, 1H), 6.84-6.89 (m, 1H), 6.92-7.02 (m; 2H), 7.28-7.40 (m,2H), 752-757 (m, 1H), 7.63-7.70 (m, 2H).

### Compound 50

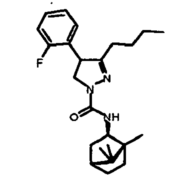

### N-[Exo-(1R,2R,4R)-1,7,7-trimethylbicyclo[2.2.1]hept-2-yl]-3-(n-butyl)-4-(2-fluorophenyl)-4,5-dihydro-(1H)-pyrazole-1-carboxamide (from the isocyanate derived from exo-1R-bornylamine.

¹H-NMR (400 MHz, CDCl₃) δ 0.81-0.92 (m, 10H), 0.97 (s, 3H), 1.11-1.77 (m, 9H), 1.89 (dd , J = 13 and 9, 1H), 2.03 2.22 (m, 2H), 3.80-3.90 (m, 2 H), 413-4.23 (m, 1H), 4.43-4.51 (m, 1H), 6.06 (br d, J∼ 9, 1H), 7.03-7.15 (m, 3H), 722-7.30 (m, 1H).

### Compound 51

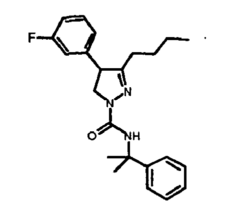

### N-(1-Methyl-1-phenyl-ethyl)-3-(n-butyl)-4-(3-fluoro phenyl)-4,5-dihydro-(1H)-pyrazole-1-carboxamide

¹H-NMR (400 MHz, CDCl₃) δ 0.87 (t, J = 7, 3 H), 1.24-1.56 (m, 4H), 1.75 (s, 3H), 1.77 (s, 3H), 2.02-2.11 (m, 1H), 2.15-2.24 (m, 1H), 3.81 (dd, J = 9.3 and 4.8 Hz, 1H), 4.07-4.19 (m, 2H), 6.36 (br s, 1H), 6.86-6.90 (m, 1H), 6.93-7.01 (m, 2 H), 7.20-7.37 (m, 4H), 7.45-7.50 (m, 2 H).

### Compound 52

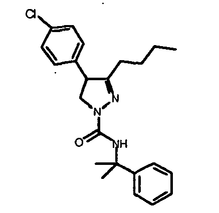

### N-(1-Methyl-1-phenyl-ethyl)-3-(n-butyl)-4-(4-chlorophenyl)-4,5-dihydro-(1H)-pyrazole-1-carboxamide

¹H-NMR (400 MHz, CDCl₃) δ 0.87 (t, J = 7, 3 H), 1.23-1.55 (m, 4H), 1.74 (s, 3H), 1.78 (s, 3H), 1.99-2.09 (m, 1H), 2.12-2.22 (m, 1H), 3.78 (dd, J = 10 and 5.5 Hz, 1H), 4.05-4.19 (m, 2H), 6.36 (br s, 1H), 7.10 (bred, J = 8, 2H), 7.20-7.37 (m, 5H), 7.48 (br d, J = 8, 2H).

### Compound 53

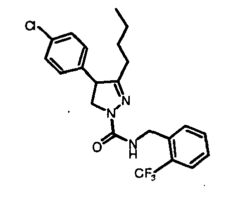

### N-[2-(trifluoromethyl)benzyl]-3-(n-butyl)-4-(4-chlorophenyl)-4,5-dihydro-(1H) pyrazole-1-carboxamide

¹H-NMR (400 MHz, CDCl₃) δ 0.85 (t, J = 7, 3H), 1.20-1.53 (m, 4H), 1.98-2.18 (m, 2H), 3.86 (dd, J=11 and 6.5, 1H), 4.08-4.15 (m, 1 H), 4.23 (t, J = 11, 1H), 4.69 (br d, J = 6.3, 2H), 6.36 (br t, J = 6.3, 1H), 7.09 (br d, J = 8, 2H), 7.31 (br d, J = 8, 2H), 7.35-7.41 (m,1H), 7.51-7.58 (m, 1H), 7.63-7.70 (m, 2H).

### Compound 54

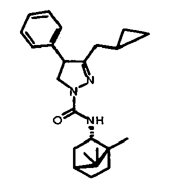

### N-[Endo-(1R,2S,4R)-1,7,7-trimethylbicyclo[2.2.1]hept-2-yl]-3-(cyclopropylmethyl)-4-phenyl-4,5-dihydro-(1H)-pyrazole-1-carboxamide (from the isocyanate derived from 1 R-(+)-bornylamine (CAS 32511-34-5)).

¹H-NMR (400 MHz, CDCl₃) δ -0.04-0.08 (m, 2 H), 0.39-0.53 (m, 2H), 0.75-0.94 (m, 8H), 0.97 (s, 3H), 121-129 (m, 1H), 1.35-1.46 (m,1H), 1.57-1.69 (m, 2H), 1.74-1.84 (m, 1H), 1 .90-1 .98 (m, 1H), 2.15-2.24 (m, 1H), 2.33-2.44 (m, 1H), 3.83-3.89 (m, 1H), 4.12-4.33 (m, 3H), 6.02-6.09 (m, 1H), 7.16 (br d, J = 8, 2H), 725-7.37 (m, 3H).

### Compound 55

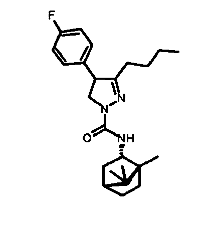

### N-[Endo-(1R,2S,4R)-1,7,7-trimethylbicyclo[2.2.1]hept-2-yl]-3-(n-butyl)-4-(4-fluorophenyl)-4,5-dihydro-(1H)-pyrazol-1-carboxamide (from the isocyanate derived from 1R-(+)-bornylamine (CAS 32511-34-5)).

¹H-NMR (400 MHz, CDCl₃) δ 0.85-0.95 (m, 10H), 0.97 (s, 3H), 1.20-1.69 (m, 9H), 1.73-1.85 (m, 1H). 2.01-2.10 (m, 1H), 2.14-224 (m, 1H). 2.34-2.45 (m, 1H). 3.79-3.86 (m, 1H), 4.08-4.25 (m, 2H), 6.01 (br d, J∼ 9, 1H), 700-7.06 (m, 2H), 7.11-7.16 (m, 2 H).

### Compound 56

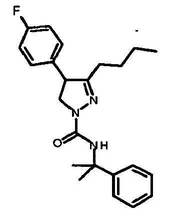

### N-(1-Methyl-1-phenyl-ethyl)-3-(n-butyl)-4-(4-fluorophenyl)-4,5-dihydro-(1H)-pyrazole-1-carboxamide

¹H-NMR (400 MHz, CDCl₃) δ 0.85 (t, J = 7, 3 H), 1.22-1.54 (m, 4H), 1.75 (s, 3H),1.77 (s, 3H), 2.00-2.09 (m, 1H), 2.13-2.22 (m, 1H), 3.78 (dd, J = 9 and 55, 1H), 4.07-4.18 (m, 2H), 6.36 (br s, 1H), 7.00-7.06 (m, 2H), 7.10-7.16 (m, 2H), 7.20-7.25 (m, 1H), 7.32-7.37 (m, 2H), 7.46-7.50 (m, 2H).

### Compound 57

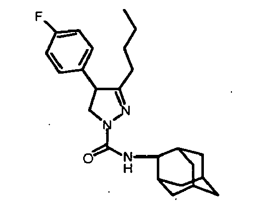

### N-(Adamant-2-yl)-3-(n-butyl)-4-(4-fluorophenyl)-4,5-dihydro-(1H)-pyrazole-1-carboxamide

¹H-NMR (400 MHz, CDCl₃) δ 0.85 (t, J = 7, 3H), 122-1.54 (m, 4H), 1.62-2.09 (m, 15H), 2.13 2.22 (m, 1H), 3.82 (dd, J = 10 and 6, 1H), 3.97-4.03 (m, 1H), 4.08-4.23 (m, 2H), 6.37 (br d, J = 9, 1H), 7.00 7.06 (m, 2H), 7.10-7.16 (m, 2H).

### Compound 58

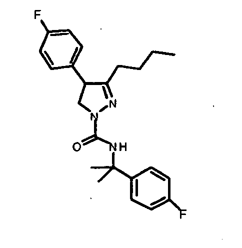

### N-(1-Methyl-1-(4-fluorophenyl)ethyl)-3-(n-butyl)-4-(4-fluorophenyl)-4,5-dihydro-(1H)-pyrazole-1-carboxamide

¹H-NMR (400 MHz, CDCl₃) δ 0.87 (t, J = 7, 3 H), 1.21-1.56 (m, 4H), 1.72 (s, 3H). 1.75 (s, 3H), 2.00 2.22 (m, 2H), 3.74-3.78 (m, 1H), 4.07-4.17 (m, 2H), 6.34 (br s, 1H). 6.98-7.06 (m, 4H), 7.09-7.15 (m, 2H), 7.40-7.46 (m, 2H).

LC/MS (Method D). Retention time:2.09 min; Found molecular mass = 400.

### Compound 59

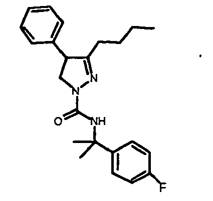

### N-(1-Methyl-1-(4-fluorophenyl)-ethyl)-3-(n-butyl)-4-phenyl-4,5-dihydro-(1H)-pyrazole-1-carboxamide

¹H-NMR (400 MHz, CDCl₃) δ 0.87 (t, J = 7, 3H), 122-1.35 (m, 2H), 1.38-1.57 (m, 2H), 1.72 (s, 3H), 1.75 (s, 3H), 2.01-2.22 (m, 2H), 3.78-3.82 (m, 1H). 4.09-4.19 (m, 2H), 6.35 (br s, 1H), 6.98-7.04 (m, 2H), 7.13-7.17 (m, 2H), 7.25 7.37 (m, 3H), 7.41 - 7.47 (m, 2H).

LC/MS (Method D). Retention time: 2.05 min ; Found molecular mass = 382.

### Compound 60

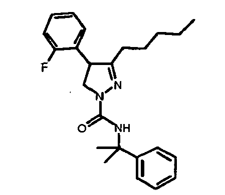

### N-(1-Methyl-1-phenyl-ethyl)-3-(n-pentyl)-4-(2-fluorophenyl)-4,5-dihydro-(1H)-pyrazole-1-carboxamide

LC/MS (Method D). Retention time: 2.13 min; Found molecular mass = 396.

### Compound 61

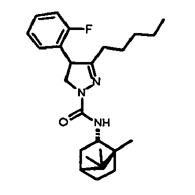

### N-[Endo-(1R,2S,4R)-1,7,7-trimethylbicyclo[2.2.1]hept-2-yl]-3-(n-pentyl)-4-(2-fluorophenyl)-4,5-dihydro-(1H)-pyrazole-1-carboxamide (from the isocyanate derived from 1R-(+)-bornylamine (CAS 32511-34-5)).

LC/MS (Method D). Retention time:2.33 min; Found molecular mass = 414.

### Compound 62

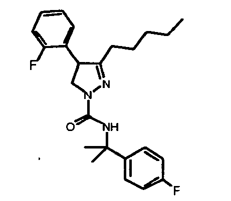

### N-(1-Methyl-1-(4-fluorophenyl)-ethyl-3-(n-pentyl)-4-(2-fluorophenyl)-4,5-dihydro-(1H)-pyrazole-1-carboxamide

LC/MS (Method D). Retention time: 2.12 min ; Found molecular mass = 414.

### Compound 63

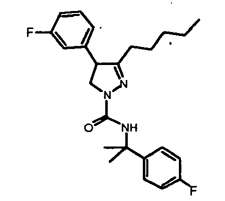

### N-(1-Methyl-1-(4-fluorophenyl)-ethyl)-3-(n-pentyl)-4-(3-fluorophenyl)-4,5-dihydro-(1H)-pyrazole-1-carboxamide

LC/MS (Method D). Retention time:2.12 min ; Found molecular mass = 414.

### Compound 64

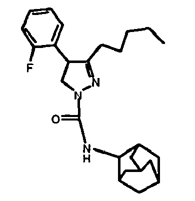

### N-(adamant-2-yl)-3-(n-pentyl)-4-(2-fluorophenyl)-4,5-dihydro-(1H)-pyrazole-1-carboxamide

LC/MS (Method D). Retention time: 2.36 min; Found molecular mass = 412.

### Compound 65

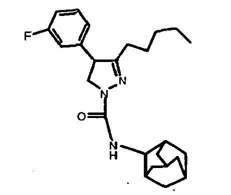

### N-(adamant-2-yl)-3-(n-pentyl)-4-(3-fluorophenyl)-4,5-dihydro-(1H)-pyrazole-1-carboxamide

LC/MS (Method D). Retention time: 2.36 min; Found molecular mass = 412.

### Compound 66

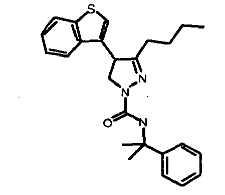

### N-(1-Methyl-1-phenyl-ethyl)-3-(n-butyl)-4-(benzo[b]thiophen-3-yl)-4,5-dihydro-(1H)-pyrazole-1-carboxamide

LC/MS (Method D). Retention time: min; Found molecular mass = 419.

### Compound 67

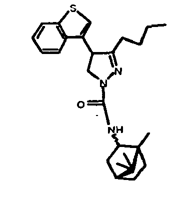

### N-[Endo-(1R,2S,4R)-1,7,7-trimethylbicyclo[2.2.1]hept-2-yl]-3-(n-butyl)-4-(benzo[b]thiophen-3-yl)-4,5-dihydro-(1H)-pyrazole-1-carboxamide (from the isocyanate derived from 1 R-(+)-bornylamine (CAS 32511-34-5)).

LC/MS (Method D). Retention time:2.34 min; Found molecular mass = 438.

### Compound 68

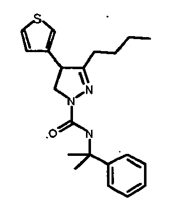

### N-(1-Methyl-1-phenyl-ethyl)-3-(n-butyl)-4-(thiophen-3-yl)-4,5-dihydro-(1H)-pyrazole-1-carboxamide

¹H-NMR (400 MHz, CDCl₃) δ 0.85 (t, J = 7, 3 H), 1.21-1.57 (m, 4H), 1.74 (s, 3H), 1.77 (s, 3H), 2.05-2.25 (m, 2H), 3.79 (dd, J~11 and 7, 1H), 4.08-4.13 (m, 1H), 4.28 (dd, J - 11 and 7, 1H) 6.36 (br s, 1H), 6.91 (dd, J= 6 and 2, 1H), 7.06-7 D8 (m, 1H), 7.19-7.24 (m, 1H), 7.30-7.37 (m, 3 H), 7.45-7.49 (m, 2H).

LC/MS (Method D). Retention time: 2.00 min ; Found molecular mass = 370.

### Compound 69

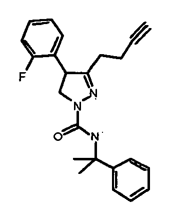

### N-(1-Methyl-1-phenyl-ethyl)-3-(but-3-ynyl)-4-(2-fluorophenyl)-4,5-dihydro-(1H)-pyrazole-1-carboxamide

LC/MS (Method D). Retention time: 1.80 min; Found molecular mass = 378.

### Compound 70

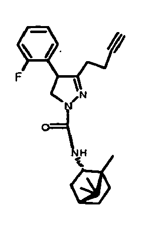

### N-[Endo-(1R,2S,4R)-1,7,7-trimethylbicyclo[2.2.1]hept-2-yl]-3-(but-3-ynyl)-4-(2-fluorophenyl)-4,5-dihydro-(1H)-pyrazole-1-carboxamide (from the isocyanate derived from 1R-(+)-bornylamine (CAS 32511-34-5)).

LC/MS (Method D). Retention time: 1.99 min; Found molecular mass = 396.

### Compound 71

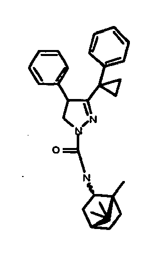

### N-[Endo-(1 R,2S,4R)-1,7,7-trimethylbicyclo[2.2.1]hept-2-yl]-3-(1-phenylcyclopropyl )-4-phenyl-4,5 -dihydro-(1H)-pyrazole-1-carboxamide (from the isocyanate derived from 1 R-(+)-bornylamine (CAS 32511-34-5)).

LC/MS (Method D). Retention time:2.27 min; Found molecular mass = 442.

### Compound 72

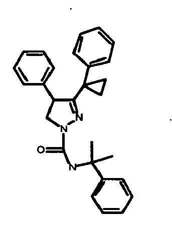

### N-(1-Methyl-1-phenyl-ethyl)-3-(1-phenylcyclopropyl)-4-phenyl-4,5-dihydro-(1H)-pyrazole-1-carboxamide

LC/MS (Method D). Retention time: 2.10 min; Found molecular mass = 424.

### Compound 73

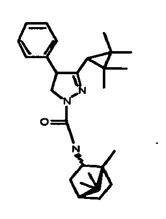

### N-[Endo-(1 R,2S,4R)-1,7,7-trimethylbicyclo[2.2.1]hept-2-yl]-3-(2,2,3,3-tetramethylcyclopropyl)-4-phenyl-4,5-dihydro-(1H)-pyrazole-1-carboxamide (from the isocyanate derived from 1 R-(+)-bornylamine (CAS 32511 -34-5)).

LC/MS (Method D). Retention time: 2.46 min; Found molecular mass = 422.

### Compound 74

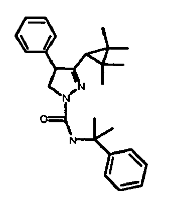

### N-(1-Methyl-1-phenyl-ethyl)-3-(2,2,3,3 -tetramethylcyclopropyl)-4-phenyl-4,5-dihydro-(1H)-pyrazole-1-carboxamide

LC/MS (Method D). Retention time: 2.21 min; Found molecular mass = 404.

### Compound 75

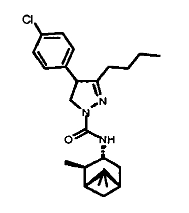

### N-[(1R,2R,3R,5S)-2,7,7-trimethylbicyclo[3.1.1]hept-3-yl]-3-(n-butyl)4-(4-chlorophenyl)-4,5-dihydro-(1H)-pyrazole-1-carboxamide (from the isocyanate derived from (1 R,2R,3R,5S)-(-)-isopinocampheylamine (CAS 69460-11-3)).

LC/MS (Method D). Retention time: 2.37 min ; Found molecular mass = 416.

### Compound 76

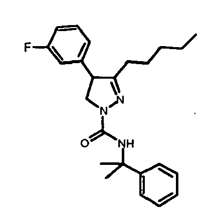

### N-(1-Methyl-1-phenyl-ethyl)-3-(n-pentyl)-4-(3-fluorophenyl)-4,5-dihydro-(1H)-pyrazole-1-carboxamide

LC/MS (Method D). Retention time: 2.15 min; Found molecular mass = 396.

### Compound 77

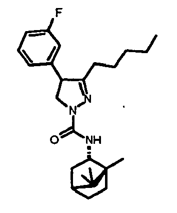

### N-[Endo-(1R,2S,4R)-1,7,7-trimethylbicyclo[2.2.1]hept-2-yl]-3-(n-pentyl)-4-(3-fluorophenyl)-4,5-dihydro-(1H)-pyrazole-1-carboxamide (from the isocyanate derived from 1 R-(+)-bornylamine (CAS 32511-34-5)).

LC/MS (Method D). Retention time: 2.32 min; Found molecular mass = 414.

### Compound 78

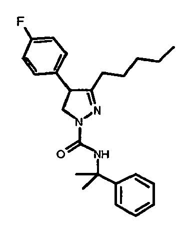

### N-(1-Methyl-1-phenyl-ethyl)-3-(n-pentyl)-4-(4-fluorophenyl)-4,5-dihydro-(1H)-pyrazole-1-carboxamide

LC/MS (Method D). Retention time:2.07 min; Found molecular mass = 396.

### Compound 79

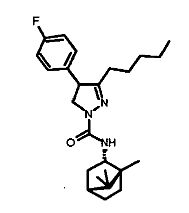

### N-[Endo-(1R,2S,4R)-1,7,7-trimethylbicyclo[2.2.1]hept-2-yl]-3-(n-pentyl)-4-(4-fluorophenyl)-4,5-dihydro-(1H)-pyrazole-1-carboxamide (from the isocyanate derived from 1R-(+)-bornylamine (CAS 32511-34-5)).

LC/MS (Method D). Retention time: 2.31 min; Found molecular mass = 414.

### Compound 80

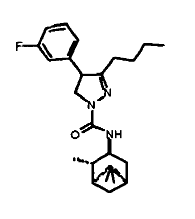

### N-[(1S,2S,3S,5R)-2,7,7-trimethylbicyclo[3.1.1]hept-3-yl]-3-(n-butyl)-4-(3-fluorophenyl)-4,5-dihydro-(1H)-pyrazole-1-carboxamide (from the isocyanate derived from (1 S,2S,3S,5R)-(+)-isopinocampheylamine.

LC/MS (Method D). Retention time:2.23 min; Found molecular mass = 400.

### Compound 81

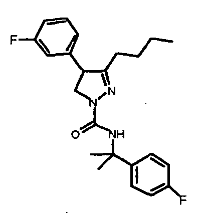

### N-(1-Methyl-1-(4-fluorophenyl)-ethyl)-3-(n-bu ty)-4-(3-fluoro phenyl)-4,5-dihydro-(1H)-pyrazole-1-carboxamide

LC/MS (Method D). Retention time:2.08 min; Found molecular mass = 400.

### Compound 82

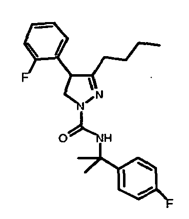

### N-(1-Methyl-1-(4-fluorophenyl)-ethyl)-3-(n-butyl)-4-(2-fluorophenyl)-4,5-dihydro-(1H)-pyrazole-1-carboxamide

LC/MS (Method D). Retention time: 2.05 min; Found molecular mass = 400.

### Compound 83

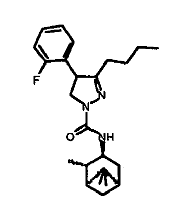

### N-[(1S,2S,3S,5R)-2,7,7-trimethylbicyclo[3.1.1]hept3-yl]-3-(n-butyl)-4-(2-fluorophenyl)-4,5-dihydro-(1H)-pyrazole-1-carboxamide (from the isocyanate derived from (1 S,2S,3S,5R)-(+)-isopinocampheylamine.

LC/MS (Method D). Retention time: 2.23 min; Found molecular mass = 400.

### Compound 84

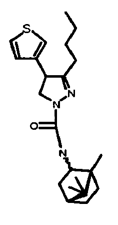

### N-[Endo-(1R,2S,4R)-1,7,7-trimethylbicyclo[2.2.1]hept-2-yl]-3-(n-butyl)-4-(thien-3-yl)-4,5-dihydro-(1H)-pyrazole-1-carboxamide (from the isocyanate derived from 1 R-(+)-bornylamine (CAS 32511 -34-5)).

LC/MS (Method D). Retention time: 2.21 min ; Found molecular mass = 387.

### Compound 85

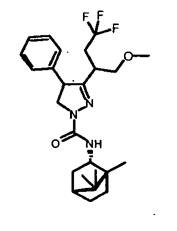

### N-[Endo-(1R,2S,4R)-1,7,7-trimethylbicyclo[2.2.1]hept-2-yl]-3-(3,3,3-trifluoro-1-methoxymethyl-propyl)-4-phenyl-4,5-dihydro-(1H)-pyrazole-1-carboxamide, mixture of diastereomer A and diastereomer B

**Part A:** 6,6,6-Trifluoro-4-methoxymethyl-2-phenyl-hex-1-en-3-one (Intermediate III-2) was converted with hydrazine hydrate to 3-(3,3,3-trifluoro-1-methoxymethyl-propyl)-4-phenyl-4,5-dihydro-(1H)-pyrazole (Intermediate IV-2) analogously to the procedure described for the synthesis of intermediate IV-1.
**Part B**: 3-(3,3,3-trifluoro-1-methoxymethyl-propyl)-4-phenyl-4,5-dihydro-(1H)-pyrazole was converted to N-[(1R,2S,5R)rel-6,6-dimethylbicyclo[3.1.1]heptan-2-methyl]-3-(3,3,3-trifluoro-1-methoxymethyl-propyl)-4-phenyl-4,5-dihydro-(1 H)-pyrazole-1-carboxamide analogously to the procedure described for the synthesis of compound 13 (via reaction with the isocyanate derived from 1 R-(+)-bornylamine (CAS 32511 -34-5)). This reaction gave a mixture of diastereoisomers. A mixture containing diastereomer A and diastereomer B was obtained via Sepacore chromatographic purification (petroleum ether/diethyl ether = 1/1 (v/v)). R_{f} (diastereomer A) = 0.15, R (diastereomer B) = 0.20. ¹H-NMR (400 MHz, CDCl₃); Mixture containing diastereomer A and diastereomer B:δ 0.82-0.94 (m, 7 H), 0.97 (s, 3H), 1.08-1.61 (m, 3H), 1.68 (br t, J = 4.5, 1H), 1.74-1.84 (m, 1H), 2.23-2.49 (m, 3H), 2.78-2.85 (m, 1H), 3.13 and 3.15 (2xs, (OCH₃ signals, 3H), 3.17-3.35 (m, 2H), 3.93-3.98 (m, 1H), 4.13-4.28 (m, 3H), 5.93 (br d, J - 9, 1H), 7.19 (br d, J - 8, 2H), 728-7.38 (m, 3H).

### Compound 86

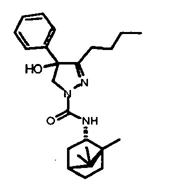

### N-[Endo-(1R,2S,4R)-1,7,7-trimethylbicyclo[2.2.1]hept-2-yl]-3-(n-butyl)-4-hydroxy-4-phenyl-4,5-dihydro-(1H)-pyrazole-1-carboxamide

**Part A:** 1-Phenylhexan-2-one was reacted in methanol with piperidine and acetic acid, followed by a formaldehyde solution (35 % solution in water) and the resulting mixture was stirred at 55 °C for 60 hours analogously to the procedure described for the synthesis of intermediate III-1 to give 2-phenyl-hept-1-en-3-one (intermediate III-3) in 70 % yield. ¹H-NMR (400 MHz, CDCl₃) δ 0.91 (t, J = 7, 3H), 1.29-1.40 (m, 2H), 1.59-1.69 (m, 2H), 2.72 (t, J = 7, 2H), 5.87 (s, 1H), 6.09 (s, 1H), 7.28-7.40 (m, 5H).
**Part B:** To a mixture of 2-phenyl-hept-1 en-3-one (3.76 g, 0.02 mol), 12 ml H₂O₂ (37 % aqueous solution) in 20 ml methanol is slowly added a mixture of 2 ml water and 1 ml concentrated aqueous NaOH (Cf. EP0114487). The resulting mixture is cooled to room temperature and stirred for 16 hours. The mixture is poured into water and extracted twice with diethyl ether. The diethyl ether layers were combined and filtered over hyflo and successively washed with water, aqueous acetic acid solution and brine. The resulting solution is dried over Na₂SO₄, filtered and concentrated to give 2.79 gram impure product. Flash chromatography (petroleum ether/ethyl ether = 49/1 (v/v) of the crude product gave 1.31 g 1-(2-phenyloxiranyl)-pentan-1-one (Intermediate V-1) as an oil in 32 % yield. ¹H-NMR (400 MHz, CDCl₆) δ 0.88 (t, J = 7, 3H), 1.23-1.35 (m, 2H), 1.47-1.63 (m, 2H), 2.40-2.61 (m, 2H), 3.02 (d, J = 6, 1H), 3.24 (d, J = 6, 1H), 732-7.40 (m, 3H), 7.45-7.50 (M, 2H).
**Part C:** 1-(2-Phenyloxiranyl)-pentan-1-one was converted with hydrazine hydrate to 3-(n-butyl)-4-hydroxy-4-phenyl-4,5-dihydro-(1H)-pyrazole (Intermediate IV-3) analogously to the procedure described for the synthesis of intermediate IV-1.
**Part E:** 3-(n-Butyl)-4-hydroxy-4-phenyl-4,5-dihydro-(1H)-pyrazole was converted to N-[(1R,2S,5R)-rel-6,6-dimethylbicyclo[3.1.1]heptan-2-methyl]-3-(n-butyl)-4-hydroxy-4-phenyl-4,5-dihydro-(1H)-pyrazole-1-carboxamide analogously to the procedure described for the synthesis of compound 13 (via reaction with the isocyanate derived from 1R-(+)-bornylamine (CAS 32511-34-5)). ¹H-NMR (400 MHz, CDCl₃) δ 0.81-0.94 (m, 10H), 0.96 (s, 3H), 121-1.32 (m, 3H), 1.35-1.70 (m, 5 H), 1.74-1.86 (m, 1H), 2.01-2.11 (m, 1H), 2.15-2.28 (m, 1H), 2.32-2.45 (m, 1H), 3.10 and 3.65 (2x br s, OH, 1H), 4.01-4.20 (m, 3H), 6.06-6.14 (m, 1H), 7.27-7.43 (m, 5 H).

### Compound 87

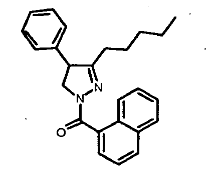

### 1-(1-Naphtoyl)-3-(n-pentyl)-4-phenyl-4,5-dihydro-(1H)-pyrazole

3-(n-Pentyl)-4-phenyl-4,5-dihydropyrazole (0.75 gram, 3.47 mmol) was dissolved in toluene (10 ml) and treated with 1-naphtoyl chloride (0.522 ml, 3.47 mmol) and the resulting solution was stirred at room temperature for 16 hours. The solution was concentrated, followed by flash chromatographic purification (heptane/ethylacetate = 6:1 (v/v)) to give 1-(1-naphtoyl)-3-(n-pentyl)-4-phenyl-4,5-dihydro-(1H)pyrazole (690 mg) as an oil.

LC/MS (Method B). Retention time: 5.87 minutes: Found molecular mass (API-ES; positive scan) = 371. Mobile phase gradient: 0 - 5 minutes: Solution A/Solution B = 30/70 (v/v)). > 5 minutes: Solution B.

R_{f} (dichloromethane/methanol = 99/1 (v/v)) = 0.35.

¹H-NMR (400 MHz, CDCl₃) δ 0.80-0.90 (m, 3H), 1.02-1.40 (m, 6H), 1.92-2.11 (m, 2H), 4.21-4.30 (m, 2 H), 4.57-4.65 (m, 1H), 7.20 (d, J = 8, 2H), 7.29-7.55 (m, 6H), 7.66 (d, J = 8, 1H), 7.84-7.94 (m, 2 H), 8.03 (br d, J = 8, 1H).

### Analogously were prepared compounds 88-94:

### Compound 88

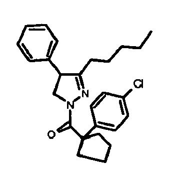

### [3-(n-pentyl)-4-phenyl-4,5-dihydro-(1H)-pyrazol-1-yl]-[1-(4-chlorophenyl) cyclopentyl] methanone

LC/MS (Method C). Retention time: 4.05 min ; Found molecular mass = 423.

### Compound 89

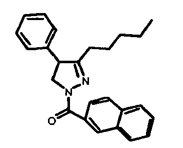

### [3-(n-pentyl)-4-phenyl-4,5-dihydro-(1H)-pyrazol-1-yl]-(napht-2-yl) methanone

LC/MS (Method C). Retention time: 3.52 min ; Found molecular mass = 371.

### Compound 90

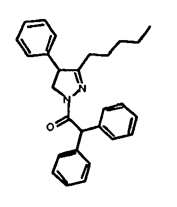

### [3-(n-pentyl)-4-phenyl-4,5-dihydro-(1H)pyrazol-1-yl]-(diphenylmethyl) methanone

LC/MS (Method C). Retention time: 3.81 min; Found molecular mass = 411.

### Compound 91

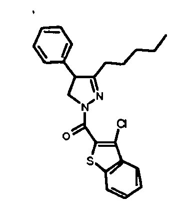

### [3-(n-pentyl)-4-phenyl-4,5-dihydro-(1H)-pyrazol-1-yl]-(3-chlorobenzothien-2-yl] methanone

LC/MS (Method C). Retention time: 3.77 min; Found molecular mass = 411.

### Compound 92

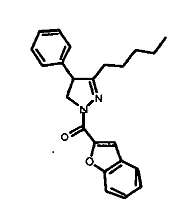

### [3-(n-pentyl)-4-phenyl-4,5-dihydro-(1H)-pyrazol-1-yl]-(benzofuran-2-yl] methanone

LC/MS (Method C). Retention time: 3.48 min ; Found molecular mass = 361.

### Compound 93

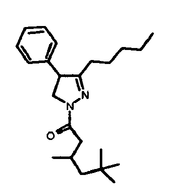

### [3-(n-pentyl)-4-phenyl-4,5-dihydro-(1H)-pyrazol-1-yl]-[2,4,4 -(trimethyl)pentyl] methanone

LC/MS (Method C). Retention time: 3.98 min; Found molecular mass = 357.

### Compound 94

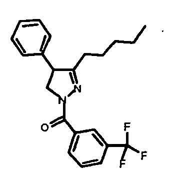

### [3-(n-pentyl)-4-phenyl-4,5-dihydro-(1H)-pyrazol-1-yl]-[3-(trifluoromethyl)phenyl] methanone

¹H-NMR (400 MHz, CDCl₃) δ 0.85 (t, J = 7, 3H), 1.19-1.30 (m, 4H), 1.44-1.60 (m, 2H), 2.05-2.23 (m, 2H), 4.10-4.25 (m, 2H), 4.51 (t, J = 11, 1H), 7.15-7.20 (m, 2H), 7.29-7.41 (m, 3H), 7.54-7.59 (m, 1H), 7.73 (d, J = 8, 1H), 8.18 (d, J = 8, 1H), 8.33 (br s, 1H).

### Compound 95

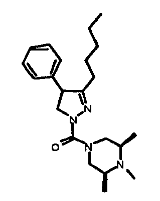

### (Cis,4,5-trimethylpiperazin-1-yl)[3-(n-pentyl)-4-phenyl-4,5-dihydro-(1H)-pyrazol-1-yl] methanone

**Part A**: To a magnetically stirred solution of N-(tert-butoxycarbonyl)-cis-3,5-dimethylpiperazine (19.7 gram, 90 mmol) in 1,4-dioxane (400 ml) was successively added a mixture of NaOH (230 ml of a 2N solution, 460 mmol) and phosphorous acid (230 ml of a 2M solution in 230 ml water, 460 mmol) followed by formaldehyde (110 ml, 37 % solution in water, 1.46 mol) and the resulting mixture was reacted for 3.5 hours at 63 °C. The reaction mixture was allowed to attain room temperature and extracted twice with dichloromethane. The organic layers were collected and washed with water and brine respectively and subsequently dried over Na₂SO₄, filtered and concentrated to give crude N-tert-butoxycarbonyl-cis-3,4,5-trimethylpiperazine (12 gram).
**Part B:** To a magnetically stirred solution of the crude N-Hert-butoxycarbonyl-cis-3,4,5-trimethylpiperazine (12 gram, - 53 mmol) in dichloromethane (180 ml) was added excess trifluoroacetic acid (40 ml) and the resulting mixture was stirred at room temperature overnight Aqueous NaOH was added and the reaction mixture was twice extracted with dichloromethane (2x100 ml). The organic layers were collected, dried over Na₂O₄, filtered and concentrated to give cis-3,4,5-trimethylpiperazine (3.44 gram, ~ 30 % yield). ¹H-NMR (400 MHz, CDCl₃) δ 1.05 (d, J = 6, 6H), 1.65 (br s, 1H), 2.03-2.13 (m, 2H), 2.27 (s, 3H), 2.53 (d, J ~ 10, 1H), 257 (d, J ~ 10,1H), 2.82-2.88 (m, 2H).
**Part C:** To a magnetically stirred solution of cis-3,4,5-trimethylpiperazine (1.5, gram, 12.7 mmol) in toluene (25 ml) was added phosgene (8 ml of a 20 % solution in toluene, 15 mmol) and triethylamine (1.7 ml) and a catalytic amount of dimethylaminopyridine (DMAP). The resulting solution was stirred for 10 minutes at room temperature and 3-(n-pentyl)-4-phenyl-4,5-dihydropyrazole (2.5 gram, 12 mmol) was added and the resulting mixture was stirred at room temperature for 16 hours. The mixture was then concentrated in vacuo, followed by flash chromatographic purification (dichloromethane/7M NH₃ in methanol = 97.5/2.5 (v/v)) to give (cis-3,4,5-trimethylpiperazin-1-yl)[3-(n-pentyl)-4-phenyl-4,5-dihydro-(1H)-pyrazol-1-yl] methanone (compound 26) (1.9 gram) as an oil. ¹H-NMR (300 MHz, CDCl₃) δ 0.81-0.87 (m, 3H), 1.11 (d, J = 6, 6H), 1.21 -1.26 (m, 4H), 1.44-1.50 (m, 2H), 2.00-2.30 (m, 7H), 2.71-2.82 (m, 2 H), 3.82 (dd, J - 11 and 7, 1H), 3.97 (dd, J - 11 and 7, 1H), 4.13-4.23 (m, 3H), 7.14-7.18 (m, 2H), 7.25-7.36 (m, 3H).

### Compounds 96 and 97

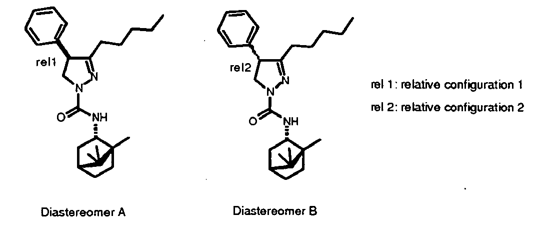

### N-Endo-[(1R,2S,4R)-1,7,7-trimethylbicyclo[2.2.1]hept-2-yl]-3-(n-pentyl)-4-phenyl-4,5-dihydro-(1H)-pyrazole-1-carboxamide (compound 27, diastereomer A) and N-Endo-[(1R,2S,4R)-1,7,7-trimethylbicyclo[2.2.1]hept-2-yl]-3-(n-pentyl)-4-phenyl-4,5-dihydro-(1H)-pyrazole-1-carboxamide (compound 28, diastereomer B)

Preparative HPLC separation of compound 13 gave compounds 96 and 97 respectively. Preparative HPLC separation procedure: A prepHPLC column LC80 (internal diameter: 8 cm) was packed with 800 grams of Chiralpak AD, 20 µ. Aceton/methanol (95/5 (v/v)) was used as the mobile phase. UV detection 220 nm. Flowrate: 2 ml/minute. Compound 96: Optical rotation ([α]_{D}) = +124 (c = 1.3, MeOH). ¹H-NMR (400 MHz, CDCl₃) δ 0.80-0.92 (m, 10H), 0.97 (s, 3H), 120-1.69 (m, 10H), 1.74-1.83 (m, 1H), 2.00-222 (m, 2H), 2.33-2.45 (m, 1H), 3.83-3.89 (m, 1H), 4.09-4.27 (m, 3H), 6.02 (br d, J - 10, 1H), 7.16 (br d, J - 8, 2H), 727-7.37 (m, 3H). ¹³C-NMR (100 MHz, CDCl₃) δ 13.74, 13.93, 18.74, 20.00, 22.32, 25.76, 28.05, 28.27, 28.45, 31.35, 38.20, 44.97, 47.99, 49.29, 53.30, 53.58, 54.42, 127.54, 127.64, 129.05, 139.67, 155.87, 158.88.

Compound 97: Optical rotation ([α]_{D}) = - 85 (c = 1.55, MeOH). ¹H-NMR (400 MHz, CDCl₃) δ 0.80-0.94 (m, 10H), 0.97 (s, 3H), 120-1.69 (m, 10H), 1.74-1.83 (m, 1H), 2.00-2.22 (m, 2H), 2.33-2.45 (m, 1H), 3.83-389 (m, 1H), 4.09-4.27 (m, 3H), 6.02 (br d, J - 10, 1H), 7.16 (br d, J - 8, 2H), 727-7.37 (m, 3H). ¹³C-NMR (100 MHz, CDCl₃) δ 13.73, 13.93, 18.73, 20.00, 22.31, 25.75, 28.03, 28.26, 28.46,31.36, 38.12, 44.99, 48.00, 49.37, 53-34, 53.62, 54.41, 127.56, 127.68, 129.06, 139.71, 155.78, 158.83.

### Compounds 98 and 99

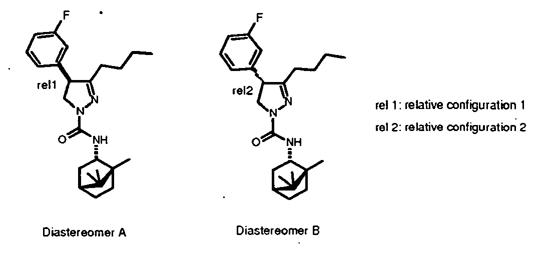

### N-Endo-[(1R,2S,4R)-1,7,7-trimethylbicyclo[2.2.1]hept-2-yl]-3-(n-butyl)-4-(3-fluorophenyl)-4,5-dihydro-(1H)-pyrazole-1-carboxamide (compound 98, diastereomer A) and N-Endo-[(1R,2S,4R)-1,7,7-trimethylbicyclo[2.2.1] hept-2-yl]-3-(n-butyl)-4-(3-fluorophenyl)-4,5-dihydro-(1H)-pyrazole-1-carboxamide (compound 99, diastereomer B)

Column chromatographic separation (gradient: petroleum ether to petroleumether/ethylacetate = 4/1 (v/v)) of N-endo-[(1 R,2S,4R)-1,7,7-trimethylbicyclo[2.2.1]hept-2-yl]-3-(n-butyl)-4-(3-fluorophenyl)-4,5-dihydro-(1H)-pyrazole-1-carboxamide gave compounds 98 and 99, respectively. Compound 98: Optical rotation ([α]_{D}) = -116 (c = 1.16, MeOH). ¹H-NMR (400 MHz, CDCl₃) δ 0.84-0.95 (m, 10H), 0.97 (s, 3H), 1.21-1.69 (m, 8H), 1.73-1.84 (m, 1H), 2.02-2.11 (m, 1H), 2.16-2.26 (m, 1H), 2.35-2.45 (m, 1H), 3.86 (dd, J = 11 and 7, 1H), 4.09-4.23 (m, 3H), 6.01 (br d, J ~ 9, 1H), 6.88 (br d, J ~ 8, 1H), 6.94-7.02 (m, 2 H), 727-7.34 (m, 1H). Compound 99: Optical rotation ([α]_{D}) = + 127 (c = 1.0, MeOH). ¹H-NMR (400 MHz, CDCl₃) δ 0.84-0.95 (m, 10H), 0.97 (s, 3H), 121-1.69 (m, 8H), 1.73-1.84 (m, 1H), 2.02-2.11 (m, 1H), 2.16-2 26 (m, 1H), 2.35-2.45 (m, 1H), 3.86 (dd, J = 11 and 7, 1H), 4.09-4.23 (m, 3H), 6.01 (br d, J - 9, 1H), 6.88 (br d, J - 8, 1H), 6.94-7.02 (m, 2H), 7.27-7.34 (m, 1H).

### Compounds 100 and 101

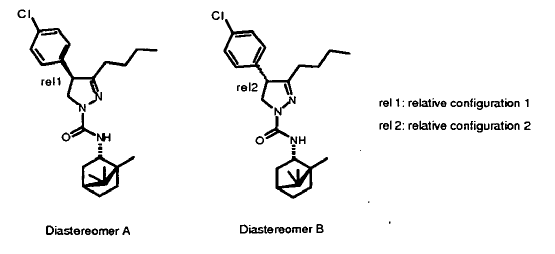

### N-Endo-[(1R,2S,4R)-1,7,7-trimethylbicyclo[2.2.1]hept-2-yl]-3-(n-butyl)-4-(4-chlorophenyl)-4,5-dihydro-(1H)-pyrazole-1-carboxamide (compound 100, diastereomer A) and N-Endo-[(1R,2S,4R)-1,7,7-trimethylbicyclo[2.2.1] hept-2-yl]-3-(n-butyl)-4-(4-chlorophenyl)-4,5-dihydro-(1H)-pyrazole-1-carboxamide (compound 101, diastereomer B)

Column chromatog raphic separation (gradient: petroleum ether to petroleumether/ethylacetate = 4/1 (v/v)) of N-endo-[(1R,2S,4R)-1,7,7-trimethylbicyclo[2.2.1]hept-2-yl]-3-(n-butyl)-4-(4-chlorophenyl)-4,5-dihydro-(1H)-pyrazole-1-carboxamide gave compounds 31 and 32, respectively. Compound 100: Optical rotation ([α]_{D}) = -120 (c = 1.0, MeOH). ¹H-NMR (400 MHz, CDCl₃) δ 0.82-0.94 (m, 10H), 0.97 (s, 3H), 1.20-1.69 (m, 8H), 1.73-1.84 (m, 1H), 2.00-2.09 (m, 1H), 2.13-2.23 (m, 1H), 2.34-2.44 (m, 1H), 3.83 (dd, J = 10.7 and 6.3, 1H), 4.07-4.23 (m, 3H), 6.01 (br d, J - 9, 1H), 7.11 (br d, J = 8.4, 2H), 7.32 (br d, J = 8.4, 2H).

Compound 101 : Optical rotation ([α]_{D}) = + 169 (c = 1.1, MeOH). ¹H-NMR (400 MHz, CDCl₃) δ 0.82-0.92 (m, 10H), 0.97 (s, 3H), 120-1.69 (m, 8H), 1.73-1.84 (m, 1H), 2.00-2.09 (m, 1H), 2.13-223 (m, 1H), 2.34-2.44 (m, 1H), 3.83 (dd, J = 10.7 and 6.3, 1H), 4.07-4.23 (m, 3H), 6.01 (br d, J - 9, 1H), 7.11 (br d, J = 8.4, 2H), 7.32 (br d, J = 8.4, 2H).

### Compound 102

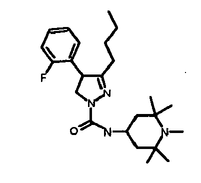

### N-(1,2,2,6,6-pentamethylpiperidin-4-yl)-3-(n-butyl)-4-(2-fluorophenyl)-4,5-dihydro-(1H)-pyrazole-1-carboxamide

To a magnetically stirred solution of 3-(n-butyl)-4-(2-fluorophenyl)-4,5-dihydro-(1H)-pyrazole-1-carbonyl chloride (Intermediate VIII-1) (1.26 g, 4.5 mmol) in dichloromethane (25 ml) was slowly added 1,2,2,6,6-pentamethylpiperidine (1.97 g, 11.6 mmol dissolved in 10 ml dichloromethane) and the resulting mixture was stirred for 16 hours at room temperature. The mixture was poured into water. The organic layer was separated and collected, dried over Na₂SO₄, filtered and concentrated in vacuo and subsequently purified by column chromatography (eluant: dichloromethane/methanol/25 % aqueous ammonia = 87.5/12/0/5 (v/v)) to give pure N-(1,2,2,6,6-pentamethylpiperidin-4-yl)-3-(n-butyl)-4-(2-fluorophenyl)-4,5-dihydro-(1H)-pyrazole-1 -carboxamide (1.35 g, 73 % yield)

LC/MS method C: Retention time: 1.27 minutes: Found molecular mass = 417.

### Compound 103

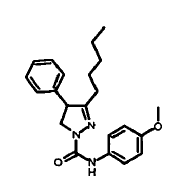

### N-(4-methoxyphenyl)-3-(n-pentyl)-4-phenyl-4,5-dihydro-(1H)-pyrazole-1-carboxamide

Compound 34 was prepared analogously to the procedure described for 34 U from 3-(n-pentyl)-4-phenyl-4,5-dihydro-(1H)-pyrazole-1-carbonyl chloride (Intermediate VIII-2) in dichloromethane in the presence of 1.2 mol equivalent DIPEA and 1.0 mol equivalent para-methoxyaniline. The mixture was reacted for 18 hours at 30°C to give N-(4-methoxyphenyl)-3-(n-pentyl)-4-phenyl-4,5-dihydro-(1H)-pyrazole-1-carboxamide.

LC/MS method C: Retention time: 3.28 minutes: Found molecular mass = 366.

### Analogously were prepared compounds 104-123:

### Compound 104

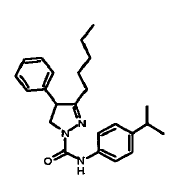

### N-(4-methoxyphenyl)-3-(n-pentyl)-4-phenyl-4,5-dihydro-(1H)-pyrazole-1-carboxamide

LC/MS method C: Retention time: 3.74 min; Found molecular mass = 378.

### Compound 105

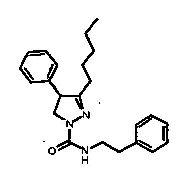

### N-(phenethyl)-3-(n-pentyl)-4-phenyl-4,5-dihydro-(1H)-pyrazole-1-carboxamide

LC/MS method C: Retention time: 3.34 min; Found molecular mass = 364.

### Compound 106

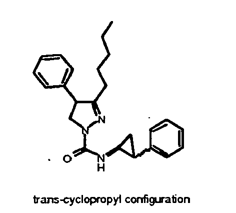

### N-(2-phenyl-trans-cyclopropyl)-3-(n-pentyl)-4-phenyl-4,5-dihydro-(1H)-pyrazole-1-carboxamide

LC/MS method C: Retention time: 3.40 min; Found molecular mass = 376.

### Compound 107

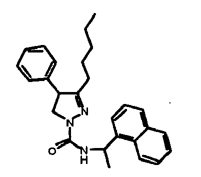

### N-(1-naphthalen-1-yl-ethyl)-3-(n-pentyl)-4-phenyl-4,5-dihydro-(1H)-pyrazole-1-carboxamide

LC/MS method C: Retention time: 3.61 min; Found molecular mass = 414.

### Compound 108

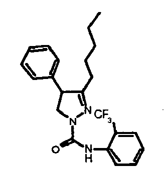

### N-[2-(trifluoromethyl)phenyl)-3-(n-pentyl)-4-phenyl-4,5-dihydro-(1H)-pyrazole-1-carboxamide

LC/MS method C: Retention time: 3.81 min; Found molecular mass = 404.

### Compound 109

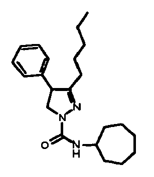

### N-cycloheptyl-3-(n-pentyl)-4-phenyl-4,5-dihydro-(1H)-pyrazole-1-carboxamide

LC/MS method C: Retention time: 3.74 min; Found molecular mass =356.

### Compound 110

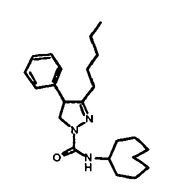

### N-cyclooctyl-3-(n-pentyl)-4-phenyl-4,5-dihydro-(1H)-pyrazole-1-carboxamide

LC/MS method C: Retention time: 3.81 min; Found molecular mass = 370.

### Compound 111

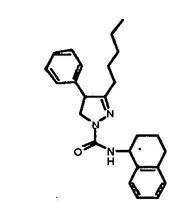

### N-(1,2,3,4 -tetrahydronaphthalen-1-yl)-3-(n-pentyl)-4-phenyl-4,5-dihydro-(1H)-pyrazole-1-carboxamide

LC/MS method C: Retention time: 3.61 min; Found mo lecular mass = 390.

### Compound 112

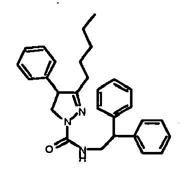

### N-[2,2-(diphenyl)ethyl]-3-(n-pentyl)-4-phenyl-4,5-dihydro-(1H)-pyrazole-1-carboxamide

LC/MS method C: Retention time: 3.59 min; Found molecular mass = 440.

### Compound 113

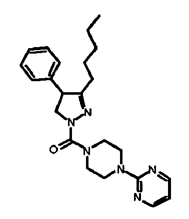

### (3-Pentyl-4-phenyl-4,5-dihydro pyrazol-1-yl)-[4-(2-pyrimidinyl)piperazin-1-yl]methanone

LC/MS method C: Retention time: 3.13 min; Found molecular mass =407.

### Compound 114

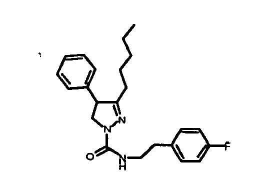

### N-[2-(4-fluorophenyl)-ethyl]-3-(n-pentyl)-4-phenyl-4,5-dihydro-(1H)-pyrazole-1-carboxamide

LC/MS method C: Retention time: 3.21 min; Found molecular mass = 382.

### Compound 115

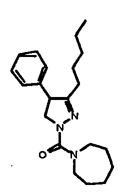

### (3-Pentyl-4-phenyl-4,5-dihydro-pyrazol-1-yl)-[azepan-1-yl]methanone

LC/MS method C: Retention time: 3.59 min; Found molecular mass = 342.

### Compound 116

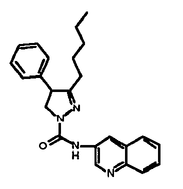

### N-(quinolin-3-yl)-3-(n-pentyl)-4-phenyl-4,5-dihydro-(1H)-pyrazole-1-carboxamide

LC/MS method C: Retention time: 3.08 min; Found molecular mass =387.

### Compound 117

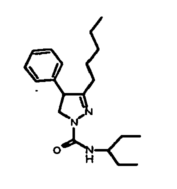

### N-[1-(ethyl)propyl]-3-(n-pentyl)-4-phenyl-4,5-dihydro-(1H)-pyrazole-1-carboxamide

LC/MS method C: Retention time: 3.30 min; Found molecular mass =330.

### Compound 118

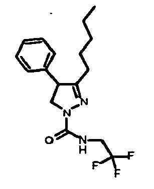

### N-(2,2,2-trifluoroethyl)-3-(n-pentyl)-4-phenyl-4,5-dihydro-(1H)-pyrazole-1-carboxamide

LC/MS method C: Retention time: 2.87 min; Found molecular mass = 342.

### Compound 119

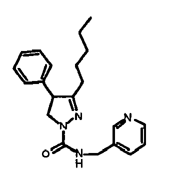

### N-(pyridin-3-ylmethyl)-3-(n-pentyl)-4-phenyl-4,5-dihydro-(1H)-pyrazole-1-carboxamide

LC/MS method C: Retention time: 2.41 min; Found molecular mass =351.

### Compound 120

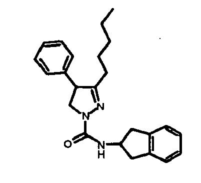

### N-(2-indanyl)-3-(n-pentyl)-4-phenyl-4,5-dihydro-(1H)-pyrazole-1-carboxamide

LC/MS method C: Retention time: 3.27 min; Found molecular mass =376.

### Compound 121

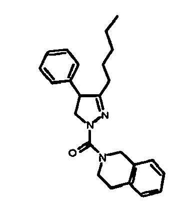

### (3-Pentyl-4-phenyl-4,5-dihydro pyrazol-1-yl)-(1,2,3,4-tetrahydroisoquinolin-2-yl)methanone

LC/MS method C: Retention time: 3.48 min; Found molecular mass = 376.

### Compound 122

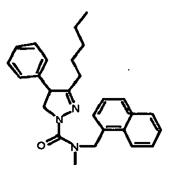

### N-(Methyl), N-(naphthalen-1-ylmethyl)-3-(n-pentyl)-4-phenyl-4,5-dihydro-(1H)-pyrazole-1-carboxamide

LC/MS method C: Retention time: 3.62 min; Found molecular mass =414.

### Compound 123

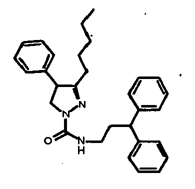

### N-(3,3-Diphenypropyl)-3-(n-pentyl)-4-phenyl-4,5-dihydro-(1H)-pyrazole-1-carboxamide

LC/MS method C: Retention time: 3.59 min; Found molecular mass = 454.

### Compound 124

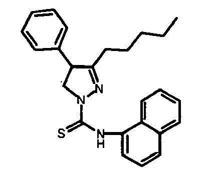

### N-(napht-1-yl)-3-(n-pentyl)-4-phenyl-4,5-dihydro-(1H)-pyrazolecarbothiamide

N-(napht-1-yl)-3-(n-pentyl)-4-phenyl-4,5-dihydro-(1H)-pyrazolecarbothiamide was obtained from 3-(n-pentyl)-4-phenyl-4,5-dihydropyrazole and an equimolar amount of 1-napthylisothiocyanate in tetrahydrofuran at 30°C for 5 hours. LC/MS (Method C). Retention time: 3.65 min; Found molecular mass = 402.

### Compound 125

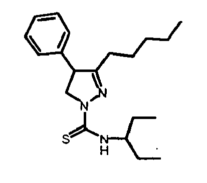

### N-[1-(ethyl)propyl]-3-(n-pentyl)-4-phenyl-4,5-dihydro-(1H)-pyrazolecarbothiamide

N-(1-(ethyl)propyl)-3-(n-pentyl)-4-phenyl-4,5-dihydro-(1H)-pyrazolecarbothiamide was obtained from 3-(n-pentyl)-4-phenyl-4,5-dihydropyrazole and an equimolar amount of 1-(ethyl)propylisothiocyanate in tetrahydrofuran at 30°C for 5 hours. LC/MS (Method C). Retention time: 3.69 min ; Found molecular mass = 346.

### Compound 126

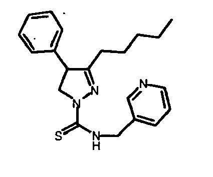

### N-[pyridin-3-ylmethyl]-3-(n-pentyl)-4-phenyl-4,5-dihydro-(1H)-pyrazolecarbothiamide

N-(pyridine-3-ylmethyl)-3-(n-pentyl)4-phenyl-4,5-dihydro-(1H)-pyrazolecarbo thiamide was obtained from 3-(n-pentyl)-4-phenyl-4,5-dihydropyrazole and an equimolar amount of pyridin-3-ylmethylisothiocyanate in tetrahydrofuran at 30°C for 5 hours. LC/MS (Method C). Retention time: 3.83 min; Found molecular mass = 367.

### Compound 127

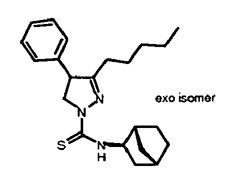

### N-[exo-bicyclo[2.2.1]hept-2-yl]-3-(n-pentyl)-4-phenyl-4,5-dihydro-(1H)-pyrazolecarbothiamide

N-[exo-bicyclo[2.2.1]hept-2-yl]-3-(n-pentyl)-4-phenyl-4,5-dihydro-(1H)-pyrazolecarbothiamide was obtained from 3-(n-pentyl)4-phenyl-4,5-dihydropyrazole and an equimolar amount of racemic exo-bicyclo[2.2.1]hept-2-ylisothiocyanate in tetrahydrofuran at 30°C for 5 hours. LC/MS (Method C). Retention time: 3.89 min; Found molecular mass = 370.

### Compound 128

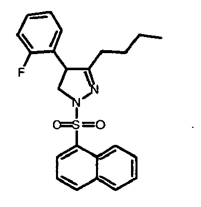

### 1-(Naphthalen-1-ylsulfonyl)-3-(n-butyl)-4-(2-fluorophenyl)-4,5-dihydro-(1H)-pyrazole

Crude 3-(n-butyl)-4-(2-fluorophenyl)-4,5-dihydropyrazole (Intermediate IV-3) (1.50 gram, 5.71 mmol maximally) was dissolved in dichloromethane (20 ml) and DIPEA (0.81 g, 1.09 ml, 6.28 mmol) and 1-naphthalenesulfonyl chloride (1.42 g, 6.28 mmol dissolved in 10 ml dichloromethane) were successively added and the resulting magnetically stirred solution was reacted at room temperature for 16 hours. The resulting mixture was poured into water. The organic layer was separated and collected, dried over Na₂SO₄, filtered and concentrated, followed by flash chromatographic purification (dichloromethane) to give 1-(naphthalen-1-ylsulfonyl)-3-(n-butyl)-4-(2-fluorophenyl)-4,5-dihydro-(1H)-pyrazole (2.07 g, 88 % yield). R_{f} = 0.4 (dichloromethane).

LC/MS (Method D). Retention time: 2.04 min; Found molecular mass = 411.

Analogously was prepared:

### Compound 129

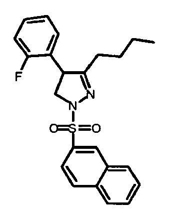

### 1-(Naphthalen-2-ylsulfonyl)-3-(n-butyl)-4-(2-fluorophenyl)-4,5-dihydro-(1H)-pyrazole

LC/MS (Method D). Retention time: 2.00 min; Found molecular mass = 411.

### Compound 130

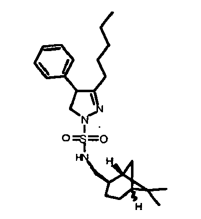

### N-[(1R,2S,5R)-rel-6,6-dimethylbicyclo[3.1.1]heptan-2-methyl]-3-(n-pentyl)-4-phenyl-4,5-dihydro-(1H)-pyrazole-1-sulfonamide

**Part A:** To a magnetically stirred solution of (-)-cis-myrtanylamine (2.0 g, 13 mmol) (CAS 382 35-68-6)) in dichloromethane (25 ml) was added triethylamine (4 ml) and chlorosulfonic acid (0.865 ml, 13 mmol, dissolved in dichloromethane (5 ml)) at 0 °C. The resulting solution was allowed to attain room temperature and reacted for 16 hours.
   The reaction mixture was poured in excess 1M hydrochloric acid. The precipitated crude [(1R,2S,5R)-rel-6,6-dimethylbicyclo[3.1.1]heptan-2-methyl]sulfamic acid (3.41 gram) was collected by filtration.
**Part B:** To a magnetically stirred solution of crude [(1R,2S,5R)-rel-6,6-dimethylbicyclo[3.1.1]heptan-2-methyl]sulfamic acid (3.41 g) in dichloro -methane (25 ml) was slowly added POCl₆ (2.78 ml POCl₃ dissolved in dichloromethane (25 ml)). The resulting mixture was heated at reflux temperature for 16 hours. Subsequent concentration in vacuo gave crude [(1R,2S,5R)-rel-6,6-dimethylbicyclo[3.1.1]heptan-2-methyl]sulfamic acid chloride (5.31 g). ¹H-NMR (300 MHz, CDCl₃) δ 0.95 (d, J = 10, 1H), 1.04 (s, 3H), 1.23 (s, 3H), 1.43-1.60 (m, 1H), 1.82-2.09 (m, 5H), 2.25-2.46 (m, 2H), 3.25-3.40 (m, 2H), 5.66 (br s, 1H).
**Part C:** 3-(n-Pentyl)-4-phenyl-4,5-dihydropyrazole (3.4 gram, 15.7 mmol) was dissolved in toluene (25 ml) and treated with crude [(1R,2S,5R)-rel-6,6-dimethylbicyclo[3.1.1]heptan-2-methyl]sulfamic acid chloride (5.31 g, 15.7 mmol maximally) and triethylamine (2.2 ml, 15.7 mmol) and the resulting solution was magnetically stirred at room temperature for 96 hours. The solution was concentrated to give a crude oil (7.7 gram). Column chromatographic purification (heptane/ethylacetate = 1:1 (v/v), followed by another column chromatographic separation using as eluant heptane/ethylacetate = 6:1 (v/v) gave N-[(1 R,2S,5R)-rel-6,6-dimethylbicyclo [3.1.1]heptan-2-methyl]-3-(n-pentyl)-4-phenyl-4,5-dihydro-(1H)-pyrazole-1-sulfonamide (675 mg) as an oil. R_{f} = 0.3 (heptane/ethylacetate = 6:1 (v/v)). ¹H-NMR (400 MHz, CDCl₃) δ 0.83 (t, J = 7, 3H), 0.93 (d, J = 10, 1H), 1.01 (s, 3H), 1.20-1.29 (m, 7H), 1.41-1.60 (m, 3H), 1.85-2.43 (m, 9H), 3.22-3.28 (m, 2H), 3.64-3.71 (m, 1H), 4.02-4.09 (m, 1H), 4.12-4.19 (m, 1H), 4.66 (br t, J = 7, 1H), 7.19-7.23 (m, 2H), 7.28-7.38 (m, 3H).

### EXAMPLE 5 : FORMULATIONS USED IN ANIMAL STUDIES

**For oral *(p.o.)* administration** : to the desired quantity (0.5 -5 mg) of the solid compound 1 in a glass tube, some glass beads were added and the solid was milled by vortexing for 2 minutes. After addition of 1 ml of a solution of 1 % methylcellulose in water and 2% (v/v) of Poloxamer 188 (Lutrol F68), the compound was suspended by vortexing for 10 minutes. The pH was adjusted to 7 with a few drops of aqueous NaOH (0.1N). Remaining particles in the suspension were further suspended by using an ultrasonic bath.

**For intraperitoneal *(i.p.)* administration:** to the desired quantity (0.5-15 mg) of the solid compound 1 in a glass tube, some glass beads were added and the solid was milled by vortexing for 2 minutes. After addition of 1 ml of a solution of 1% methylcellulose and 5% mannitol in water, the compound was suspended by vortexing for 10 minutes. Finally the pH was adjusted to 7.

### EXAMPLE 6: PHARMACOLOGICAL METHODS

### In vitro affinity for cannabinoid-CB₁ receptors

The affinity of the co mpounds of the invention for cannabinoid CB₁ receptors can be determined using membrane preparations of Chinese hamster ovary (CHO) cells in which the human cannabinoid CB₁ receptor is stably transfected in conjunction with [³H]CP-55,940 as radioligand. After incubation of a freshly prepared cell membrane preparation with the [³H]-ligand, with or without addition of compounds of the invention, separation of bound and free ligand is performed by filtration over glassfiber filters. Radioactivity on the filter is measured by liquid scintillation counting.

### In vitro affinity for cannabinoid -CB₂ receptors

The affinity of the compounds of the invention for cannabinoid CB₂ receptors can be determined using membrane preparations of Chinese hamster ovary (CHO) cells in which the human cannabinoid CB₂ receptor is stably transfected in conjunction with [³H]CP-55,940 as radioligand. After incubation of a freshly prepared cell membrane preparation with the [³H]-ligand, with or without addition of compounds of the invention, separation of bound and free ligand is performed by filtration over glassfiber filters. Radioactivity on the filter is measured by liquid scintillation counting.

### In vitro cannabinoid-CB₁ receptor (ant)agonism

In vitro CB₁ receptor antagonism/agonism can be assessed with the human CB₁ receptor cloned in Chinese hamster ovary (CHO) cells. CHO cells are grown in a Dulbecco's Modified Eagle's medium (DMEM) culture medium, supplemented with 10% heat-inactivated fetal calf serum. Medium is aspirated and replaced by DMEM, without fetal calf serum, but containing [³H]-arachidonic acid and incubated overnight in a cell culture stove (5% CO₂/95% air; 37 ºC; water-saturated atmosphere). During this period [³H]-arachidonic acid is incorporated in membrane phospholipids. On the test day, medium is aspirated and cells are washed three times using 0.5 ml DMEM, containing 0.2% bovine serum albumin (BSA). CB₁ agonist stimulation leads to activation of PLAe followed by release of [³H]-arachidonic acid into the medium. This CB₁ agonist-induced release is concentration-dependently antagonized by CB₁ receptor antagonists, such as for example rimonabant.

### In vitro cannabinoid-CB₂ receptor (ant)agonism

Functional activity at the cannabinoid CB₂ receptor was assessed using a forskolin-stimulated cAMP accumulation assay. The ability of compounds to stimulate and inhibit adenylate cyclase activity was assessed in Chinese ovarian hamster (CHO) K₁ cells expressing human CB2 (Euroscreen,Brussel) receptor. CHO cells were grown in a CHO-S-SFM-II culture medium, supplemented with 10 % heat- inactivated foetal calf serum, 2mM glutamine, 400µg/ml Hygromycine B and 500 µg/ml G418 at 37 °C in 93 % air / 5 % CO₂. For incubation with test compounds, confluent cultures grown in 24 well plates were used. Each condition or substance was routinely tested in quadruplicate. Cells were loaded with 1 mCi [³H]-adenine in 0.5 ml medium per well. After 2 hours, cultures were washed with 0.5 ml PBS containing 1 mM IBMX and incubated for 20 minutes with 0.5 ml PBS containing 1mM IBMX and 3x10⁻⁷ M forskolin with or without the test compound. Antagonistic effects of test compounds were determined as inhibition of 0.1 µM JWH-133-decreased [³H]cAMP formation. After aspiration the reaction was stopped with 1ml trichloroacetic acid (5% w/v). The [³H]-ATP and [³H]-cAMP formed in the cellular extract were assayed as follows: a volume of 0.8 ml of the extract was passed over Dowex (50WX-4200- 400 mesh) and aluminum oxide columns, eluted with water and 0.1 M imidazole (pH=7.5). Eluates were mixed with 7 ml Ultima-Flo [AP] and the ß-radioactivity was counted with a liquid scintillation counter. The conversion of [³H]-ATP into [³H]-cAMP was expressed as the ratio in percentage radioactivity in the cAMP fraction as compared to the combined radioactivity in both cAMP and ATP fractions, and basal activity was subtracted to correct for spontaneous activity. Reference compounds used to assess cannabinoid CB₂ receptor mediated adenylate cyclase activity were the full cannabinoid CB₂ receptor agonists JWH-133 (Huffman, 1999^{b}) and WIN 55,212-2 (Huffman, 1999^{a}), and the inverse agonist or antagonist SR-144528 (Rinaldi-Carmona, 1998). Compounds were studied in a concentration range of 10⁻¹⁰ M to 10⁻⁶M. pEC₅₀ and the pA₂ were calculated according to Cheng-Prusoff equation (Cheng and Prusoff, 1973). Two independent experiments were performed in triplicate.

### EXAMPLE7: PHARMACOLOGICAL TESTRESULTS

Cannabinoid CB₁/CB₂ receptor affinity data, expressed as pK values (mean results of at least three independent experiments, performed according to the protocols given above) as well as CB₁ receptor agonist functional data of representative compounds of this inventionare shown in the table below.

**Table 1. CB₁ and CB₂ receptor affinities and CB₁ functional agonistic activity of representative compounds of this invention.**

| | **Human CB₁** | **Human CB₂** | **Human CB₁** |
|---|---|---|---|
| | | | |
| | **receptor binding** | | **Arachidonic acid release (CB₁)** |
| | | | |
| **Compound** | **pKi** | **pKᵢ** | **pEC₅₀** |
| | | | |
| 1 | 8.4 | 7.9 | 5.7 |
| 7 | 7.4 | 8.0 | 6.2 |
| 11 | 7.8 | 8.1 | 5.7 |
| 12 | 7.8 | 7.3 | |
| 13 | 8.1 | 8.1 | 9.3 |
| 15 | 7.0 | 7.8 | |
| 18 | 82 | 7.5 | |
| 39 | 5.4 | 6.9 | |
| 40 | 5.8 | 6.8 | |
| 41 | 6.5 | 6.7 | |
| 54 | 7.6 | 7.4 | 6.6 |
| 87 | 7.1 | 7.0 | 7.8 |
| 97 | 8.3 | 8.3 | > 9.0 |
| 109 | 82 | 7.6 | |
| 130 | 6.5 | 6.9 | |

These data illustrate the affinities of representative compounds for the CB₁ and CB₂ receptor as well as the CB₁ agonistic properties achieved by the structural modifications forming the basis of the present invention.

**Table 2. CB₂ functional agonistic/antagonistic activity of representative compounds of this invention.**

| | **Human CB₂** | **Human CB₂** |
|---|---|---|
| | | |
| | **CB₂-mediated adenylate cyclase activity** | **CB₂-mediated adenylate cyclase activity** |
| | | |
| **Compound** | **pEC₅₀** | **PA₂** |
| | | |
| 39 | 8.1 | |
| 40 | 8.1 | |
| 97 | | 9.2 |

These data illustrate the functional cannabinoid-CB₂ agonistic or antagonistic acivity of representative compounds of the present invention.

### EXAMPLE 8: PHARMACEUTICAL PREPARATIONS

For clinical use, compounds of formula (I) are formulated into a pharmaceutical compositions which are important and novel embodiments of the invention because of the presence of the compounds, more particularly specific compounds disclosed herein. Types of pharmaceutical compositions that may be used include but are not limited to tablets, chewable tablets, capsules (including microcapsules), solutions, parenteral solutions, ointments (creams and gels), suppositories, suspensions, and other types disclosed herein or apparent to a person skilled in the art from the specification and general knowledge in the art. The compositions are used for oral, intravenous, subcutaneous, tracheal, bronchial, intranasal, pulmonary, transdermal, buccal, rectal, parenteral or some other mode of administration. The pharmaceutical formulation contains at least one compound of formula (I) in admixture with a pharmaceutically acceptable adjuvant, diluent and/or carrier. The total amount of active ingredients suitably is in the range of from about 0.1% (w/w) to about 95% (w/w) of the formulation, suitably from 0.5% to 50% (w/w) and preferably from 1% to 25% (w/w).

The compounds of the invention can be brought into forms suitable for administration by means of usual processes using auxiliary substances such as liquid or solid, powdered ingredients, such as the pharmaceutically customary liquid or solid fillers and extenders, solvents, emulsifiers, lubricants, flavorings, colorings and/or buffer substances. Frequently used auxiliary substances which may be mentioned are magnesium carbonate, titanium dioxide, lactose, saccharose, sorbitol, mannitol and other sugars or sugar alcohols, talc, lactoprotein, gelatin, starch, amylopectin, cellulose and its derivatives, animal and vegetable oils such as fish liver oil, sunflower, groundnut or sesame oil, polyethylene glycol and solvents such as, for example, sterile water and mono - or polyhydric alcohols such as glycerol, as well as with disintegrating agents and lubricating agents such as magnesium stearate, calcium stearate, sodium stearyl fumarate and polyethylene glycol waxes. The mixture may then be processed into granules or pressed into tablets.

The active ingredients may be separately premixed with the other non-active ingredients, before being mixed to form a formulation. The active ingredients may also be mixed with each other, before being mixed with the non-active ingredients to form a formulation.

Soft gelatine capsules may be prepared with capsules containing a mixture of the active ingredients of the invention, vegetable oil, fat, or other suitable vehicle for soft gelatine capsules. Hard gelatine capsules may contain granules of the active ingredients. Hard gelatine capsules may also contain the active ingredients in combination with solid powdered ingredients such as lactose, saccharose, sorbitol, mannitol, potato starch, corn starch, amylopectin, cellulose derivatives or gelatine. Dosage units for rectal administration may be prepared (i) in the form of suppositories which contain the active substance mixed with a neutral fat base; (ii) in the form of a gelatine rectal capsule which contains the active substance in a mixture with a vegetable oil, paraffin oil or other suitable vehicle for gelatine rectal capsules; (iii) in the form of a ready-made micro enema; or (iv) in the form of a dry micro enema formulation to be reconstituted in a suitable solvent just prior to administration.

Liquid preparations may be prepared in the form of syrups, elixirs, concentrated drops or suspensions, e.g. solutions or suspensions containing the active ingredients and the remainder consisting, for example, of sugar or sugar alcohols and a mixture of ethanol, water, glycerol, propylene glycol and polyethylene glycol. If desired, such liquid preparations may contain coloring agents, flavoring agents, preservatives, saccharine and carboxymethyl cellulose or other thickening agents. Liquid preparations may also be prepared in the form of a dry powder to be reconstituted with a suitable solvent prior to use. Solutions for parenteral administration may be prepared as a solution of a formulation of the invention in a pharmaceutically acceptable solvent. These solutions may also contain stabilizing ingredients, preservatives and/or buffering ingredients. Solutions for parenteral administration may also be prepared as a dry preparation to be reconstituted with a suitable solvent before use.

Also provided according to the present invention are formulations and 'kits of parts' comprising one or more containers filled with one or more of the ingredients of a pharmaceutical composition of the invention, for use in medical therapy. Associated with such container(s) can be various written materials such as instructions for use, or a notice in the form prescribed by a governmental agency regulating the manufacture, use or sale of pharmaceuticals products, which notice reflects approval by the agency of manufacture, use, or sale for human or veterinary administration. The use of formulations of the present invention in the manufacture of medicaments for use in the treatment of a condition in which modulation of cannabinoid CB₁ receptors is required or desired, and methods of medical treatment or comprising the administration of a therapeutically effective total amount of at least one compound of formula (I), either as such or, in the case of prodrugs, after administration, to a patient suffering from, or susceptible to, a condition in which modulation of cannabinoid CB₁ receptors is required or desired.

### REFERENCES

Albericio, F., et al., Tetrahedron Lett., 38, 4853-4856, 1997.
Akaji, K. et al., Tetrahedron Lett, 35, 3315-3318, 1994.
Barluenga et al. Chem. Eur. J., 5, (3) 883-896, 1999
Bickel, M.H., "The pharmacology and Biochemistry of Noxides", Pharmaco-logical Reviews, 21(4), 325 - 355,1969.
Bodanszky, M. and A. Bodanszky: The Practice of Peptide Synthesis, Springer-Verlag, New York, ISBN: 0-387-57505-7, 1994.
Bundgaard, H. (editor), "Design of Prodrugs", Elsevier, 1985.
Cheng, Y. and Prusoff, W.H., Biochem. Pharmacol, 22, 3099-3108, 1973
De Petrocellis, L. et al,. Br. J. Pharmacol., 141, 765-774, 2004.
Di Marzo, V. et al., Nature Rev. Drug Discov., 3, 771-784, 2004.
Ettmayer, P. et al., "Lessons learned from marketed and investigational prodrugs", J.Med.Chem., 47, 2393-2404, 2004.
Hertzog, D.L. Expert Opin. Ther. Patents, 14, 1435-1452, 2004
Huffman et al., Curr. Med. Chem., 6, 705-720, 1999
Huffman et al., Bioorg. Med. Chem., 7, 2905-2914, 1999
Järvinen, T., "Design and Pharmaceutical applications of prodrugs", p. 733-796 in: S.C. Gad "Drug. Discovery Handbook ", John Wiley & Sons, New Jersey, U.S.A., 2005.
King, F.D., (editor), page 215 in: "Medicinal Chemistry: Principles and Practice", 1994.
Lambert, D.M. and Fowler, C.J. J. Med. Chem., 48, 5059-5087, 2005.
Lange, J.H.M. et al., J. Med. Chem., 47, 627-643, 2004.
Lange, J.H.M. et al., Bioorg. Med. Chem. Lett, 15, 4794-4798, 2005.
Lange, J.H.M. and Kruse, C.G., C. Curr. Opin. Drug Discovery Dev, 7, 498-506, 2004
Lange, J.H.M. and Kruse, C.G. Drug Discov. Today, 10, 693-702,2005;
Montalbetti, C.A.G.N. & V. Falque, Tetrahedron, 61, 10827-52. 2005.
Muccioli, G.G. et al., Curr. Med. Chem., 12, 1361-1394, 2005
Muccioli, G.G. and Lambert, D.M., Expert Opin. Ther. Patents, 16, 1405-1423, 2006
Ogata, J., et al., J. Med. Chem. 1987, 30, 1054-1068, 1987^{a}
Ogata, J., et al., J. Med. Chem. 1987, 30, 1497-1502, 1987^{b}
Padgett, L.W. Life Sc., 77, 1767-1798, 2005.
Raitio, K.H., et al., Curr. Med. Chem.,12, 1217-1237, 2005.
Rinaldi-Carmona et al., J. Pharmacol. Exp. Ther., 284, 644-650, 1998
Smith, R.A. and Fathi, Z. IDrugs, 8, 53-66, 2005.
Stella,J., "Prodrugs as therapeutics", Expert Opin. Ther. Patents, 14(3), 277-280, 2004.
Thakur, G.A. et al., MiniRev. Med. Chem., 5, 631-640, 2005.
Vandevoorde, S. and Lambert, D.M. Curr. Pharm. Des., 11, 2647-68, 2005
Van Sickle, M.D. et al., Science, 310, 329-332, 2005
Wang et al., Synth. Commun., 33 (9), 1449-1457, 2003.

## Claims

1. Compounds of the general formula (1) 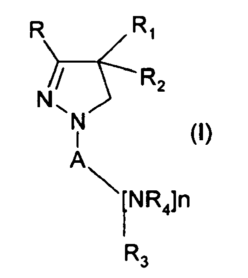 wherein
- R represents a C₂-₁₀ alkyl group, a C₄-₁₀ alkenyl group, a C₄₋₁₀ alkynyl group, a C₂₋₁₀-heteroalkyl group, a C₅₋₈-cycloalkyl-C₁₋₅-alkyl group or a C₅₋₈-heterocycloalkyl-C₁₋₅-alkyl group wherein the heteroatom(s) are either N, O or S, which C₂₋₁₀ alkyl group, C₄₋₁₀ alkenyl group, C₄₋₁₀ alkynyl group, C₂₋₁₀-heteroalkyl group, C₅₋₈-cycloalkyl-C₁₋₅-alkyl group or C₅₋₈-heterocycloalkyl-C₁₋₅-alkyl group may be substituted with 1-5 substituents selected from methyl, ethyl, hydroxy, amino or fluoro, or R represents an aryl-C₁₋₃-alkyl group or an aryl-C₁₋₃-heteroalkyl group in which the aryl groups may be substituted with 1-5 substituents Y, which can be the same or different, selected from the group C₁₋₃-alkyl or alkoxy, hydroxy, halogen, trifluoromethyl, trifluoromethylthio, trifluoromethoxy, nitro, amino, mono- or dialkyl (C₁₋₂)-amino, mono- or dialkyl (C₁₋₂)-amido, (C₁₋₃)-alkyl sulfonyl, dimethylsulfamido, C₁₋₃-alkoxycarbonyl, carboxyl, trifluoromethyl-sulfonyl, cyano, carbamoyl, sulfamoyl, phenyl and acetyl, or R represents a cyclopropyl group which cyclopropylgroup may be substituted with 1-5 substituents selected from methyl, ethyl, fluoro or with a C₃₋₅ linear or branched alkyl group or with a benzyl or aryl group, in which the aryl or benzyl group may be substituted with 1-5 substituents Y,
- R₁ represents hydrogen, hydroxy, C₁₋₃-alkoxy, acetyloxy or propionyloxy,
- R₂ represents an aryl group which may be substituted with 1-5 substituents Y, wherein Y has the abovementioned meaning,
- n is either 0 or 1
- R₃ represents a linear C₃₋₁₀ alkyl group, a branched C₅₋₁₀ alkyl group, a cyclopropyl, cyclobutyl, cyclopentyl, cycloheptyl or cyclooctyl group, C₅₋₁₀ bicycloalkyl group, C₆₋₁₀ tricycloalkyl group or C₈₋₁₁ tetracycloalkyl group which groups may be substituted with 1-5 substituents selected from methyl, ethyl, hydroxy, amino, fluoro or R₃ represents a C₃₋₈ cycloalkyl group which C₃₋₈ cycloalkyl group is substituted with an aryl group which aryl group may be substituted with 1-5 substituents Y wherein Y has the abovementioned meaning, or R₃ represents a 2,2,2-trifluoroethyl or 2-fluoroethyl group or R₃ represents a cyclohexyl group which group is substituted with 1-5 substituents selected from methyl, ethyl, hydroxy, amino or fluoro, or R₃ represents a C₅₋₈ heterocycloalkyl group, C₆₋₁₀ bicycloheteroalkyl group, C₇₋₁₀ tricycloheteroalkyl group, which groups may be substituted with 1-5 substituents selected from methyl, ethyl, hydroxy, amino or fluoro, or R₃ represents a C₃-₈ cycloalkyl-C₁-₃-alkyl group, C₅₋₁₀-bicycloalkyl-C₁₋₃-alkyl group, C₆₋₁₀-tricycloalkyl-C₁₋₃-alkyl group, which groups may be substituted with 1-5 substituents selected from methyl, ethyl, hydroxy, amino or fluoro, or R₃ represents a branched or linear C₃₋₈ heterocycloalkyl-C₁₋₃-alkyl group, C₅₋₁ₒ bicycloheteroalkyl-C₁₋₃-alkyl group, C₆₋₁₀ tricycloheteroalkyl-C₁₋₃-alkyl group, which groups may be substituted with 1-5 substituents selected from methyl, ethyl, hydroxy, amino or fluoro, or R₃ represents an aryl group, which group may be substituted with 1-5 substituents Y, wherein Y has the abovementioned meaning, or R₃ represents a aryl-C₁₋₅-alkyl group or a diaryl-C₁₋₅-alkyl group, in which groups the phenyl or heteroaromatic rings may be substituted with 1-5 substituents Y, wherein Y has the abovementioned meaning, or R₃ represents a linear or branched C₄₋₈ alkenyl or C₄₋₈ alkynyl group which linear or branched C₄₋₈ alkenyl or C₄₋₈ alkynyl group may be substituted with 1-3 fluoro atoms, or, only when n=1, R₃ may additionally represent a branched or linear C₂₋₁₀ heteroalkyl group, containing 1-2 heteroatoms selected from N,O or S,
- R₄ represents a hydrogen atom, a C₁₋₄ alkyl group or R₃ and R₄- together with the nitrogen atom to which they are bonded - form a saturated or unsaturated, nonaromatic or partly aromatic, monocyclic, bicyclic or tricyclic heterocyclic group having 5 to 11 ring atoms, which heterocyclic group may be substituted with 1-5 substituents selected from aryl, aryl-C₁₋₃-alkyl, diarylmethyl, or Y, wherein Y has the abovementioned meaning,
- A represents a carbonyl (C=O), thiocarbonyl (C=S) or sulfonyl (SO₂) group with the proviso that when A represents a thiocarbonyl (C=S),group, n has the value 1,
and stereoisomers and N-oxides thereof, and isotopically-labelled compounds of formula (I), as well as pharmacologically acceptable salts, hydrates, solvates, complexes and conjugates of said compounds of formula (I) and its stereoisomers, N-oxides, or isotopically-labelled analogs,
with the understanding that the term 'heteroalkyl', as used herein, includes alkyl groups with heteroatoms in any position, thus including N-bound, O-bound or S-bound alkyl groups; and with the understanding that the term 'aryl', as used herein, means monocyclic or fused bicyclic aromatic or heteroaromatic groups, which heteroaromatic groups contain one or two heteroatoms selected from the group (N, O, S).

2. Compounds as claimed in claim 1 of the general formula (I), wherein R₁ represents a hydrogen atom, and the other symbols have the meanings as given in claim 1.

3. Compounds as claimed in claim 2 of the general formula (I) wherein A represents a carbonyl group, and the other symbols have the meanings as given in claim 2.

4. Compounds as claimed in claim 3 of the general formula (I) wherein R₂ represents a phenyl, thienyl or pyridyl group, which phenyl, pyridyl or thienyl group may be substituted with 1, 2 or 3 substituents Y, and the other symbols have the meanings as given in claim 3.

5. Compounds as claimed in claim 4 of the general formula (I) wherein n is 1, and the other symbols have the same meanings as given in claim 4.

6. Compounds as claimed in claim 5 of the general formula (I) wherein R₄ represents a hydrogen atom, and the other symbols have the same meanings as given in claim 5.

7. Compounds as claimed in claim 6 of the general formula (I) wherein R represents a C₃₋₈ branched or linear alkyl group, which C₃₋₈ branched or linear alkyl group may be substituted with 1-3 fluoro atoms, and the other symbols have the same meanings as given in claim 6.

8. The compound according to claim 1 which is:
N-[(1R,2S,5R)-rel-6,6-dimethylbicyclo[3.1.1]heptan-2-methyl]-3-(n-pentyl-4-phenyl-4,5-dihydro-(1H)-pyrazole-1-carboxamide
N-(1-Adamantyl)-3-(n-pentyl)-4-phenyl-4,5-dihydro-(1H)-pyrazole-1-carboxamide
N-(Exo-bicydo[2.2.1]hept-2-yl)-3-(n-pentyl)4-phenyl-4,5-dihydro-(1H)-pyrazole-1-carboxamide
N-Phenyl-3-(n-pentyl)-4-phenyl-4,5-dihydro-(1H)-pyrazole-1-carboxamide
N-[(1R,2S,5R)-rel-6,6-dimethylbicyclo[3.1.1]heptan-2-methyl]-3-(benzyl)-4-phenyl-4,5-dihydro-(1H)-pyrazole-1-carboxamide
N-(1-Adamantyl)-3-(benzyl)-4-phenyl-4,5-dihydro-(1H)-pyrazole-1-carboxamide
N-[(1R,2S,5R)-rel-6,6-dimethylbicyclo[3.1.1]heptan-2-methyl]-3-(n-butyl)-4-phenyl-4,5-dihydro-(1H)-pyrazole-1-carboxamide
N-[(1R,2S,5R)-rel-6,6-dimethylbicyclo[3.1.1]heptan-2-methyl]-3-[3-(1-piperidinyl)propyl]-4-phenyl-4,5-dihydro-(1H)-pyrazole-1-carboxamide
N-[(1R,2S,5R)-rel-6,6-dimethylbicyclo[3.1.1]heptan-2-methyl]-3-(n-propyl)-4-phenyl-4,5-dihydro-(1H)-pyrazole-1-carboxamide
N-(Benzyl)-3-(n-pentyl)-4-phenyl-4,5-dihydro-(1H)-pyrazole-1-carboxamide
N-(1-Adamantyl)methyl-3-(n-pentyl)-4-phenyl-4,5-dihydro-(1H)-pyrazole-1-carboxamide
N-(Cyclohexylmethyl)-3-(n-pentyl)-4-phenyl-4,5-dihydro-(1H)-pyrazole-1-carboxamide
N-[Endo-(1R,2S,4R)-1,7,7-trimethylbicyclo[2.2.1]hept-2-yl]-3-(n-pentyl)-4-phenyl-4,5-dihydro-(1H)-pyrazole-1-carboxamide
N-[endo-(1S)-1,3,3-trimethylbicycto[2.2.1]hept-2-yl]-3-(n-pentyl)-4-phenyl-4,5-dihydro-(1H)-pyrazole-1-carboxamide
N-[(11R,2S,5R)-rel-6,6-dimethylbicydo[3.1.1]heptan-2-methyl]-3-(n-propyl)4-(2-pyridyl)-4,5-dihydro-(1H)-pyrazole-1-carboxamide
N-(1-Phenyl-ethyl)-3-(n-pentyl)-4-phenyl-4,5-dihydro-(1H)-pyrazole-1-carboxamide
N-(2-Adamantyl)-3-(n-pentyl)-4-phenyl-4,5-dihydro-(1H)-pyrazole-1-carboxamide
N-(1-Naphtyl)-3-(n-pentyl)-4-phenyl-4,5-dihydro-(1H)-pyrazole-1-carboxamide
N-(1-Methyl-1-phenyl-ethyl)-3-(n-pentyl)-4-phenyl-4,5-dihydro-(1H)-pyrazole-1-carboxamide
N-(2,2-Diphenylpropyl)-3-(n-pentyl)4-phenyl-4,5-dihydro-(1H)-pyrazole-1-carboxamide
N-((3-Trifluoromethyl)benzyl)-3-(n-pentyl)-4-phenyl-4,5-dihydro-(1H)-pyrazole-1-carboxamide
N-(2,2-Dimethylpropyl)-3-(n-pentyl)-4-phenyl-4,5-dihydro-(1H)-pyrazole-1-carboxamide
N-(Naphthalen-1-yl-methyl)-3-(n-pentyl)-4-phenyl-4,5-dihydro-(1H)-pyrazole-1-carboxamide
N-[(3-Dimethylamino)-2,2-dimethylpropyl]-3-(n-pentyl)-4-phenyl-4,5-dihydro-(1H)-pyrazole-1-carboxamide
N-[Endo-(1R,2S,4R)-1,7,7-trimethylbicyclo[2.2.1]hept-2-yl]-3-(n-butyl)-4-phenyl-4,5-dihydro-(1H)-pyrazole-1-carboxamide
N-(2-(4-fluorophenyl)-1,1-dimethyl-ethyl)-3-(n-butyl)-4-phenyl-4,5-dihydro-(1H)-pyrazole-1-carboxamide
N-[Endo-(1R,2S,4R)-1,7,7-trimethylbicyclo[2.2.1]hept-2-yl]-3-(4,4,4-trifluoro-n-butyl)-4-phenyl-4,5-dihydro-(1H)-pyrazole-1-carboxamide
N-(2-(4-fluorophenyl)-1,1-dimethyl-ethyl)-3-(4,4,4-trifluoro-n-butyl)-4-phenyl-4,5-dihydro-(1H)-pyrazole-1-carboxamide
N-(2-(4-Fluorophenyl)1,1-dimethyl-ethyl)-3-(n-pentyl)-4-phenyl-4,5-dihydro-(1H)-pyrazole-1-carboxamide
N-[Endo-(1R,2S,4R)-1,7,7-Trimethylbicyclo[2.2.1]hept-2-yl]-3-(1,1-dimethyl-n-butyl)-4-phenyl-4,5-dihydro-(1H)-pyrazole-1-carboxamide
N-[Endo-(1R,2S,4R)-1,7,7-Trimethylbicyclo[2.2.1]hept-2-yl]-3-(3,3,3-trifluoropropyl)-4-phenyl-4,5-dihydro-(1H)-pyrazole-1-carboxamide
N-[Endo-(1R,2S,4R)-1,7,7-Trimethylbicyclo[2.2.1]hept-2-yl]-3-(1,1-dimethylpropyl)-4-phenyl-4,5-dihydro-(1H)-pyrazole-1-carboxamide
N-(2-(4-Fluorophenyl)-1,1-dimethyl-ethyl)3-(1,1-dimethylpropyl)-4-phenyl-4,5-dihydro-(1H)-pyrazole-1-carboxamide
N-(Endo-(R,2S,4R)-1,7,7-trimethylbicyclo[2.2.1]hept-2-yl]-3-(1,1-dimethyl-3,3,3-trifluoropropyl)-4-phenyl-4,5-dihydro-(1H)-pyrazole-1-carboxamide
N-[endo-(1R)-1,3,3-trimethylbicyclo[2.2.1]hept-2-yl]-3-(n-butyl)-4-phenyl-4,5-dihydro-(1H)-pyrazole-1-carboxamide
N-(1-Methyl-1-phenyl-ethyl)-3-(n-butyl)-4-phenyl-4,5-dihydro-(1H)-pyrazole-1-carboxamide
N-(2-Adamantyl)-3-(n-butyl)-4-phenyl-4,5-dihydro-(1H)-pyrazole-1-carboxamide
N-[Exo-(1R,2R,4R)-1,7,7-trimethylbicyclo[2.2.1]hept-2-yl]-3-(n-butyl)-4-phenyl-4,5-dihydro-(1H)-pyrazole-1-carboxamide
N-(2-phenyl-1,1-dimethyl-ethyl)-3-(n-butyl)-4-(3-fluorophenyl)-4,5-dihydro-(1H)-pyrazole-1-carboxamide
N-(2-phenyl-1,1-dimethyl-ethyl)-3-(n-butyl)-4-(2-fluorophenyl)-4,5-dihydro-(1H)-pyrazole-1-carboxamide
N-Phenyl-3-(4-chlorobenzyl)-4-(4-chlorophenyl)-4,5-dihydro-(1H)-pyrazole-1-carboxamide
N-(4-Methoxyphenyl)-3-(4-chlorobenzyl)-4-(4-chlorophenyl)-4,5-dihydro-(1H)-pyrazole-1-carboxamide
N-[Endo-(1R,2S,4R)-1,7,7-trimethylbicyclo[2.2.1]hept-2-yl]-3-(n-butyl)-4-(2methoxyphenyl)-4,5-dihydro-(1H)-pyrazole-1-carboxamide
N-(1-Methyl-1-phenyl-ethyl)-3-(n-butyl)-4-(2-methoxyphenyl)-4,5-dihydro-(1H)-pyrazole-1-carboxamide
N-[Endo-(1R,2S,4R)-1,7,7-trimethylbicyclo[2.2.1]hept-2-yl]-3-(n-butyl)-4-(2-fluorophenyl)-4,5-dihydro-(1H)-pyrazole-1-carboxamide
N-[Endo-(1R,2S,4R)-1,7,7-trimethylbicyclo[2.2.1]hept-2-yl]-3-(n-butyl)-4-(pyrid-3-yl)-4,5-dihydro-(1H)-pyrazole-1-carboxamide
N-[(1R,2R,3R,5S)-2,7,7-trimethylbicyclo[3.1.1]hept-3-yl]-3-(n-butyl)-4-(3-fluorophenyl)-4,5-dihydro-(1H)-pyrazole-1-carboxamide
N-[endo-(1R)-1,3,3-trimethylbicyclo[2.2.1]hept-2-yl]-3-(n-butyl)-4-(2-fluorophenyl)-4,5-dihydro-(1H)-pyrazole-1-carboxamide
N-[2-(trifluoromethyl)benzyl]-3-(n-butyl)4-(3-fluorophenyl)-4,5-dihydro-(1H)-pyrazole-1-carboxamide
N-[Exo-(1R,2R,4R)-1,7, 7-trimethylbicyclo[2.2.1]hept-2-yl]-3-(n-butyl)-4-(2-fluorophenyl)-4,5-dihydro-(1H)-pyrazole-1-carboxamide
N-(1-Methyl-1-phenyl-ethyl)-3-(n-butyl)-4-(3-fluorophenyl)-4,5-dihydro-(1H)-pyrazole-1-carboxamide
N-(1-Methyl-1-phenyl-ethyl)-3-(n-butyl)-4-(4-chlorophenyl)-4,5-dihydro-(1H)-pyrazole-1-carboxamide
N-[2-(trifluoromethyl)benzyl]-3-(n-butyl)-4-(4-chlorophenyl)-4,5-dihydro-(1H)-pyrazole-1-carboxamide
N-[Endo-(1R,2S,4R)-1,7,7-trimethylbicyclo[2.2.1]hept-2-yl]-3-(cyclopropylmethyl)-4-phenyl-4,5-dihydro-(1H)-pyrazole-1-carboxamide
N-[Endo-(1R,2S,4R)-1,7,7-trimethylbicyclo[2.2.1]hept-2-yl]-3-(n-butyl)-4-(4-fluorophenyl)-4,5-dihydro-(1H)-pyrazole-1-carboxamide
N-(1-Methyl-1-phenyl-ethyl)-3-(n-butyl)-4-(4-fluorophenyl)-4,5-dihydro-(1H)-pyrazole-1-carboxamide
N-(Adamant-2-yl)-3-(n-butyl)-4-(4-fluorophenyl)-4,5-dihydro-(1H)-pyrazole-1-carboxamide
N-(1-Methyl-1-(4-fluorophenyl)-ethyl)-3-(n-butyl)-4-(4-fluorophenyl)-4,5-dihydro-(1H)-pyrazole-1-carboxamide
N-(1-Methyl-1-(4-fluorophenyl)-ethyl)-3-(n-butyl)-4-phenyl-4,5-dihydro-(1H)-pyrazole-1-carboxamide
N-(1-Methyl-1-phenyl-ethyl)-3-(n-pentyl)-4-(2-fluorophenyl)-4,5-dihydro-(1H)-pyrazole-1-carboxamide
N-[Endo-(1R,2S,4R)-1,7,7-trimethylbicyclo[2.2.1]hept-2-yl]-3-(n-pentyl)-4-(2-fluorophenyl)-4,5-dihydro-(1H)-pyrazole-1-carboxamide
N-(1-Methyl-1-(4-fluorophenyl)-ethyl)-3-(n-pentyl)-4-(2-fluorophenyl)-4,5-dihydro-(1H)-pyrazole-1-carboxamide
N-(1-Methyl-1-(4-fluorophenyl)-ethyl)-3-(n-pentyl)-4-(3-fluorophenyl)-4,5-dihydro-(1H)-pyrazole-1-carboxamide
N-(adamant-2-yl)-3-(n-pentyl)-4-(2-fluorophenyl)-4,5-dihydro-(1H)-pyrazole-1-carboxamide
N-(adamant-2-yl)-3-(n-pentyl)-4-(3-fluorophenyl)-4,5-dihydro-(1H)-pyrazole-1-carboxamide
N-(1-Methyl-1-phenyl-ethyl)-3-(n-butyl)-4-(benzo[b]thiophen-3-yl)-4,5-dihydro-(1H)-pyrazole-1-carboxamide
N-[Endo-(1R,2S,4R)-1,7,7-trimethylbicyclo[2.2.1]hept-2-yl]-3-(n-butyl)-4-(benzo[b]thiophen-3-yl)-4,5-dihydro-(1H)-pyrazole-1-carboxamide
N-(1-Methyl-1-phenyl-ethyl)-3-(n-butyl)-4-(thiophen-3-yl)-4,5-dihydro-(1H)-pyrazole-1-carboxamide
N-(1-Methyl-1-phenyl-ethyl)-3-(but-3-ynyl)-4-(2-fluorophenyl)-4,5-dihydro-(1H)-pyrazole-1-carboxamide
N-[Endo-(1R,2S,4R)-1,7,7-trimethylbicyclo[2.2.1]hept-2-yl]-3-(but-3-ynyl)-4-(2-fluorophenyl)-4,5-dihydro-(1H)-pyrazole-1-carboxamide
N-[Endo-(1R,2S,4R)-1,7,7-trimethylbicyclo[2.2.1]hept-2-yl]-3-(1-phenylcyclopropyl)-4-phenyl-4,5-dihydro-(1H)-pyrazole-1-carboxamide
N-(1-Methyl-1-phenyl-ethyl)-3-(1-phenylcyclopropyl)-4-phenyl-4,5-dihydro-(1H)-pyrazole-1-carboxamide
N-[Endo-(1R,2S,4R)-1,7,7-trimethylbicyclo[2.2.1]hept-2-yl]-3-(2,2,3,3-tetramethylcyclopropyl)-4-phenyl-4,5-dihydro-(1H)-pyrazole-1-carboxamide
N-(1-Methyl-1-phenyl-ethyl)-3-(2,2,3,3-tetramethylcydopropyl)-4-phenyl-4,5-dihydro-(1H)-pyrazole-1-carboxamide
N-[(1R,2R,3R,5S)-2,7,7-trimethylbicyclo[3.1.1]hept-3-yl]-3-(n-butyl)4-(4-chlorophenyl)-4,5-dihydro-(1H)-pyrazole-1-carboxamide
N-(1-Methyl-1-phenyl-ethyl)-3-(n-pentyl)-4-(3-fluorophenyl)-4,5-dihydro-(1H)-pyrazole-1-carboxamide
N-[Endo-(1R,2S,4R)-1,7,7-trimethylbicyclo[2.2.1]hept-2-yl]-3-(n-pentyl)-4-(3-fluorophenyl)-4,5-dihydro-(1H)-pyrazole-1-carboxamide
N-(1-Methyl-1-phenyl-ethyl)-3-(n-pentyl)-4-(4-fluorophenyl)-4,5-dihydro-(1H)-pyrazole-1-carboxamide
N-[Endo-(1R,2S,4R)1,7,7-trimethylbicyclo[2.2.1]hept-2-yl]-3-(n-pentyl)4-(4-fluorophenyl)4,5-dihydro-(1H)-pyrazole-1-carboxamide
N-[(1S,2S,35,5R)-2,7,7-trimethylbicylo[3.1.1]hept-3-yl]-3-(n-butyl)-4-(3-fluorophenyl)-4,5-dihydro-(1H)-pyrazole-1-carboxamide
N-(1-Methyl-1-(4-fluorophenyl)-ethyl)-3-(n-butyl)-4-(3-fluorophenyl)-4,5-dihydro-(1H)-pyrazole-1-carboxamide
N-(1-Methyl-1-(4-fluorophenyl)-ethyl)-3-(n-butyl)-4-(2-fluorophenyl)-4,5-dihydro-(1H)-pyrazole-1-carboxamide
N-[(1S,2S,3S,5R)-2,7,7-trimethylbicyclo[3.1.1]hept-3-yl]-3-(n-butyl)-4-(2-fluorophenyl)-4,5-dihydro-(1H)-pyrazole-1-carboxamide
N-[Endo-(1R,2S,4R)-1,7,7-trimethylbicyclo[2.2.1]hept-2-yl]-3-(n-butyl)-4-(thien-3-yl)-4,5-dihydro-(1H)-pyrazole-1-carboxamide
N-[Endo-(1R,2S,4R)-1,7,7-trimethylbicyclo[2.2.1]hept-2-yl]-3-(3,3,3-trifluoro-1-methoxymethyl-propyl)-4-phenyl-4,5-dihydro-(1H)-pyrazole-1-carboxamide
N-[Endo-(1R,2S,4R)-1,7,7-trimethylbicyclo[2.2.1]hept-2-yl]-3-(n-butyl)-4-hydroxy-4-phenyl-4,5-dihydro-(1H)-pyrazole-1-carboxamide
1-(1-Naphtoyl)-3-(n-pentyl)-4-phenyl-4,5-dihydro-(1H)-pyrazole
[3-(n-pentyl)-4-phenyl-4,5-dihydro-(1H)-pyrazol-1-yl]-[1-(4-chlorophenyl)cyclopentyl] methanone
[3-(n-pentyl)-4-phenyl-4,5-dihydro-(1H)-pyrazol-1-yl]-(napht-2-yl) methanone
(3-(n-pentyl)-4-phenyl-4,5-dihydro-(1H)-pyrazol-1-yl]-(diphenylmethyl) methanone [3-(n-pentyl)-4-phenyl-4,5-dihydro-(1H)-pyrazol-1-yl]-(3-chlorobenzothien-2-yl] methanone
[3-(n-pentylr4-phenyl-4,5-dihydro-(1H)-pyrazol-1-ylJ-(benzofuran-2-yl] methanone
[3-(n-pentyl)-4-phenyl-4,5-dihydro-(1H)-pyrazol-1-yl]-[2,4,4-(trimethyl)pentyl] methanone
[3-(n-pentyl)-4-phenyl-4,5-dihydro-(1H)pyrazol-1-yl]-[3-(trifluoromethyl)phenyl] methanone
(Cis-3,4,5-trimethylpiperazin-1-yl)[3-(n-pentyl)-4-phenyl-4,5-dihydro-(1H)-pyrazol-1-yl] methanone
N-Endo-[(1R,2S,4R)-1,7,7-trimethylbicyclo[2.2.1]hept-2-yl]-3-(n-pentyl)-4-phenyl-4,5-dihydro-(1H)-pyrazole-1-carboxamide (diastereomer A)
N-Endo-[(1R,2S,4R)-1,7,7-trimethylbicyclo[2.2.1]hept-2-yl]-3-(n-pentyl)-4-phenyl-4,5-dihydro-(1H)-pyrazole-1-carboxamide (diastereomer B)
N-Endo-[(1R,2S,4R)-1,7,7-trimethylbicyclo[2.2.1]hept-2-yl]-3-(n-butyl)-4-(3-fluorophenyl)-4,5-dihydro-(1H)-pyrazole-1-carboxamide (diastereomer A)
N-Endo-((1R,2S,4R)-1,7,7-trimethylbicyclo[2.2.1] hept-2-yl]-3-(n-butyl)-4-(3-fluorophenyl)-4,5-dihydro-(1H)-pyrazole-1-carboxamide (diastereomer B)
N-Endo-[(1R,2S,4R)-1,7,7-trimethylbicyclo[2.2.1]hept-2-yl]-3-(n-butyl)-4-(4-chlorophenyl)-4,5-dihydro-(1H)-pyrazole-1-carboxamide (diastereomer A)
N-Endo-[(1R,2S,4R)-1,7,7-trimethylbicyclo[2.2.1]hept-2-yl]-3-(n-butyl)-4-(4-chlorophenyl)-4,5-dihydro-(1H)-pyrazole-1-carboxamide (diastereomer B)
N-(1,2,2,6,6-pentamethylpiperidin-4-yl)-3-(n-butyl)-4-(2-fluorophenyl)-4,5-dihydro-(1H)-pyrazole-1-carboxamide
N-(4-methoxyphenyl)-3-(n-pentyl)-4-phenyl-4,5-dihydro-(1H)-pyrazole-1-carboxamide
N-(4-methoxyphenyl)-3-(n-pentyl)-4-phenyl-4,5-dihydro-(1H)-pyrazole-1-carboxamide
N-(phenethyl)-3-(n-pentyl)-4-phenyl-4,5-dihydro-(1H)-pyrazole-1-carboxamide
N-(2-phenyl-trans-cyclopropyl)-3-(n-pentyl)-4-phenyl-4,5-dihydro-(1H)-pyrazole-1-carboxamide
N-(1-naphthalen-1-yl-ethyl)-3-(n-pentyl)-4-phenyl-4,5-dihydro-(H)-pyrazole-1-carboxamide
N-[2-(trifluoromethyl)phenyl)-3-(n-pentyl)-4-phenyl-4,5-dihydro-(1H)-pyrazole-1-carboxamide
N-cycloheptyl-3-(n-pentyl)-4-phenyl-4,5-dihydro-(1H)pyrazole-1-carboxamide
N-cyclooctyl-3-(n-pentyl)-4-phenyl-4,5-dihydro-(1H)-pyrazole-1-carboxamide
N-(1,2,3,4-tetrahydronaphthalen-1-yl)-3-(n-pentyl)-4-phenyl-4,5-dihydro-(1H)-pyrazole-1-carboxamide
N-[2,2-(diphenyl)ethyl]-3-(n-pentyl)-4-phenyl-4,5-dihydro-(1H)-pyrazole-1-carboxamide
(3-Pentyl-4-phenyl-4,5-dihydropyrazol-1-yl)-[4-(2-pyrimidinyl)piperazin-1-yl]methanone
N-[2-(4-fluorophenyl)ethyl]-3-(n-pentyl)-4-phenyl-4,5-dihydro-(1H)-pyrazole-1-carboxamide
(3-Pentyl-4-phenyl-4,5-dihydropyrazol-1-yl)-[azepan-1-yl]methanone
N-(quinolin-3-yl)-3-(n-pentyl)-4-phenyl-4,5-dihydro-(1H)-pyrazole-1-carboxamide
N-[1-(ethyl)propyl]-3-(n-pentyl]-4-phenyl-4,5-dihydro-(1H)-pyrazole-1-carboxamide
N-(2,2,2-trifluoroethyl)-3-(n-pentyl)-4-phenyl-4,5-dihydro-(1H)-pyrazole-1-carboxamide
N-(pyridin-3-ylmethyl)-3-(n-pentyl)-4-phenyl-4,5-dihydro-(1Hrpyrazole-1-carboxamide
N-(2-indanyl)-3-(n-pentyl)-4-phenyl-4,5-dihydro-(1H)-pyrazole-1-carboxamide
(3-Pentyl-4-phenyl-4,5-dihydropyrazol-1-yl)-(1,2,3,4-tetrahydroisoquinolin-2-yl)methanone
N-(Methyl), N-(naphthalen-1-ylmethyl)-3-(n-pentyl)-4-phenyl-4,5-dihydro-(1H)-pyrazole-1-carboxamide
N-(3,3-Diphenypropyl)-3-(n-pentyl-4-phenyl-4,5-dihydro-(1H)-pyrazole-1-carboxamide
N-(napht-1-yl)-3-(n-pentyl)-4-phenyl-4,5-dihydro-(1H)-pyrazolecarbothiamide
N-[1-(ethyl)propyl]-3-(n-pentyl]-4-phenyl-4,5-dihydro-(1H)-pyrazolecarbothiamide
N-[pyridin-3-ylmethyl]-3-(n-pentyl)-4-phenyl-4,5-dihydro-(1H)-pyrazolecarbothiamide
N-[exo-bicyclo[2.2.1]hept-2-yl]-3-(n-pentyl)-4-phenyl-4,5-dihydro-(1H)-pyrazolecarbothiamide
1-(Naphthalen-1-ylsulfonyl)-3-(n-butyl)-4-(2-fluorophenyl)-4,5-dihydro-(1H)-pyrazole
1-(Naphthalen-2-ylsulfonyl)-3-(n-butyl)-4-(2-fluorophenyl)-4,5-dihydro-(1H)-pyrazole
N-[(1R,2S,5R)-rel-6,6-dimethylbicyclo[3.1.1]heptan-2-methyl]-3-(n-pentyl)-4-phenyl-4,5-dihydro-(1H)-pyrazole-1-sulfonamide

9. Compounds of the general formula (IV). 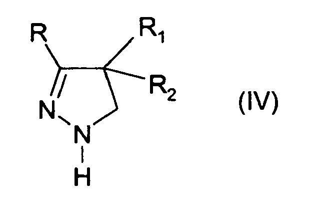 wherein R and R₁ have the same meanings as given in claim 1 and R₂ represents an phenyl group which may be substituted with 1-5 substituents Y2 which can be the same or different, selected from the group C₁₋₃-alkoxy, hydroxy, trifluoromethyl, trifluoromethylthio, trifluoromethoxy, nitro, amino, mono- or dialkyl (C₁₋₂)-amino, mono- or dialkyl (C₁₋₂)-amido, (C₁₋₃)-alkyl sulfonyl, dimethylsulfamido, C₁₋₃-alkoxycarbonyl, carboxyl, trifluoromethyl-sulfonyl, cyano, carbamoyl, sulfamoyl, ortho-halogen, meta-halogen, ortho-C₁₋₃-alkyl, meta-C₁₋₃-alkyl and acetyl, or R₂ represents a thienyl or pyridyl group, which groups may be substituted with one or two substituents Y, which Y group has the meaning as given in claim 1, such compounds being useful in the synthesis of compounds of the general formula (1).

10. Compounds of the general formula (VIII) 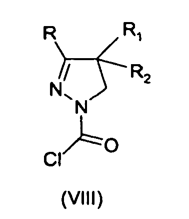 wherein R and R₂ have the same meanings as given in claim 1 and R₁ represents hydrogen, such compounds being useful in the synthesis of compounds of the general formula (I) wherein n = 1.

11. A compound as claimed in any of the claims 1-8, or a pharmacologically acceptable salt, hydrate, solvate or complex thereof, for use as a medicament.

12. A medicament, **characterized in that** it contains a compound according to one of the claims 1-8, or a pharmacologically acceptable salt, hydrate, solvate or complex thereof.

13. A pharmaceutical composition comprising, in addition to a pharmaceutically acceptable carrier and/or at least one pharmaceutically acceptable auxiliary substance, a pharmacologically active amount of at least one compound of one of the claims 1-8, or a pharmacologically acceptable salt, hydrate, solvate or complex thereof, as an active ingredient.

14. A pharmaceutical composition as claimed in claim 13, for the prevention or the treatment of multiple sclerosis, traumatic brain injury, pain, appetite disorders, epilepsy, Alzheimer's disease, Tourette's syndrome, cerebral ischaemia or gastrointestinal disorders.

15. The pharmaceutical composition according to claim 13, further comprising: at least one additional therapeutic agent.

16. A method of preparing pharmaceutical compositions as claimed in claim 13, **characterized in that** a compound of one of the claims 1-8 is brought into a form suitable for administration.

17. A pharmaceutical composition made by mixing a compound of claim 1 and a pharmaceutically acceptable carrier and/or at least one pharmaceutically acceptable auxiliary substance.

18. Use of a compound as claimed in claims 1-8 for the preparation of a pharmaceutical composition for the treatment of multiple sclerosis, traumatic brain injury, pain, appetite disorders, epilepsy, Alzheimer's disease, Tourette's syndrome, cerebral ischaemia and gastrointestinal disorders.

## Patentansprüche

1. Verbindung der allgemeinen Formel (I) 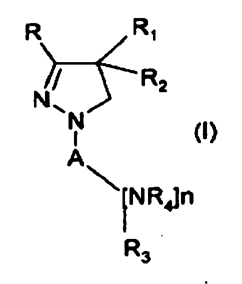 wobei
- R für eine C₂₋₁₀-Alkylgruppe, eine C₄₋₁₀-Alkenylgruppe, eine C₄₋₁₀-Alkynylgruppe, eine C₂₋₁₀-Heteroalkylgruppe, eine C₅₋₈-Cycloalkyl-C₁₋₅-Alkylgruppe oder eine C₅₋₈-Heterocycloalkyl-C₁₋₅-Alkylgruppe steht, wobei das/die Heteroatom(e) entweder N, O oder S ist/sind, wobei die C₂₋₁₀-Alkylgruppe, C₄₋₁₀-Alkenylgruppe, C₄₋₁₀-Alkynylgruppe, C₂₋₁₀-Heteroalkylgruppe, C₅₋₈-Cycloalkyl-C₁₋₅-Alkylgruppe oder C₅₋₈-Heterocycloalkyl-C₁₋₅-Alkylgrupp mit 1-5 Substituenten, ausgewählt aus Methyl, Ethyl, Hydroxy, Amino oder Fluor, substituiert sein kann oder R für eine Aryl-C₁₋₃-Alkyl-Gruppe oder eine Aryl-C₁₋₃-Heteroalkylgruppe steht, bei denen die Arylgruppen mit 1-5 Substituenten Y substituiert sein können, die gleich oder verschieden sein können, ausgewählt aus der Gruppe C₁₋₃-Alkyl oder Alkoxy, Hydroxy, Halogen, Trifluormethyl, Trifluormethylthio, Trifluormethoxy, Nitro, Amino, Mono- oder Dialkyl-(C₁₋₂)-Amino, Mono- oder Dialkyl-(C₁₋₂)-Amido, (C₁₋₃)-Alkylsulfonyl, Dimethylsulfamido, C₁₋₃-Alkoxycarbonyl, Carboxyl, Trifluormethylsulfonyl, Cyano, Carbamoyl, Sulfamoyl, Phenyl und Acetyl, oder R für eine Cyclopropylgruppe steht, wobei die Cyclopropylgruppe mit 1-5 Substituenten, ausgewählt aus Methyl, Ethyl, Fluor, substituiert sein kann oder mit einer linearen oder verzweigten C₃₋₅-Alkylgruppe oder mit einer Benzyl- oder Arylgruppe, wobei die Aryl- oder Benzylgruppe mit 1-5 Substituenten Y substituiert sein kann,
- R₁ für Wasserstoff, Hydroxy, C₁₋₃-Alkoxy, Acetyloxy oder Propionyloxy steht,
- R₂ für eine Arylgruppe steht, die mit 1-5 Substituenten Y substituiert sein kann, wobei Y die oben erwähnte Bedeutung hat,
- n entweder 0 oder 1 ist,
- R₃ für eine lineare C₃₋₁₀-Aklylgruppe, eine verzweigte C₅₋₁₀-Alkylgruppe, eine Cyclopropyl-, Cyclobutyl-, Cyclopentyl-, Cycloheptyl- oder Cyclooctylgruppe, eine C₅₋₁₀-Bicycloalkylgruppe, eine C₆₋₁₀-Tricycloalkylgruppe oder eine C₈₋₁₁-Tetracycloalkylgruppe steht, wobei die Gruppen mit 1-5 Substituenten, ausgewählt aus Methyl, Ethyl, Hydroxy, Amino, Fluor, substituiert sein können oder R₃ für eine C₃₋₈-Cycloalkylgruppe steht, wobei die C₃₋₈-Cycloalkylgruppe mit einer Arylgruppe substituiert ist und die Arylgruppe mit 1-5 Substituenten Y substituiert sein kann, wobei Y die oben erwähnte Bedeutung hat, oder R₃ für eine 2,2,2-Trifluorethyl- oder 2-Fluorethylgruppe steht oder R₃ für eine Cyclohexylgruppe steht, wobei die Gruppe mit 1-5 Substituenten, ausgewählt aus Methyl, Ethyl, Hydroxy, Amino oder Fluor, substituiert ist oder R₃ für eine C₅₋₈-Heterocycloalkylgruppe, eine C₆₋₁₀-Bicycloheteroalkylgruppe, eine C₇₋₁₀-Tricycloheteroalkylgruppe steht, wobei die Gruppen mit 1-5 Substituenten, ausgewählt aus Methyl, Ethyl, Hydroxy, Amino oder Fluor, substituiert sein können oder R₃ für eine C₃₋₈-Cyloalkyl-C₁₋₃-Alkylgruppe, eine C₅₋₁₀-Bicycloalkyl-C₁₋₃-Alkylgruppe, eine C₆₋₁₀-Tricycloalkyl-C₁₋₃-Alkylgruppe steht, wobei die Gruppen mit 1-5 Substituenten, ausgewählt aus Methyl, Ethyl, Hydroxy, Amino oder Fluor, substituiert sein können oder R₃ für eine verzweigte oder lineare C₃₋₈-Heterocycloalkyl-C₁₋₃-Alkylgruppe, eine C₅₋₁₀-Bicycloheteroalkyl-C₁₋₃-Alkylgruppe, eine C₆₋₁₀-Tricycloheteroalkyl-C₁₋₃Alkylgruppe steht, wobei die Gruppen mit 1-5 Substituenten, ausgewählt aus Methyl, Ethyl, Hydroxy, Amino oder Fluor, substituiert sein können oder R₃ für eine Arylgruppe steht, wobei die Gruppe mit 1-5 Substituenten Y substituiert sein kann, wobei Y die oben erwähnte Bedeutung hat oder R₃ für eine Aryl-C₁₋₅-Alkylgruppe oder eine Diaryl-C₁₋₅-Alkylgruppe steht, wobei die Phenyl- oder heteroaromatischen Ringe in diesen Gruppen mit 1-5 Substituenten Y substituiert sein können, wobei Y die oben erwähnte Bedeutung hat, oder R₃ für eine lineare oder verzweige C₄₋₈-Alkenyl- oder C₄₋₈-Akynylgruppe steht, wobei die lineare oder verzweigte C₄₋₈-Alkenyl- oder C₄₋₈-Alkynylgruppe mit 1-3 Fluoratomen substituiert sein kann, oder, nur wenn n = 1, R₃ zusätzlich für eine verzweigte oder lineare C₂₋₁₀-Heteroalkylgruppe, die 1-2 Heteroatome ausgewählt aus N, O oder S enthält, steht,
- R₄ für ein Wasserstoffatom, eine C₁₋₄-Alkylgruppe steht oder R₃ und R₄ - gemeinsam mit dem Stickstoffatom an das sie gebunden sind - eine gesättigte oder ungesättigte, nicht-aromatische oder teilweise aromatische, monozyklische, bizyklische oder trizyklische heterozyklische Gruppe mit 5 bis 11 Ringatomen bilden, wobei die heterozyklische Gruppe mit 1-5 Substituenten, ausgewählt aus Aryl, Aryl-C₁₋₃-Alkyl, Diarylmethyl oder Y, substituiert sein kann, wobei Y die oben erwähnte Bedeutung hat,
- A für eine Carbonyl- (C = O), Thiocarbonyl-, (C = S) oder Sulfonylgruppe (SO₂ steht, unter der Bedingung, dass, wenn A für eine Thiocarbonylgruppe (C = S) steht, n den Wert 1 hat,
und Stereoisomere und N-Oxide davon, und isotopisch markierte Verbindungen von Formel (I), ebenso wie pharmakologisch akzeptable Salze, Hydrate, Solvate, Komplexe und Konjugate der Verbindungen der Formel (I) und deren Stereoisomere, N-Oxide oder isotopisch markierte Analoge, mit der Voraussetzung, dass der Begriff "Heteroalkyl", wie hier verwendet, Alkylgruppen mit Heteroatomen in jeder Position einschließt, wodurch N-gebundene, O-gebundene oder S-gebundene Alkylgruppen eingeschlossen sind; und mit der Voraussetzung, dass der Begriff "Aryl", wie hier verwendet, monozyklische oder fusionierte bizyklische aromatische oder heteroaromatische Gruppen meint, wobei die heteroaromatischen Gruppen ein oder zwei Heteroatome, ausgewählt aus der Gruppe (N, O, S), enthalten.

2. Verbindungen nach Anspruch 1 der allgemeinen Formel (I), wobei R₁ für ein Wasserstoffatom steht und die anderen Symbole die Bedeutungen haben, die ihnen in Anspruch 1 zugeschrieben wurden.

3. Verbindungen nach Anspruch 2 der allgemeinen Formel (I), wobei A für eine Carbonylgruppe steht und die anderen Symbole die Bedeutungen haben, die ihnen in Anspruch 2 zugeschrieben wurden.

4. Verbindungen nach Anspruch 3 der allgemeinen Formel (I), wobei R₂ für eine Phenyl-, Thienyl- oder Pyridylgruppe steht, wobei die Phenyl-, Pyridyl- oder Thienylgruppe mit 1, 2 oder 3 Substituenten Y substituiert sein kann, und die anderen Symbole die Bedeutungen haben, die ihnen in Anspruch 3 zugeschrieben wurden.

5. Verbindungen nach Anspruch 4 der allgemeinen Formel (I), wobei n 1 ist und die anderen Symbole die Bedeutungen haben, die ihnen in Anspruch 4 zugeschrieben wurden.

6. Verbindungen nach Anspruch 5 der allgemeinen Formel (I), wobei R₄ für ein Wasserstoffatom steht und die anderen Symbole die Bedeutungen haben, die ihnen in Anspruch 5 zugeschrieben wurden.

7. Verbindungen nach Anspruch 6 der allgemeinen Formel (I), wobei R für eine verzweigte oder lineare C₃₋₈-Alkylgruppe steht, wobei die verzweigte oder lineare C₃₋₈-Alkylgruppe mit 1-3 Fluoratomen substituiert sein kann und die anderen Symbole die Bedeutungen haben, die ihnen in Anspruch 6 zugeschrieben wurden.

8. Verbindung nach Anspruch 1, welche ist:
N-[(1R,2S,5R)-rel-6,6-Dimethylbicyclo[3.1.1]heptan-2-methyl]-3-(n-pentyl)-4-phenyl-4,5-dihydro-(1H)-pyrazol-1-carboxymid
N-(1-Adamantyl)-3-(n-pentyl)-4-phenyl-4,5-dihydro-(1H)-pyrazol-1-carboxamid
N-(exo-Bicyclo[2.2.1]hept-2-yl)-3-(n-pentyl)-4-phenyl-4,5-dihydro-(1H)-pyrazol-1-carboxamid
N-Phenyl-3-(n-pentyl)-4-phenyl-4,5-dihydro-(1H)-pyrazol-1-carboxamid
N-[(1R,2S,5R)-rel-6,6-Dimethylbicyclo[3.1.1]heptan-2-methyl]-3-(benzyl)-4-phenyl-4,5-dihydro-(1H)-pyrazol-1-carboxamid
N-(1-Adamantyl)-3-(benzyl)-4-phenyl-4,5-dihydro-(1H)-pyrazol-1-carboxamid
N-[(1R,2S,5R)-rel-6,6-Dimethylbicyclo[3.1.1]heptan-2-methyl]-3-(n-butyl)-4-phenyl-4,5-dihydro-(1H)-pyrazol-1-carboxamid
N-[(1R,2S,5R)-rel-6,6-Dimethylbicyclo[3.1.1]heptan-2-methyl]-3-[3-(1-piperidinyl)propyl]-4-phenyl-4,5-dihydro-(1H)-pyrazol-1-carboxamid
N-[(1R,2S,5R)-rel-6,6-Dimethylbicyclo[3.1.1]heptan-2-methyl]-3-(n-propyl)-4-phenyl-4,5-dihydro-(1H)-pyrazol-1-carboxamid
N-(Benzyl)-3-(n-pentyl)-4-phenyl-4,5-dihydro-(1H)-pyrazol-1-carboxamid
N-(1-Adamantyl)methyl-3-(n-pentyl)-4-phenyl-4,5-dihydro-(1H)-pyrazol-1-carboxamid
N-(Cyclohexylmethyl)-3-(n-pentyl)-4-phenyl-4,5-dihydro-(1H)-pyrazol-1-carboxamid
N-[endo-(1R,2S,4R)-1,7,7-Trimethylbicyclo[2.2.1]hept-2-yl]-3-(n-pentyl)-4-phenyl-4,5-dihydro-(1H)-pyrazol-1-carboxamid
N-[endo-(1S)-1,3,3-Trimethylbicyclo[2.2.1]hept-2-yl]-3-(n-pentyl)-4-phenyl-4,5-dihydro-(1H)-pyrazol-1-carboxamid
N-[(1R,2S,5R)-rel-6,6-Dimethylbicyclo[3.1.1]heptan-2-methyl-3-(n-propyl)-4-(2-pyridyl)-4,5-dihydro-(1H)-pyrazol-1-carboxamid
N-(1-Phenylethyl)-3-(n-pentyl)-4-phenyl-4,5-dihydro-(1H)-pyrazol-1-carboxamid
N-(2-Adamantyl)-3-(n-pentyl)-4-phenyl-4,5-dihydro-(1H)-pyrazol-1-carboxamid
N-(1-Naphtyl)-3-(n-pentyl)-4-phenyl-4,5-dihydro-(1H)-pyrazol-1-carboxamid
N-(1-Methyl-1-phenylethyl)-3-(n-pentyl)-4-phenyl-4,5-dihydro-(1H)-pyrazol-1-carboxamid
N-(2,2-Dipehylpropyl)-3-(n-pentyl)-4-phenyl-4,5-dihydro-(1H)-pyrazol-1-carboxamid
N-((3-Trifluormethyl)benzyl)-3-(n-pentyl)-4-phenyl-4,5-dihydro-(1H)-pyrazol-1-carboxamid
N-(2,2-Dimethypropyl)-3-(n-pentyl)-4-phenyl-4,5-dihydro-(1H)-pyrazol-1-carboxamid
N-(Naphthalen-1-yl-methyl)-3-(n-pentyl)-4-phenyl-4,5-dihydro-(1H)-pyrazol-1-carboxamid
N-[(Dimethylamino)-2,2-dimethylpropyl]-3-(n-pentyl)-4-phenyl-4,5-dihydro-(1H)-pyrazol-1-carboxamid
N-[endo-(1R,2S,4R)-1,7,7-Trimethylbicyclo[2.2.1]hept-2-yl]-3-(n-butyl)-4-phenyl-4,5-dihydro-(1H)-pyrazol-1-carboxamid
N-2-(4-Fluorphenyl)-1,1-dimethylethyl)-3-(n-butyl)-4-phenyl-4,5-dihydro-(1H)-pyrazol-1-carboxamid
N-[endo-(1R,2S,4R)-1,7,7-Trimethylbicyclo[2.2.1]hept-2-yl]-3-(4,4,4-trifluor-n-butyl)-4-phenyl-4,5-dihydro-(1H)-pyrazol-1-carboxamid
N-(2-(4-Fluorphenyl)-1,1-dimethylethyl)-3-(4,4,4-trifluor-n-butyl)-4-phenyl-4,5-dihydro-(1H)-pyrazol-1-carboxamid
N-(2-(4-Fluorphenyl)-1,1-dimethylethyl)-3-(n-pentyl)-4-phenyl-4,5-dihydro-(1H)-pyrazol-1-carboxamid
N-[endo-(1R,2S,4R)-1,7,7-Trimethylbicyclo[2.2.1]hept-2-yl]-3-(1,1-dimethyl-n-butyl)-4-phenyl-4,5-dihydro-(1H)-pyrazol-1-carboxamid
N-[endo-(1R,2S,4R)-1,7,7-Trimethylbicyclo[2.2.1]hept-2-yl]-3-(3,3,3-trifluorpropyl)-4-phenyl-4,5-dihydro-(1H)-pyrazol-1-carboxamid
N-[endo-(1R,2S,4R)-1,7,7-Trimethylbicyclo[2.2.1]hept-2-yl]-3-(1,1-diemthylpropyl)-4-pheny-4,5-dihydro-(1H)-pyrazol-1-carboxamid
N-(2-(4-Fluorphenyl)-1,1-dimethylethyl)-3-(1,1-dimethylpropyl)-4-phenyl-4,5-dihydro-(1H)-pyrazol-1-carboxamid
N-[endo-(1R,2S,4R)-1,7,7-Trimethylbicyclo[2.2.1]hept-2-yl]-3-(1,1-dimethyl-3,3,3-trifluorpropyl)-4-phenyl-4,5-dihydro-(1H)-pyrazol-1-carboxamid
N-[endo-(1R)-1,3,3-Trimethylbicyclo[2.2.1]hept-2-yl]-3-(n-butyl)-4-phenyl-4,5-dihydro-(1H)-pyrazol-1-carboxamid
N-(1-Methyl-1-phenylethyl)-3-(n-butyl)-4-phenyl-4,5-dihydro-(1H)-pyrazol-1-carboxamid
N-(2-Adamantyl)-3-(n-butyl)-4-phenyl-4,5-dihydro-(1H)-pyrazol-1-carboxamid
N-[exo-(1R,2R,4R)-1,7,7-Trimethylbicyclo[2.2.1]hept-2-yl]-3-(n-butyl)-4-phenyl-4,5-dihydro-(1H)-pyrazol-1-carboxamid
N-(2-Phenyl-1,1-dimethylethyl)-3-(n-butyl)-4-(3-fluorphenyl)-4,5-dihydro-(1H)-pyrazol-1-carboxamid
N-(2-Phenyl-1,1-diemthylethyl)-3-(n-butyl)-4-(2-fluorphenyl)-4,5-dihydro-(1H)-pyrazol-1-carboxamid
N-Phenyl-3-(4-chlorbenzyl)-4-(4-chlorphenyl)-4,5-dihydro-(1H)-pyrazol-1-carboxamid
N-(4-Methoxyphenyl)-3-(4-chlorbenzyl)-4-(4-chlorphenyl)-4,5-dihydro-(1H)-pyrazol-1-carboxamid
N-[endo-(1R,2S,4R)-1,7,7-Trimethylbicyclo[2.2.1]hept-2-yl]-3-(n-butyl)-4-(2-methoxyphenyl)-4,5-dihydro-(1H)-pyrazol-1-carboxamid
N-(1-Methyl-1-phenylethyl)-3-(n-butyl)-4-(2-methoxyphenyl)-4,5-dihydro-(1H)-pyrazol-1-carboxamid
N-[endo-(1R,25,4R)-1,7,7-Trimethylbicyclo[2.2.1]hept-2-yl]-3-(n-butyl)-4-(2-fluorphenyl)-4,5-dihydro-(1H)-pyrazol-1-carboxamid
N-[endo-(1R,2S,4R)-1,7,7-Trimethylbicyclo[2.2.1]hept-2-yl]-3-(n-butyl)-4-(pyrid-3-yl)-4,5-dihydro-(1H)-pyrazol-1-carboxamid
N-[(1R,2R,3R,5S)-2,7,7-trimethylbicyclo[3.1.1]hept-3-yl]-3-(n-butyl)-4-(3-fluorphenyl)-4,5-dihydro-(1H)-pyrazol-1-carboxamid
N-[endo-(1R)-1,3,3-Trimethylbicyclo[2.2.1]hept-2-yl]-3-(n-butyl)-4-(2-fluorphenyl)-4,5-dihydro-(1H)-pyrazol-1-carboxamid
N-[2-(Trifluormethyl)benzyl]-3-(n-butyl)-4-(3-fluorphenyl)-4,5-dihydro-(1H)-pyrazol-1-carboxamid
N-[exo-(1R,2R,4R)-1,7,7-Trimethylbicyclo[2.2.1]hept-2-yl]-3-(n-butyl)-4-(2-fluorphenyl)-4,5-dihydro-(1H)-pyrazol-1-carboxamid
N-(1-Methyl-1-phenylethyl)-3-(n-butyl)-4-(3-fluorphenyl)-4,5-dihydro-(1H)-pyrazol-1-carboxamid
N-(1-Methyl-1-phenylethyl)-3-(n-butyl)-4-(4-chlorphenyl)-4,5-dihydro-(1H)-pyrazol-1-carboxamid
N-[2-(Trifluormethyl)benzyl]-3-(n-butyl)-4-(4-chlorphenyl)-4,5-dihydro-(1H)-pyrazol-1-carboxamid
N-[endo-(1R,2S,4R)-1,7,7-Trimethylbicyclo[2.2.1]hept-2-yl]-3-(cyclopropylmethyl)-4-phenyl-4,5-dihydro-(1H)-pyrazol-1-carboxamid
N-[endo-(1R,2S,4R)-1,7,7-Trimethylbicyclo[2.2.1]hept-2-yl]-3-(n-butyl)-4-(4-fluorphenyl)-4,5-dihydro-(1H)-pyrazol-1-carboxamid
N-(1-Methyl-1-phenylethyl)-3-(n-butyl)-4-(4-fluorphenyl)-4,5-dihydro-(1H)-pyrazol-1-carboxamid
N-(Adamant-2-yl)-3-(n-butyl)-4-(4-fluorphenyl)-4,5-dihydro-(1H)-pyrazol-1-carboxamid
N-(1-Methyl-1-(4-fluorphenyl)ethyl)-3-(n-butyl)-4-(4-fluorphenyl)-4,5-dihydro-(1H)-pyrazol-1-carboxamid
N-(1-Methyl-1-(4-fluorphenyl)ethyl)-3-(n-butyl)-4-phenyl-4,5-dihydro-(1H)-pyrazol-1-carboxamid
N-(1-Methyl-1-phenylethyl)-3-(n-pentyl)-4-(2-fluorphenyl)-4,5-dihydro-(1H)-pyrazol-1-carboxamid
N-[endo-(1R,2S,4R)-1,7,7-Trimethylbicyclo[2.2.1]hept-2-yl]-3-(n-pentyl)-4-(2-fluorphenyl)-4,5-dihydro-(1H)-pyrazol-1-carboxamid
N-(1-Methyl-1-(4-fluorphenyl)ethyl)-3-(n-pentyl)-4-(2-fluorphenyl)-4,5-dihydro-(1H)-pyrazol-1-carboxamid
N-(1-Methyl-1-(4-fluorphenyl)ethyl)-3-(n-pentyl)-4-(3-fluorphenyl)-4,5-dihydro-(1H)-pyrazol-1-carboxamid
N-(Adamant-2-yl)-3-(n-pentyl)-4-(2-fluorphenyl)-4,5-dihydro-(1H)-pyrazol-1-carboxamid
N-(Adamant-2-yl)-3-(n-pentyl)-4-(3-fluorphenyl)-4,5-dihydro-(1H)-pyrazol-1-carboxamid
N-(1-Methyl-1-phenylethyl)-3-(n-butyl)-4-(benzo[b]thiophen-3-yl)-4,5-dihydro-(1H)-pyrazol-1-carboxamid
N-[endo- (1R,25,4R)-1,7,7-Trimethylbicyclo[2.2.1]hept-2-yl]-3-(n-butyl)-4-(benzo[b]thiophen-3-yl)-4,5-dihydro-(1H)-pyrazol-1-carboxamid
N-(1-Methyl-1-phenylethyl)-3-(n-butyl)-4-(thiophen-3-yl)-4,5-dihydro-(1H)-pyrazol-1-carboxamid
N-(1-Methyl-1-phenylethyl)-3-(but-3-ynyl)-4-(2-fluorphenyl)-4,5-dihydro-(1H)-pyrazol-1-carboxamid
N-[endo-(1R,2S,4R)-1,7,7-Trimethylbicyclo[2.2.]hept-2-yl]-3-(but-3-ynyl)-4-(2-fluorphenyl)-4,5-dihydro-(1H)-pyrazol-1-carboxamid
N-[endo-(1R,2S,4R)-1,7,7-Trimethylbicyclo[2.2.1]hept-2-yl]-3-(1-phenylcyclopropyl)-4-phenyl-4,5-dihydro-(1H)-pyrazol-1-carboxamid
N-(1-Methyl-1-phenylethyl)-3-(1-phenylcyclopropyl)-4-phenyl-4,5-dihydro-(1H)-pyrazol-1-carboxamid
N-[endo-(1R,2S,4R)-1,7,7-Trimethylbicyclo[2.2.1]hept-2-yl]-3-(2,2,3,3-tetramethylcyclopropyl)-4-phenyl-4,5-dihydro-(1H)-pyrazol-1-carboxamid
N-(1-Methyl-1-phenylethyl)-3-(2,2,3,3-tetramethylcyclopropyl)-4-phenyl-4,5-dihydro-(1H)-pyrazol-1-carboxamid
N-[(1R,2R,3R,5S)-2,7,7-Trimethylbicyclo[3.1.1]hept-3-yl]-3-(n-butyl)-4-(4-chlorphenyl)-4,5-dihydro-(1H)-pyrazol-1-carboxamid
N-(1-Methyl-1-phenylethyl)-3-(n-pentyl)-4-(3-fluorphenyl)-4,5-dihydro-(1H)-pyrazol-1-carboxamid
N-[endo-(1R,2S,4R)-1,7,7-Trimethylbicyclo[2.2.1]hept-2-yl]-3-(n-pentyl)-4-(3-fluorphenyl)-4,5-dihydro-(1H)-pyrazol-1-carboxamid
N-(1-Methyl-1-phenylethyl)-3-(n-pentyl)-4-(4-fluorphenyl)-4,5-dihydro-(1H)-pyrazol-1-carboxamid
N-[endo-(1R,2S,4R)-1,7,7-Trimethylbicyclo[2.2.1]hept-2-yl]-3-(n-pentyl-)-4-(4-fluorphenyl)-4,5-dihydro-(1H)-pyrazol-1-carboxamid
N-[(1S,2S,3S,5R)-2,7,7-Trimethylbicyclo[3.1.1]hept-3-yl]-3-(n-butyl)-4-(3-fluorphenyl)-4,5-dihydro-(1H)-pyrazol-1-carboxamid
N-(1-Methyl-1-(4-fluorphenyl)ethyl)-3-(n-butyl)-4-(3-fluorphenyl)-4,5-dihydro-(1H)-pyrazol-1-carboxamid
N-(1-Methyl-1-(4-fluorphenyl)ethyl)-3-(n-butyl)-4-(2-fluorphenyl)-4,5-dihydro-(1H)-pyrazol-1-carboxamid
N-[(1S,2S,3S,5R)-2,7,7-Trimethylbicyclo[3.1.1]hept-3-yl]-3-(n-butyl)-4-(2-fluorphenyl)-4,5-dihydro-(1H)-pyrazol-1-carboxamid
N-[endo-(1R,2S,4R)-1,7,7-Trimethylbicyclo[2.2.1]hept-2-yl]-3-(n-butyl)-4-(thien-3-yl)-4,5-dihydro-(1H)-pyrazol-1-carboxamid
N-[endo-(1R,2S,4R)-1,7,7-Trimethylbicyclo(2.2.1]hept-2-yl]-3-(3,3,3-trifluoro-1-methoxymethylpropyl)-4-phenyl-4,5-dihydro-(1H)-pyrazol-1-carboxamid
N-[endo-(1R,2S,4R)-1,7,7-Trimethylbicyclo[2.2.1]hept-2-yl]-3-(n-butyl)-4-hydroxy-4-phenyl-4,5-dihydro-(1H)-pyrazol-1-carboxamid
1-(1-Naphtoyl)-3-(n-pentyl)-4-phenyl-4,5-dihydro-(1H)-pyrazol
[3-(n-Pentyl)-4-phenyl-4,5-dihydro-(1H)-pyrazol-1-yl]-[1-(4-chlorphenyl)cyclopentyl]methanon
[3-(n-Pentyl)-4-phenyl-4,5-dihydro-(1H)-pyrazol-1-yl]-(napht-2-yl)methanon
[3-(n-Pentyl)-4-phenyl-4,5-dihydro-(1H)-pyrazol-1-yl]-(diphenylmethyl)methanon
[3-(n-Pentyl)-4-phenyl-4,5-dihydro-(1H)-pyrazol-1-yl]-(3-chlorbenzothien-2-yl]methanon
[3-(n-Pentyl)-4-phenyl-4,5-dihydro-(1H)-pyrazol-1-yl]-(benzofuran-2-yl]methanon
[3-(n-Pentyl)-4-phenyl-4,5-dihydro-(1H)-pyrazol-1-yl]-[2,4,4-(trimethyl)pentyl]methanon
[3-(n-Pentyl)-4-phenyl-4,5-dihydro-(1H)-pyrazol-1-yl]-[3-(trifluormethyl)phenyl]methanon
(Cis-3,4,5-Trimethylpiperazin-1-yl)[3-(n-pentyl)-4-phenyl-4,5-dihydro-(1H)-pyrazol-1-yl]methanon
N-endo[(1R,2S,4R)-1,7,7-Trimethylbicyclo[2.2.1]hept-2-yl]-3-(n-pentyl)-4-phenyl-4,5-dihydro-(1H)-pyrazol-1-carboxamid (Diastereomer A)
N-endo[(1R,2S,4R)-1,7,7-Trimethylbicyclo[2.2.1]hept-2-yl]-3-(n-pentyl)-4-phenyl-4,5-dihydro-(1H)-pyrazol-1-carboxamid (Diastereomer B)
N-endo[(1R,2S,4R)-1,7,7-Trimethylbicyclo[2.2.1]hept-2-yl]-3-(n-butyl)-4-(3-fluorphenyl)-4,5-dihydro-(1H)-pyrazol-1-carboxamid (Diastereomer A)
N-endo[(1R,2S,4R)-1,7,7-Trimethylbicyclo[2.2.1]hept-2-yl]-3-(n-butyl)-4-(3-fluorphenyl)-4,5-dihydro-(1H)-pyrazol-1-carboxamid (Diastereomer B)
N-endo[(1R,2S,4R)-1,7,7-Trimethylbicyclo[2.2.1]hept-2-yl]-3-(n-butyl)-4-(chlorphenyl)-4,5-dihydro-(1H)-pyrazol-1-carboxamid (Diastereomer A)
N-endo[(1R,2S,4R)-1,7,7-Trimethylbicyclo[2.2.1]hept-2-yl]-3-(n-butyl)-4-(chlorphenyl)-4,5-dihydro-(1H)-pyrazol-1-carboxamid (Diastereomer B)
N-(1,2,2,6,6-Pentamethylpiperidin-4-yl)-3-(n-butyl)-4-(2-fluorphenyl)-4,5-dihydro-(1H)-pyrazol-1-carboxamid
N-(4-Methoxyphenyl)-3-(n-pentyl)-4-phenyl-4,5-dihydro-(1H)-pyrazol-1-carboxamid
N-(4-Methoxyphenyl)-3-(n-pentyl)-4-phenyl-4,5-dihydro-(1H)-pyrazol-1-carboxamid
N-(Phenethyl)-3-(n-pentyl)-4-phenyl-4,5-dihydro-(1H)-pyrazol-1-carboxamid
N-(2-Phenyltranscyclopropyl)-3-(n-pentyl)-4-phenyl-4,5-dihydro-(1H)-pyrazol-1-carboxamid
N-(1-Naphthalen-1-yl-ethyl)-3-(n-pentyl)-4-phenyl-4,5-dihydro-(1H)-pyrazol-1-carboxamid
N-[2-(Trifluormethyl)phenyl)-3-(n-pentyl)-4-phenyl-4,5-dihydro-(1H)-pyrazol-1-carboxamid
N-Cycloheptyl-3-(n-pentyl)-4-phenyl-4,5-dihydro-(1H)-pyrazol-1-carboxamid
N-Cyclooctyl-3-(n-pentyl)-4-phenyl-4,5-dihydro-(1H)-pyrazol-1-carboxamid
N-(1,2,3,4-Tetrahydronaphthalen-1-yl)-3-(n-pentyl)-4-phenyl-4,5-dihydro-(1H)-pyrazol-1-carboxamid
N-[2,2-(Diphenyl)ethyl]-3-(n-pentyl)-4-phenyl-4,5-dihydro-(1H)-pyrazol-1-carboxamid
(3-Pentyl-4-phenyl-4,5-dihydropyrazol-1-yl)-[4-(2-pyrimidinyl)piperazin-1-yl]methanon
N-[2-(4-Fluorphenyl)ethyl]-3-(n-pentyl)-4-phenyl-4,5-dihydro-(1H)-pyrazol-1-carboxamid
(3-Pentyl-4-phenyl-4,5-dihydropyrazol-1-yl)-[azepan-1-yl]methanon
N-(Quinolin-3-yl)-3-(n-pentyl)-4-phenyl-4,5-dihydro-(1H)-pyrazol-1-carboxamid
N-[1-(Ethyl)propyl]-3-(n-pentyl)-4-phenyl-4,5-dihydro-(1H)-pyrazol-1-carboxamid
N-(2,2,2-Trifluorethyl)-3-(n-pentyl)-4-phenyl-4,5-dihydro-(1H)-pyrazol-1-carboxamid
N-(Pyridin-3-ylmethyl)-3-(n-pentyl)-4-phenyl-4,5-dihydro-(1H)-pyrazol-1-carboxamid
N-(2-Indanyl)-3-(n-pentyl)-4-phenyl-4,5-dihydro-(1H)-pyrazol-1-carboxamid
(3-Pentyl-4-phenyl-4,5-dihydropyrazol-1-yl)-(1,2,3,4-tetrahydroisoquinolin-2-yl)methanon
N-(Methyl)-N-(Naphthalen-1-ylmethyl)-3-(n-pentyl)-4-phenyl-4,5-dihydro-(1H)-pyrazol-1-carboxamid
N-(3,3-Diphenylpropyl)-3-(n-pentyl)-4-phenyl-4,5-dihydro-(1H)-pyrazol-1-carboxamid
N-(Napht-1-yl)-3-(n-pentyl)-4-phenyl-4,5-dihydro-(1H)-pyrazolcarbothiamid
N-[1-(Ethyl)propyl]-3-(n-pentyl)-4-phenyl-4,5-dihydro-(1H)-pyrazolcarbothiamid
N-[Pyridin-3-ylmethyl]-3-(n-pentyl)-4-phenyl-4,5-dihydro-(1H)-pyrazolcarbothiamid
N-[exo-Bicyclo[2.2.1]hept-2-yl]-3-(n-pentyl)-4-phenyl-4,5-dihydro-(1H)-pyrazolcarbothiamid
1-(Naphthalen-1-ylsulfonyl)-3-(n-butyl)-4-(2-fluorphenyl)-4,5-dihydro-(1H)-pyrazol
1-(Naphthalen-2-ylsulfonyl)-3-(n-butyl)-4-(2-fluorphenyl)-4,5-dihydro-(1H)-pyrazol
N-[(1R,2S,5R)-rel-6,6-Dimethylbicyclo[3.1.1]heptan-2-methyl]-3-(n-pentyl)-4-phenyl-4,5-dihydro-(1H)-pyrazol-1-sulfonamid

9. Verbindungen der allgemeinen Formel (IV) 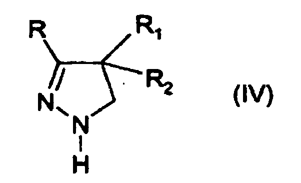 wobei R und R₁ die selben Bedeutungen haben, die ihnen in Anspruch 1 zugeschrieben wurden, und R₂ für eine Phenylgruppe steht, die mit 1-5 Substituenten Y2 substituiert sein kann, welche gleich oder verschieden sein können, ausgewählt aus der Gruppe C₁₋₃-Alkoxy, Hydroxy, Trifluormethyl, Trifluormethylthio, Trifluormethoxy, Nitro, Amino, Mono- oder Dialkyl-(C₁₋₂)-Amino, Mono- oder Dialkyl-(C₁₋₂)-Amido, (C₁₋₃)-Alkylsulfonyl, Dimethylsulfamido, C₁₋₃-Alkoxycarbonyl, Carboxyl, Trifluormethylsulfonyl, Cyano, Carbamoyl, Sulfamoyl, ortho-Halogen, meta-Halogen, ortho-C₁₋₃-Alkyl, meta-C₁₋₃-Alkyl und Acetyl, oder R₂ für eine Thienyl- oder Pyridylgruppe steht, wobei die Gruppen mit ein oder zwei Substituenten Y substituiert sein können, wobei die Y-Gruppe die Bedeutung hat, die ihr in Anspruch 1 zugeschrieben wurde, und wobei solche Verbindungen bei der Synthese von Verbindungen der allgemeinen Formel (I) nützlich sind.

10. Verbindungen der allgemeinen Formel (VIII) 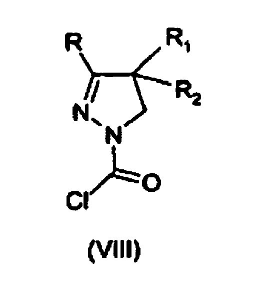 wobei R und R₂ die selben Bedeutungen haben, die ihnen in Anspruch 1 zugeschrieben wurden, und R₁ für Wasserstoff steht, wobei solche Verbindungen bei der Synthese von Verbindungen der allgemeinen Formel (I) nützlich sind, wobei n = 1.

11. Verbindung nach einem der Ansprüche 1-8 oder ein pharmakologisch akzeptables Salz, Hydrat, Solvat oder Komplex davon, zur Verwendung als ein Medikament.

12. Medikament **dadurch gekennzeichnet, dass** es eine Verbindung gemäß einem der Ansprüche 1-8 oder ein pharmakologisch akzeptables Salz, Hydrat, Solvat oder Komplex davon enthält.

13. Pharmazeutische Zusammensetzung, die zusätzlich zu einem pharmazeutisch akzeptablen Träger und/oder zumindest einem pharmazeutisch akzeptablen Hilfsstoff, eine pharmakologisch wirksame Menge von zumindest einer Verbindung nach einem der Ansprüche 1-8 oder ein pharmakologisch akzeptables Salz, Hydrat, Solvat oder Komplex davon als einen Wirkstoff umfasst.

14. Pharmazeutische Zusammensetzung nach Anspruch 13 für die Prävention oder die Behandlung von multipler Sklerose, traumatischer Hirnverletzung, Schmerz, Appetitstörungen, Epilepsie, der Alzheimer-Krankheit, dem Tourettesyndrom, zerebraler Ischämie oder Magen-Darm-Störungen.

15. Pharmazeutische Zusammensetzung nach Anspruch 13, die des Weiteren zumindest ein zusätzliches Therapeutikum umfasst.

16. Verfahren zur Herstellung pharmazeutischer Zusammensetzungen nach Anspruch 13, **dadurch gekennzeichnet, dass** eine Verbindung nach einem der Ansprüche 1-8 in eine für die Verabreichung geeignete Form gebracht wird.

17. Pharmazeutische Zusammensetzung hergestellt durch Mischen einer Verbindung nach Anspruch 1 und eines pharmazeutisch akzeptablen Trägers und/oder zumindest einem pharmazeutisch akzeptablen Hilfsstoff.

18. Verwendung einer Verbindung nach einem der Ansprüche 1-8 für die Herstellung von einer pharmazeutischen Zusammensetzung für die Behandlung von multipler Sklerose, traumatischer Hirnverletzung, Schmerz, Appetitstörungen, Epilepsie, der Alzheimer-Krankheit, dem Tourettesyndrom, zerebraler Ischämie und Magen-Darm-Störungen.

## Revendications

1. Composés de la formule générale (I) 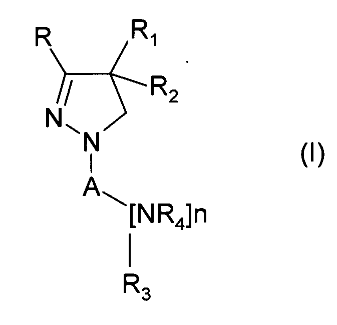 dans laquelle
- R représente un groupe alkyle en C₂₋₁₀, un groupe alcényle en C₄₋₁₀, un groupe alcynyle en C₄₋₁₀, un groupe hétéroalkyle en C₂₋₁₀, un groupe (cycloalkyle en C₅₋₈)-(alkyle en C₁₋₅) ou un groupe (hétérocycloalkyle en C₅₋₈)-(alkyle en C₁₋₅), dans lequel le(les) hétéroatome(s) est(sont) soit N, O, soit S, lequel groupe alkyle en C₂₋₁₀, groupe alcényle en C₄₋₁₀, groupe alcynyle en C₄₋₁₀, groupe hétéroalkyle en C₂₋₁₀, groupe (cycloalkyle en C₅₋₈)-(alkyle en C₁₋₅) ou groupe (hétérocycloalkyle en C₅₋₈)-(alkyle en C₁₋₅) peut être substitué par 1-5 substituants choisis parmi un groupe méthyle, éthyle, hydroxy, amino ou fluoro, ou R représente un groupe aryl-(alkyle en C₁₋₃) ou un groupe aryl-(hétéroalkyle en C₁₋₃) dans lequel les groupes aryle peuvent être substitués par 1-5 substituants Y, lesquels peuvent être identiques ou différents, choisis dans le groupe constitué des groupes alkyle ou alcoxy en C₁₋₃, hydroxy, un atome d'halogène, des groupes trifluorométhyle, trifluorométhylthio, trifluoro-méthoxy, nitro, amino, (mono- ou dialkyle en C₁₋₂)-amino, (mono- ou dialkyle en C₁₋₂)-amido, (alkyle en C₁₋₃)sulfonyle, diméthylsulfamido, (alcoxy en C₁₋₃)-carbonyle, carboxyle, trifluorométhylsulfonyle, cyano, carbamoyle, sulfamoyle, phényle et acétyle, ou R représente un groupe cyclopropyle, lequel groupe cyclopropyle peut être substitué par 1-5 substituants choisis parmi un groupe méthyle, éthyle, fluoro ou par un groupe alkyle linéaire ou ramifié en C₃₋₅ ou par un groupe benzyle ou aryle, dans lequel le groupe aryle ou benzyle peut être substitué par 1-5 substituants Y,
- R₁ représente un atome d'hydrogène, un groupe hydroxy, alcoxy en C₁₋₃, acétyloxy ou propionyloxy,
- R₂ représente un groupe aryle qui peut être substitué par 1-5 substituants Y, où Y a la signification indiquée ci-dessus,
- n est soit égal à 0, soit à 1,
- R₃ représente un groupe alkyle linéaire en C₃₋₁₀, un groupe alkyle ramifié en C₅₋₁₀, un groupe cyclopropyle, cyclobutyle, cyclopentyle, cycloheptyle ou cyclooctyle, un groupe bicycloalkyle en C₅₋₁₀, un groupe tricycloalkyle en C₆₋₁₀ ou un groupe tétracycloalkyle en C₈₋₁₁, lesquels groupes peuvent être substitués par 1-5 substituants choisis parmi les groupes méthyle, éthyle, hydroxy, amino, fluoro, ou R₃ représente un groupe cycloalkyle en C₃₋₈, lequel groupe cycloalkyle en C₃₋₈ est substitué par un groupe aryle, lequel groupe aryle peut être substitué par 1-5 substituants Y, où Y a la signification indiquée ci-dessus, ou R₃ représente un groupe 2,2,2-trifluoroéthyle ou 2-fluoroéthyle ou R₃ représente un groupe cyclohexyle, lequel groupe est substitué par 1-5 substituants choisis parmi les groupes méthyle, éthyle, hydroxy, amino ou fluoro, ou R₃ représente un groupe hétérocycloalkyle en C₅₋₈, un groupe bicyclohétéroalkyle en C₆₋₁₀, un groupe tricyclohétéroalkyle en C₇₋₁₀, lesquels groupes peuvent être substitués par 1-5 substituants choisis parmi les groupes méthyle, éthyle, hydroxy, amino ou fluoro, ou R₃ représente un groupe (cycloalkyle en C₃₋₈)-(alkyle en C₁₋₃), un groupe (bicycloalkyle en C₅₋₁₀)-(alkyle en C₁₋₃), un groupe (tricycloalkyle en C₆₋₁₀)-(alkyle en C₁₋₃), lesquels groupes peuvent être substitués par 1-5 substituants choisis parmi un groupe méthyle, éthyle, hydroxy, amino ou fluoro, ou R₃ représente un groupe (hétérocycloalkyle en C₃₋₈)-(alkyle en C₁₋₃) ramifié ou linéaire, un groupe (bicyclohétéroalkyle en C₅₋₁₀)-(alkyle en C₁₋₃), un groupe (tricyclohétéroalkyle en C₆₋₁)-(alkyle en C₁₋₃), lesquels groupes peuvent être substitués par 1-5 substituants choisis parmi les groupes méthyle, éthyle, hydroxy, amino ou fluoro, ou R₃ représente un groupe aryle, lequel groupe peut être substitué par 1-5 substituants Y, où Y a la signification indiquée ci-dessus, ou R₃ représente un groupe aryl-(alkyle en C₁₋₅) ou un groupe diaryl-(alkyle en C₁₋₅), dans lesquels groupes les cycles phényle ou hétéroaromatiques peuvent être substitués par 1-5 substituants Y, où Y a la signification indiquée ci-dessus, ou R₃ représente un groupe alcényle en C₄₋₈ ou un groupe alcynyle en C₄₋₈ linéaire ou ramifié, lequel groupe alcényle en C₄₋₈ ou groupe alcynyle en C₄₋₈ linéaire ou ramifié peut être substitué par 1-3 atomes de fluor, ou, uniquement lorsque n = 1, R₃ peut de plus représenter un groupe hétéroalkyle en C₂₋₁₀ linéaire ou ramifié contenant 1-2 hétéroatomes choisis parmi N, O ou S,
- R₄ représente un atome d'hydrogène, un groupe alkyle en Ci-₄ ou R₃ et R₄ - avec l'atome d'azote auquel ils sont liés - forment un groupe monocyclique, bicyclique ou tricyclique hétérocyclique non-aromatique ou partiellement aromatique, saturé ou insaturé ayant de 5 à 11 atomes cycliques, lequel groupe hétérocyclique peut être substitué par 1-5 substituants choisis parmi le groupe aryle, aryl-(alkyle en C₁₋₃), diarylméthyle, ou Y, où Y a la signification indiquée ci-dessus,
- A représente un groupe carbonyle (C=0), thiocarbonyle (C=S) ou sulfonyle (SO₂) à condition que lorsque A représente un groupe thiocarbonyle (C=S), n a la valeur de 1,
et stéréoisomères et N-oxydes de ceux-ci, et composés isotopiquement-marqués de la formule (I) ainsi que sels, hydrates, solvates, complexes et conjugués desdits composés de la formule (I) pharmacologiquement acceptables et leurs stéréoisomères, N-oxydes ou analogues isotopiquement-marqués,
avec la compréhension que le terme "hétéroalkyle", comme utilisé ici, comprend des groupes alkyle avec des hétéroatomes dans une position quelconque, incluant ainsi des groupes alkyle N-liés, O-liés ou S-liés ; et avec la compréhension que le terme "aryle", comme utilisé ici, indique des groupes aromatiques ou hétéroaromatiques monocycliques ou bicycliques condensés, lesquels groupes hétéroaromatiques contiennent un ou deux hétéroatomes choisis dans le groupe (N, O, S).

2. Composés selon la revendication 1 de la formule générale (I), dans laquelle R₁ représente un atome d'hydrogène et les autres symboles ont les significations données dans la revendication 1.

3. Composés selon la revendication 2 de la formule générale (I), dans laquelle A représente un groupe carbonyle et les autres symboles ont les significations données dans la revendication 2.

4. Composés selon la revendication 3 de la formule générale (I), dans laquelle R₂ représente un groupe phényle, thiényle ou pyridyle, lequel groupe phényle, pyridyle ou thiényle peut être substitué par 1, 2 ou 3 substituants Y, et les autres symboles ont les significations données dans la revendication 3.

5. Composés selon la revendication 4 de la formule générale (I), dans laquelle n est égal à 1 et les autres symboles ont les mêmes significations que données dans la revendication 4.

6. Composés selon la revendication 5 de la formule générale (I), dans laquelle R₄ représente un atome d'hydrogène et les autres symboles ont les mêmes significations que données dans la revendication 5.

7. Composés selon la revendication 6 de la formule générale (I), dans laquelle R représente un groupe alkyle linéaire ou ramifié en C₃₋₈, lequel groupe alkyle linéaire ou ramifié en C₃₋₈ peut être substitué par 1-3 atomes de fluor, et les autres symboles ont les mêmes significations que données dans la revendication 6.

8. Composé selon la revendication 1, lequel est le :
N-[(1R,2S,SR)-rel-6,6-diméthylbicyclo[3.1.1]heptane-2-méthyl]-3-(n-pentyl)-4-phényl-4,5-dihydro-(1H)-pyrazole-1-carboxamide N-(1-adamantyl)-3-(n-pentyl)-4-phényl-4,5-dihydro-(1H)-pyrazole-1-carboxamide
N-(exo-bicyclo[2.2.1]hept-2-yl)-3-(n-pentyl)-4-phényl-4,5-dihydro-(1H)-pyrazole-1-carboxamide
N-phényl-3-(n-pentyl)-4-phényl-4,5-dihydro-(1H)-pyrazole-1-carboxamide N-[(1R,2S,5R)-rel-6,6-diméthylbicyclo[3.1.1] heptan-2-méthyl]-3-(benzyl)-4-phényl-4,5-dihydro-(1H)-pyrazole-1-carboxamide
N-(1-adamantyl)-3-(benzyl)-4-phényl-4,5-dihydro-(1H)-pyrazole-1-carboxamide
N-[(1R,2S,5R)-rel-6,6-diméthylbicyclo[3.1.1]heptan-2-méthyl]-3-(n-butyl)-4-phényl-4,5-dihydro-(1H)-pyrazole-1-carboxamide
N-[(1R,25,5R)-rel-6,6-diméthylbicydo[3.1.1]heptan-2-méthyl]-3-[3-(1-pipéridinyl)propyl]-4-phényl-4,5-dihydro-(iH)-pyrazole-1-carboxamide N-[(1R,2S,5R)-rel-6,6-diméthylbicyclo[3.1.1]heptan-2-méthyl]-3-(n-propyl)-4-phényl-4,5-dihydro-(1H)-pyrazole-1-carboxamide N-(benzyl)-3-(n-pentyl)-4-phényl-4,5-dihydro-(1H)-pyrazole-1-carboxamide
N-(1-adamantyl)méthyl-3-(n-pentyl)-4-phényl-4,5-dihydro-(1H)-pyrazole-1-carboxamide
N-(cyclohexylméthyl)-3-(n-pentyl)-4-phényl-4,5-dihydro-(1H)-pyrazole-1-carboxamide
N-[endo-(1R,2S,4R)-1,7,7-triméthylbicyclo[2.2.1]hept-2-yl]-3-(n-pentyl)-4-phényl-4,5-dihydro-(1H)-pyrazole-1-carboxamide
N-[endo-(1S)-1,3,3-triméthylbicyclo[2.2.1]hept-2-yl]-3-(n-pentyl)-4-phényl-4,5-dihydro-(1H)-pyrazole-1-carboxamide
N-[(1R,2S,5R)-rel-6,6-diméthylbicyclo[3.1.1]heptan-2-méthyl]-3-(n-propyl)-4-(2-pyridyl)-4,5-dihydro-(1H)-pyrazole-1-carboxamide N-(1-phényl-éthyl)-3-(n-pentyl)-4-phényl-4,5-dihydro-(1H)-pyrazole-1-carboxamide
N-(2-adamantyl)-3-(n-pentyl)-4-phényl-4,5-dihydro-(1H)-pyrazole-1-carboxamide
N-(1-naphtyl)-3-(n-pentyl)-4-phényl-4,5-dihydro-(1H)-pyrazole-1-carboxamide
N-(1-méthyl-1-phényl-éthyl)-3-(n-pentyl)-4-phényl-4,5-dihydro-(1H)-pyrazole-1-carboxamide
N-(2,2-diphénylpropyl)-3-(n-pentyl)-4-phényl-4,5-dihydro-(1H)-pyrazole-1-carboxamide
N-((3-trifluorométhyl)benzyl)-3-(n-pentyl)-4-phényl-4,5-dihydro-(1H)-pyrazole-1-carboxamide
N-(2,2-diméthylpropyl)-3-(n-pentyl)-4-phényl-4,5-dihydro-(1H)-pyrazole-1-carboxamide
N-(naphtalène-1-yl-méthyl)-3-(n-pentyl)-4-phényl-4,5-dihydro-(1H)-pyrazole-1-carboxamide
N-[(3-diméthylamino)-2,2-diméthylpropyl]-3-(n-pentyl)-4-phényl-4,5-dihydro-(1H)-pyrazole-1-carboxamide
N-[endo-(1R,2S,4R)-1,7,7-triméthylbicyclo[2.2.1]hept-2-yl]-3-(n-butyl)-4-phényl-4,5-dihydro-(1H)-pyrazole-1-carboxamide
N-(2-(4-fluorophényl)-1,1-diméthyl-éthyl)-3-(n-butyl)-4-phényl-4,5-dihydro-(1H)-pyrazole-1-carboxamide
N-[endo-(1R,2S,4R)-1,7,7-triméthylbicyclo[2.2.1]hept-2-yl]-3-(4,4,4-trifluoro-n-butyl)-4-phényl-4,5-dihydro-(1H)-pyrazole-1-carboxamide N-(2-(4-fluorophényl)-1,1-diméthyl-éthyl)-3-(4,4,4-trifluoro-n-butyl)-4-phényl-4,5-dihydro-(1H)-pyrazole-1-carboxamide
N-(2-(4-fluorophényl)-1,1-diméthyl-éthyl)-3-(n-pentyl)-4-phényl-4,5-dihydro-(1H)-pyrazole-1-carboxamide
N-[endo-(1R,2S,4R)-1,7,7-triméthylbicyclo[2.2.1]hept-2-yl]-3-(1,1-diméthyl-n-butyl)-4-phényl-4 ,5-dihydro-(1H)-pyrazole-1-carboxamide N-[endo-(1R,2S,4R)-1,7,7-triméthylbicyclo[2.2.1]hept-2-yl]-3-(3,3,3-trifluoropropyl)-4-phényl-4,5-dihydro-(1H)-pyrazole-1-carboxamide N-[endo-(1R,2S,4R)-1,7,7-triméthylbicyclo[2.2.1] hept-2-yl]-3-(1,1-diméthylpropyl)-4-phényl-4,5-dihydro-(1H)-pyrazole-1-carboxamide N-(2-(4-fluorophényl)-1,1-diméthyl-éthyl)-3-(1,1-diméthylpropyl)-4-phényl-4,5-dihydro-(1H)-pyrazole-1-carboxamide
N-[endo-(1R,2S,4R)-1,7,7-triméthylbicyclo[2.2.1]hept-2-yl]-3-(1,1-diméthyl-3,3,3-trifluoropropyl)-4-phényl-4,5-dihydro-(1H)-pyrazole-1-carboxamide
N-[endo-(1R)-1,3,3-triméthylbicyclo[2.2.1]hept-2-yl]-3-(n-butyl)-4-phényl-4,5-dihydro-(1H)-pyrazole-1-carboxamide
N-(1-méthyl-1-phényl-éthyl)-3-(n-butyl)-4-phényl-4,5-dihydro-(1H)-pyrazole-1-carboxamide
N-(2-adamantyl)-3-(n-butyl)-4-phényl-4,5-dihydro-(1H)-pyrazole-1-carboxamide
N-[exo-(1R,2R,4R)-1,7,7-triméthylbicyclo[2.2.1]hept-2-yl]-3-(n-butyl)-4-phényl-4,5-dihydro-(1H)-pyrazole-1-carboxamide
N-(2-phényl-1,1-diméthyl-éthyl)-3-(n-butyl)-4-(3-fluorophényl)-4,5-dihydro-(1H)-pyrazole-1-carboxamide
N-(2-phényl-1,1-diméthyl-éthyl)-3-(n-butyl)-4-(2-fluorophényl)-4,5-dihydro-(1H)-pyrazole-1-carboxamide
N-phényl-3-(4-chlorobenzyl)-4-(4-chlorophényl)-4,5-dihydro-(1H)-pyrazole-1-carboxamide
N-(4-méthoxyphényl)-3-(4-chlorobenzyl)-4-(4-chlorophényl)-4,5-dihydro-(1H)-pyrazole-1-carboxamide
N-[endo-(1R,2S,4R)-1,7,7-triméthylbicyclo[2.2.1]hept-2-yl]-3-(n-butyl)-4-(2-méthoxyphényl)-4,5-dihydro-(1H)-pyrazole-1-carboxamide
N-(1-méthyl-1-phényl-éthyl)-3-(n-butyl)-4-(2-méthoxyphényl)-4,5-dihydro-(1H)-pyrazole-1-carboxamide
N-[endo-(1R,2S,4R)-1,7,7-triméthylbicyclo[2.2.1]hept-2-yl]-3-(n-butyl)-4-(2-fluorophényl)-4,5-dihydro-(1H)-pyrazole-1-carboxamide
N-[endo-(lR,2S,4R)-1,7,7-triméthylbicyclo[2.2. 1]hept-2-yl]-3-(n-butyl)-4-(pyrid-3-yl)-4,5-dihydro-(1H)-pyrazole-1-carboxamide
N-[(1R,2R,3R,5S)-2,7,7-triméthylbicyclo[3.1.1]hept-3-yl]-3-(n-butyl)-4-(3-fluorophényl)-4,5-dihydro-(1H)-pyrazole-1-carboxamide
N-[endo-(1R)-1,3,3-triméthylbicyclo[2.2.1]hept-2-yl]-3-(n-butyl)-4-(2-fluorophényl)-4,5-dihydro-(iH)-pyrazole-1-carboxamide
N-[2-(trifluorométhyl)benzyl]-3-(n-butyl)-4-(3-fluorophényl)-4,5-dihydro-(1H)-pyrazole-1-carboxamide
N-[exo-(1R,2R,4R)-1,7,7-triméthylbicyclo[2.2.1]hept-2-yl]-3-(n-butyl)-4-(2-fluorophényl)-4,5-dihydro-(1H)-pyrazole-1-carboxamide
N-(1-méthyl-1-phényl-éthyl)-3-(n-butyl)-4-(3-fluorophényl)-4,5-dihydro-(1H)-pyrazole-1-carboxamide
N-(1-méthyl-1-phényl-éthyl)-3-(n-butyl)-4-(4-chlorophényl)-4,5-dihydro-(1H)-pyrazole-1-carboxamide
N-[2-(trifluorométhyl)benzyl]-3-(n-butyl)-4-(4-chlorophényl)-4,5-dihydro-(1H)-pyrazole-1-carboxamide
N-[endo-(1R,2S,4R)-1,7,7-triméthylbicyclo[2.2.1]hept-2-yl]-3-(cydopropylméthyl)-4-phényl-4,5-dihydro-(1H)-pyrazole-1-carboxamidé
N-[endo-(1R,2S,4R)-1,7,7-triméthylbicyclo[2.2.1]hept-2-yl]-3-(n-butyl)-4-(4-fluorophényl)-4,5-dihydro-(1H)-pyrazole-1-carboxamide N-(1-méthyl-1-phényl-éthyl)-3-(n-butyl)-4-(4-fluorophényl)-4,5-dihydro-(1H)-pyrazole-1-carboxamide
N-(adamant-2-yl)-3-(n-butyl)-4-(4-fluorophényl)-4,5-dihydro-(1H)-pyrazole-1-carboxamide
N-(1-méthyl-1-(4-fluorophényl)-éthyl)-3-(n-butyl)-4-(4-fluorophényl)-4,5-dihydro-(1H)-pyrazole-1-carboxamide
N-(1-méthyl-1-(4-fluorophényl)-éthyl)-3-(n-butyl)-4-phényl-4,5-dihydro-(1H)-pyrazole-1-carboxamide
N-(1-méthyl-1-phényl-éthyl)-3-(n-pentyl)-4-(2-fluorophényl)-4,5-dihydro-(1H)-pyrazole-1-carboxamide
N-[endo-(1R,2S,4R)-1,7,7-triméthylbicyclo[2.2.1]hept-2-yl]-3-(n-pentyl)-4-(2-fluorophényl)-4,5-dihydro-(1H)-pyrazole-1-carboxamide N-(1-méthyl-1-(4-fluorophényl)-éthyl)-3-(n-pentyl)-4-(2-fluorophényl)-4,5-dihydro-(1H)-pyrazole-1-carboxamide
N-(1-méthyl-1-(4-fluorophényl)-éthyl)-3-(n-pentyl)-4-(3-fluorophényl)-4,5-dihydro-(1H)-pyrazole-1-carboxamide
N-(adamant-2-yl)-3-(n-pentyl)-4-(2-fluorophényl)-4,5-dihydro-(1H)-pyrazole-1-carboxamide
N-(adamant-2-yl)-3-(n-pentyl)-4-(3-fluorophényl)-4,5-dihydro-(1H)-pyrazole-1-carboxamide
N-(1-méthyl-1-phényl-éthyl)-3-(n-butyl)-4-(benzo[b]thiophèn-3-yl)-4,5-dihydro-(1H)-pyrazole-1-carboxamide
N-[endo-(1R,2S,4R)-1,7,7-triméthylbicyclo[2.2.1]hept-2-yl]-3-(n-butyl)-4-(benzo[b]thiophèn-3-yl)-4,5-dihydro-(1H)-pyrazole-1-carboxamide N-(1-méthyl-1-phényl-éthyl)-3-(n-butyl)-4-(thiophène-3-yl)-4,5-dihydro-(1H)-pyrazole-1-carboxamide
N-(1-méthyl-1-phényl-éthyl)-3-(but-3-ynyl)-4-(2-fluorophényl)-4,5-dihydro-(1H)-pyrazole-1-carboxamide
N-[endo-(1R,2S,4R)-1,7,7-triméthylbicyclo[2.2.1]hept-2-yl]-3-(but-3-ynyl)-4-(2-fluorophényl)-4,5-dihydro-(1H)-pyrazole-1-carboxamide N-[endo-(1R,2S,4R)-1,7,7-triméthylbicyclo[2.2.1]hept-2-yl]-3-(1-phénylcyclopropyl)-4-phényl-4,5-dihydro-(1H)-pyrazole-1-carboxamide N-(1-méthyl-1-phényl-éthyl)-3-(1-phénylcyclopropyl)-4-phényl-4,5-dihydro-(1H)-pyrazole-1-carboxamide
N-[endo-(1R,2S,4R)-1,7,7-triméthylbicyclo[2.2.1]hept-2-yl]-3-(2,2,3,3-tétraméthylcyclopropyl)-4-phényl-4,5-dihydro-(1H)-pyrazole-1-carboxamide
N-(1-méthyl-1-phényl-éthyl)-3-(2,2,3,3-tétraméthylcyclopropyl)-4-phényl-4,5-dihydro-(1H)-pyrazole-1-carboxamide
N-[(1R,2R,3R,5S)-2,7,7-triméthylbicyclo[3.1.1]hept-3-yl]-3-(n-butyl)-4-(4-chlorophényl)-4,5-dihydro-(1H)-pyrazole-1-carboxamide N-(1-méthyl-1-phényl-éthyl)-3-(n-pentyl)-4-(3-fluorophényl)-4,5-dihydro-(1H)-pyrazole-1-carboxamide
N-[endo-(1R,2S,4R)-1,7,7-triméthylbicyclo[2.2.1]hept-2-yl]-3-(n-pentyl)-4-(3-fluorophényl)-4,5-dihydro-(1H)-pyrazole-1-carboxamide N-(1-méthyl-1-phényl-éthyl)-3-(n-pentyl)-4-(4-fluorophényl)-4,5-dihydro-(1H)-pyrazole-1-carboxamide
N-[endo-(1R,2S,4R)-1,7,7-triméthylbicyclo[2.2.1]hept-2-yl]-3-(n-pentyl)-4-(4-fluorophényl)-4,5-dihydro-(1H)-pyrazole-1-carboxamide N-[(1S,2S,3S,5R)-2,7,7-triméthylbicyclo[3.1.1]hept-3-yl]-3-(n-butyl)-4-(3-fluorophényl)-4,5-dihydro-(1H)-pyrazole-1-carboxamide N-(1-méthyl-1-(4-fluorophényl)-éthyl)-3-(n-butyl)-4-(3-fluorophényl)-4,5-dihydro-(1H)-pyrazole-1-carboxamide
N-(1-méthyl-1-(4-fluorophényl)-éthyl)-3-(n-butyl)-4-(2-fluorophényl)-4,5-dihydro-(1H)-pyrazole-1-carboxamide
N-[(1S,2S,3S,5R)-2,7,7-triméthylbicyclo[3.1.1]hept-3-yl]-3-(n-butyl)-4-(2-fluorophényl)-4 ,5-dihydro-(1H)-pyrazole-1-carboxamide N-[endo-(1R,2S,4R)-1,7,7-triméthylbicyclo[2.2.1]hept-2-yl]-3-(n-butyl)-4-(4-thièn-3-yl)-4,5-dihydro-(1H)-pyrazole-1-carboxamide N-[endo-(1R,2S,4R)-1,7,7-triméthylbicyclo[2.2.1]hept-2-yl]-3-(3,3,3-trifluoro-1-méthoxyméthyl-propyl)-4-phényl-4 ,5-dihydro-(1H)-pyrazole-1-carboxamide
N-[endo-(1R,2S,4R)-1,7,7-triméthylbicyclo[2.2.1]hept-2-yl]-3-(n-butyl)-4-hydroxy-4-phényl-4,5-dihydro-(1H)-pyrazole-1-carboxamide 1-(1-naphtoyl)-3-(n-pentyl)-4-phényl-4,5-dihydro-(1H)-pyrazole [3-(n-pentyl)-4-phényl-4,5-dihydro-(1H)-pyrazol-1-yl]-[1-(4-chlorophényl)cyclopentyl]méthanone
[3-(n-pentyl)-4-phényl-4,5-dihydro-(1H)-pyrazol-1-yl]-(napht-2-yl)méthanone
[3-(n-pentyl)-4-phényl-4,5-dihydro-(1H)-pyrazol-1-yl]-(diphénylméthyl)-méthanone
[3-(n-pentyl)-4-phényl-4,5-dihydro-(1H)-pyrazol-1-yl]-(3-chlorobenzothièn-2-yl]méthanone
[3-(n-pentyl)-4-phényl-4,5-dihydro-(1H)-pyrazol-1-yl]-(benzofuran-2-yl]méthanone
[3-(n-pentyl)-4-phényl-4,5-dihydro-(1H)-pyrazol-1-yl]-[2,4,4-(triméthyl)pentyl]méthanone
[3-(n-pentyl)-4-phényl-4,5-dihydro-(iH)-pyrazol-1-yl]-[3-(trifluorométhyl)phényl]méthanone
(Cis-3,4,5-triméthylpipérazin-1-yl)[3-(n-pentyl)-4-phényl-4,5-dihydro-(1H)-pyrazol-1-yl]méthanone
N-endo-[(1R,2S,4R)-1,7,7-triméthylbicyclo[2.2.1]hept-2-yl]-3-(n-pentyl)-4-phényl-4,5-dihydro-(1H)-pyrazole-1-carboxamide (diastéréomère A) N-endo-[(1R,2S,4R)-1,7,7-triméthylbicyclo[2.2.1]hept-2-yl]-3-(n-pentyl)-4-phényl-4,5-dihydro-(1H)-pyrazole-1-carboxamide (diastéréomère B) N-endo-[(1R,2S,4R)-1,7,7-triméthylbicyclo[2.2.1]hept-2-yl]-3-(n-butyl)-4-(3-fluorophényl)-4,5-dihydro-(1H)-pyrazole-1-carboxamide (diastéréomère A)
N-endo-[(1R,2S,4R)-1,7,7-triméthylbicyclo[2.2.1]hept-2-yl]-3-(n-butyl)-4-(3-fluorophényl)-4,5-dihydro-(1H)-pyrazole-1-carboxamide (diastéréomère B)
N-endo-[(1R,2S,4R)-1,7,7-triméthylbicyclo[2.2.1]hept-2-yl]-3-(n-butyl)-4-(4-chlorophényl)-4,5-dihydro-(1H)-pyrazole-1-carboxamide (diastéréomère A)
N-endo-[(1R,2S,4R)-1,7,7-triméthylbicyclo[2.2.1]hept-2-yl]-3-(n-butyl)-4-(4-chlorophényl)-4,5-dihydro-(1H)-pyrazole-1-carboxamide (diastéréomère B)
N-(1,2,2,6,6-pentaméthylpipéridin-4-yl)-3-(n-butyl)-4-(2-fluorophényl)-4,5-dihydro-(1H)-pyrazole-1-carboxamide
N-(4-méthoxyphényl)-3-(n-pentyl)-4-phényl-4,5-dihydro-(1H)-pyrazole-1-carboxamide
N-(4-méthoxyphényl)-3-(n-pentyl)-4-phényl-4,5-dihydro-(1H)-pyrazole-1-carboxamide
N-(phénéthyl)-3-(n-pentyl)-4-phényl-4,5-dihydro-(1H)-pyrazole-1-carboxamide
N-(2-phényl-trans-cyclopropyl)-3-(n-pentyl)-4-phényl-4,5-dihydro-(1H)-pyrazole-1-carboxamide
N-(1-naphtalèn-1-yl-éthyl)-3-(n-pentyl)-4-phényl-4,5-dihydro-(1H)-pyrazole-1-carboxamide
N-[2-(trifluorométhyl)phényl]-3-(n-pentyl)-4-phényl-4,5-dihydro-(1H)-pyrazole-1-carboxamide
N-cycloheptyl-3-(n-pentyl)-4-phényl-4,5-dihydro-(1H)-pyrazole-1-carboxamide
N-cyclooctyl-3-(n-pentyl)-4-phényl-4,5-dihydro-(1H)-pyrazole-1-carboxamide
N-(1,2,3,4-tétrahydronaphtalène-1-yl)-3-(n-pentyl)-4-phényl-4,5-dihydro-(1H)-pyrazole-1-carboxamide
N-[2,2-(diphényl)éthyl]-3-(n-pentyl)-4-phényl-4,5-dihydro-(iH)-pyrazole-1-carboxamide
(3-pentyl-4-phényl-4,5-dihydropyrazole-1-yl)-[4-(2-pyrimidinyl)pipérazin-1-yl]méthanone
N-[2-(4-fluorophényl)éthyl]-3-(n-pentyl)-4-phényl-4,5-dihydro-(1H)-pyrazole-1-carboxamide
(3-pentyl-4-phényl-4,5-dihydropyrazol-1-yl)-[azépan-1-yl]méthanone N-(quinoléin-3-yl)-3-(n-pentyl)-4-phényl-4,5-dihydro-(1H)-pyrazole-1-carboxamide
N-[1-(éthyl)propyl]-3-(n-pentyl)-4-phényl-4,5-dihydro-(1H)-pyrazole-1-carboxamide
N-(2,2,2-trifluoroéthyl)-3-(n-pentyl)-4-phényl-4,5-dihydro-(1H)-pyrazole-1-carboxamide
N-(pyridin-3-ylméthyl)-3-(n-pentyl)-4-phényl-4,5-dihydro-(1H)-pyrazole-1-carboxamide
N-(2-indanyl)-3-(n-pentyl)-4-phényl-4,5-dihydro-(1H)-pyrazole-1-carboxamide
(3-pentyl-4-phényl-4,5-dihydropyrazole-1-yl)-(1,2,3,4-tétrahydroisoquinoléin-2-yl]méthanone
N-(méthyl),N-(naphtalèn-1-ylméthyl)-3-(n-pentyl)-4-phényl-4,5-dihydro-(1H)-pyrazole-1-carboxamide
N-(3,3-diphénylpropyl)-3-(n-pentyl)-4-phényl-4,5-dihydro-(1H)-pyrazole-carbothiamide
N-(napht-1-yl)-3-(n-pentyl)-4-phényl-4,5-dihydro-(1H)-pyrazole-carbotriamide
N-[1-(éthyl)propyl]-3-(n-pentyl)-4-phényl-4,5-dihydro-(1H)-pyrazole-1-carboxamide
N-[pyridine-3-ylméthyl]-3-(n-pentyl)-4-phényl-4,5-dihydro-(1H)-pyrazole-carbothiamide
N-[exo-bicyclo[2.2.1]hept-2-yl]-3-(n-pentyl)-4-phényl-4,5-dihydro-(iH)-pyrazolecarbothiamide
1-(naphtalèn-1-ylsulfonyl)-3-(n-butyl)-4-(2-fluorophényl)-4,5-dihydro-(1H)-pyrazole
1-(naphtalèn-2-ylsulfonyl)-3-(n-butyl)-4-(2-fluorophényl)-4,5-dihydro-(1H)-pyrazole
N-[(1R,2S,5R)-rel-6,6-diméthylbicyclo[3.1.1]heptan-2-méthyl]-3-(n-pentyl)-4-phényl-4,5-dihydro-(1H)-pyrazole-1-sulfonamide

9. Composés de la formule générale (IV) 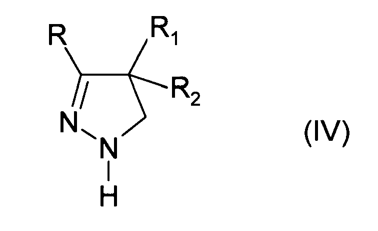 dans laquelle R et R₁ ont les mêmes significations que données dans la revendication 1 et R₂ représente un groupe phényle qui peut être substitué par 1-5-substituants Y2 qui peuvent être identiques ou différents, choisis dans le groupe constitué des groupes alcoxy en C₁₋₃, hydroxy, trifluorométhyle, trifluorométhylthio, trifluorométhoxy, nitro, amino, (mono- ou dialkyle en C₁₋₂)-amino, (mono- ou dialkyle en C₁₋₂)-amido, (alkyle en C₁₋₃)-sulfonyle, diméthylsulfamido, (alcoxy en C₁₋₃)-carbonyle, carboxyle, trifluorométhylsulfonyle, cyano, carbamoyle, sulfamoyle, ortho-halogène, méta-halogène, ortho-(alkyle en C₁₋₃), méta-(alkyle en C₁₋₃) et acétyle, ou R₂ représente un groupe thiényle ou pyridyle, lesquels groupes peuvent être substitués par un ou deux substituants Y, lequel groupe Y a la même signification que donné dans la revendication 1, de tels composés étant utiles dans la synthèse des composés de la formule générale (I).

10. Composés de la formule générale (VIII) 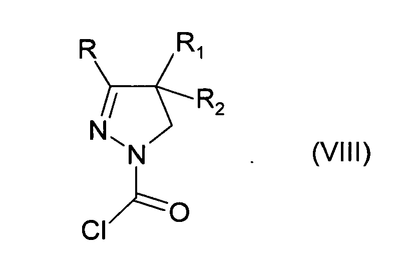 dans laquelle R et R₂ ont les mêmes significations que données dans la revendication 1 et R₁ représente un atome d'hydrogène, de tels composés étant utiles dans la synthèse des composés de la formule générale (I) dans laquelle n = 1.

11. Composé selon l'une quelconque des revendications 1-8, ou sel, hydrate, solvate ou complexe de celui-ci pharmacologiquement acceptable destiné à une utilisation comme médicament.

12. Médicament **caractérisé en ce qu'**il contient un composé selon l'une quelconque des revendications 1-8, ou un sel, hydrate, solvate ou complexe de celui-ci pharmacologiquement acceptable.

13. Composition pharmaceutique comprenant, en plus d'un support pharmaceutiquement acceptable et/ou d'au moins une substance auxiliaire pharmaceutiquement acceptable, une quantité pharmacologiquement active d'au moins un composé selon l'une quelconque des revendications 1-8 ou d'un sel, hydrate, solvate ou complexe de celui-ci pharmacologiquement acceptable, comme ingrédient actif.

14. Composition pharmaceutique selon la revendication 13 pour la prévention ou le traitement de la sclérose en plaques, d'une lésion cérébrale traumatique, de la douleur, de troubles de l'appétit, de l'épilepsie, de la maladie d'Alzheimer, du syndrome de la Tourette, de l'ischémie cérébrale ou de troubles gastro-intestinaux.

15. Composition pharmaceutique selon la revendication 13 comprenant de plus : au moins un agent thérapeutique supplémentaire.

16. Procédé de préparation de compositions pharmaceutiques selon la revendication 13, **caractérisé en ce qu'**un composé selon l'une quelconque des revendications 1-8 est mis dans une forme appropriée pour l'administration.

17. Composition pharmaceutique fabriquée en mélangeant un composé selon la revendication 1 et un support pharmaceutiquement acceptable et/ou au moins une substance auxiliaire pharmaceutiquement acceptable.

18. Utilisation d'un composé selon l'une quelconque des revendications 1-8 pour la préparation d'une composition pharmaceutique destinée au traitement de la sclérose en plaques, d'une lésion cérébrale traumatique, de la douleur, de troubles de l'appétit, de l'épilepsie, de la maladie d'Alzheimer, du syndrome de la Tourette, de l'ischémie cérébrale et de troubles gastro-intestinaux.
